# EUROPEAN PATENT APPLICATION

(11) **EP 4 438 738 A1**
(43) Date of publication of application: **02.10.2024**
(21) Application number: 23461542.5
(22) Date of filing: 30.03.2023
(51) Int. Cl.: C12Q 1/6804

(54) **METHOD OF DETECTION OF DNA END(S)**

(71) Applicant: INTODNA SPÓLKA AKCYJNA, 30-348 Kraków (PL)
(72) Inventor: KORDON-KISZALA, Magdalena, Kraków (PL); SOLARCZYK, Kamil, Kraków (PL); SZCZYGIEL, Malgorzata, Kraków (PL); WALIGÓRSKA, Agnieszka, Kraków (PL); UZNANSKA, Karolina, Kraków (PL); PRUCSI, Zsombor, Kraków (PL)
(74) Representative: Boult Wade Tennant LLP

(57) **Abstract**

The invention relates to methods for detection of nucleic acid end(s) in a biological material containing nucleic acids. The invention also relates to kits for detection of nucleic acid end(s) in a biological material and associated uses of the kits. In addition, the invention relates to methods for assessing effectiveness of a therapeutic agent.

## Description

### FIELD

The invention relates to methods for detection of nucleic acid end(s) in a biological material containing nucleic acids. The invention also relates to kits for detection of nucleic acid end(s) in a biological material and associated uses of the kits. In addition, the invention relates to methods for assessing effectiveness of a therapeutic agent.

### BACKROUND

Since the early days of the synthetic lethality concept in DNA damage response, the homologous recombination (HR) status of cancer cells has been the focus of attention of researchers and clinicians. Functional homologous repair deficiency (HRD) may be defined by a RAD51-low score. For example, the RAD-51-low score has emerged as an indicator of patient outcome and response to therapeutic agents used to treat triple negative breast cancer. The RAD51-low score is also used to assess the therapeutic efficiency of PARP inhibitors. While different approaches to detect RAD51 exist (e.g. the RAD51 immunofluorescence (IF)), functional biomarkers that can assess RAD51-driven HR proficiency are missing.

Mismatch repair (MMR) is considered to be one of the fundamental pathways that guards genome stability and mutations in genes encoding for crucial MMR proteins (e.g. PMS1, PMS2 or MLH1) have been shown to promote cancer initiation. On the other hand, inactivation of MMR components in cancer cells leads to increased neoantigen formation and in consequence, exposes the tumour to the immune system. Therapeutic strategies aiming at inhibiting MMR are thus under development, yet methods reporting on the status of MMR are lacking.

WO2019/035727 A1 describes a method for detecting DNA end(s). The method relies on molecules, such as BrdU, binding directly to the DNA end(s), followed by binding of binding molecules (e.g. antibodies) to the molecules bound to the DNA end(s). In the method, the binding molecules are conjugated to oligonucleotides which are able to hybridize with further oligonucleotides to form a circular structure. The circular structure provides a template for rolling circle amplification. Once amplified, the amplification product may be detected by detection methods known in the art, for example, fluorescence microscopy. However, this method does not allow for the direct detection of nucleic acid binding proteins which are involved in nucleic acid (e.g. DNA) damage response, mismatch repair and homologous recombination.

Thus, a need exists for more accurate approaches for detection of proteins involved in nucleic acid (e.g. DNA) damage response and repair, mismatch repair and homologous recombination.

### SUMMARY OF THE INVENTION

The inventors modified the method of WO2019/035727 A1 (termed "STRIDE") to allow detection of nucleic acid binding proteins (such as PMS1, PMS2, MLH1, RAD51 or RPA70) bound to nucleic acids at the nucleic acid end(s) (e.g. DNA ends) in biological material. The inventors discovered an accurate method for detection and quantification of proteins involved in nucleic acid (e.g. DNA) damage response and repair, mismatch repair and homologous recombination. Thus, the approach provides a method for simultaneous detection of nucleic acid end(s) and nucleic acid binding protein(s) bound to the nucleic acid at those end(s). Notably, this method includes detection of cytosolic nucleic acids (especially DNA), which may be bound for example by cyclic GMP-AMP synthase (cGAS).

In one aspect, the invention provides a method for detection of nucleic acid end(s) in a biological material containing nucleic acids, wherein the method comprises the steps of:
a) incubating the biological material with a proteinase;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

Incubating the biological material with a proteinase improves detection of a nucleic acid binding protein in the biological material (relative to the situation where no proteinase is used). Specifically, incubating the biological material with a proteinase increases accessibility of the nucleic acid end(s) without significantly affecting the levels of the nucleic acid binding protein in the biological material.

Without being bound by theory, increasing accessibility of the nucleic acid end(s) may minimise steric hindrance in the locality of the DNA ends to be detected.

It is to be understood that each aspect and embodiment which refers to a method for detection of nucleic acid end(s) in the biological material also refers to a method for detection of nucleic acid binding proteins which are bound to the nucleic acid at the nucleic acid end(s). Thus, each aspect and embodiment described herein may be understood as referring to a method for detection of a nucleic acid binding protein in a biological material comprising the nucleic acid binding protein.

The step of incubating the biological material with a proteinase may be performed under such conditions to increase accessibility of the nucleic acid end(s) to the level achieved using proteinase K at a concentration of about 7 µg/mL (e.g. 7.1 µg/mL) for less than 2 minutes at a temperature of about 22 ºC (i.e. room temperature). The incubation with proteinase may be performed with reference to any one of Examples 1-3.

The invention provides a method for detection of nucleic acid end(s) in a biological material containing nucleic acids, wherein the method comprises the steps of:
a) incubating the biological material with a proteinase to increase accessibility of the nucleic acid end(s) to the level achieved using proteinase K at a concentration of 6 µg/mL to 8 µg/mL for less than 2 minutes at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention provides a method for detection of nucleic acid end(s) in a biological material containing nucleic acids, wherein the method comprises the steps of:
a) incubating the biological material with a proteinase to increase accessibility of the nucleic acid end(s) to the level achieved using proteinase K at a concentration of about 7 µg/mL (e.g. 7.1 µg/mL) for less than 2 minutes at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention provides a method for detection of single stranded nucleic acid end(s) in a biological material containing nucleic acids, wherein the method comprises the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 60 seconds at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the single stranded nucleic acid end(s).

The invention provides a method for detection of double stranded nucleic acid end(s) in a biological material containing nucleic acids, wherein the method comprises the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the double stranded nucleic acid end(s).

The invention provides a method for detection of nucleic acid end(s) in a biological material containing nucleic acids, wherein the method comprises the steps of:
a) incubating the biological material with a proteinase for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention provides a method for detection of single stranded nucleic acid end(s) in a biological material containing nucleic acids, wherein the method comprises the steps of:
a) incubating the biological material with a proteinase for less than 60 seconds;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the single stranded nucleic acid end(s).

The invention provides a method for detection of double stranded nucleic acid end(s) in a biological material containing nucleic acids, wherein the method comprises the steps of:
a) incubating the biological material with a proteinase for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the double stranded nucleic acid end(s).

In all aspects and embodiments described herein, preferably, the nucleic acids comprise or consist of DNA.

In all aspects and embodiments described herein, preferably, the nucleic acid end(s) are DNA end(s).

In all aspects and embodiments described herein, preferably, the proteinase is proteinase K.

In all aspects and embodiments described herein, preferably, the nucleic acid binding protein and the nucleic acid binding molecule are not the same molecules.

In an aspect, the invention provides a kit for detection of nucleic acid end(s) in a biological material, the kit comprising:
a) nucleic acid binding molecules;
b) a binding molecule that binds to the nucleic acid binding molecules; and
c) a binding molecule that binds to a nucleic acid binding protein.

In an aspect, the invention provides a kit for detection of nucleic acid end(s) in a biological material, the kit comprising:
a) nucleic acid binding molecules;
b) a monoclonal antibody that binds to the nucleic acid binding molecules; and
c) a monoclonal antibody that binds to a nucleic acid binding protein.

In an aspect, the invention provides a method for assessing effectiveness of a therapeutic agent, the method comprising the steps:
a) performing the detection method described herein on a sample obtained from a subject before the administration of the therapeutic agent,
b) performing the detection method described herein on a sample obtained from the subject after the administration of the therapeutic agent,
c) comparing the amount of the nucleic acid end(s) detected in steps a) and b), wherein the therapeutic agent is effective if the amount of the detected nucleic acid end(s) is greater in step a) than step b),
wherein steps a) and b) can be performed in any order.

Preferably, the therapeutic agent targets PMS1, PMS2 or MLH1.

In an aspect, the invention provides a method for assessing effectiveness of a therapeutic agent, the method comprising the steps:
a) performing the detection method described herein on a sample obtained from a subject before the administration of the therapeutic agent,
b) performing the detection method described herein on a sample obtained from the subject after the administration of the therapeutic agent,
c) comparing the amount of the nucleic acid end(s) detected in steps a) and b), wherein the therapeutic agent is effective if the amount of the nucleic acid end(s) is greater in step b) than step a),
wherein steps a) and b) can be performed in any order.

Preferably, the therapeutic agent targets RPA (e.g. RPA70) or RAD51.

In an aspect, the invention provides a method for assessing nucleic acid damage caused by an agent, the method comprising the steps:
a) performing the detection method described herein on a sample obtained from a subject before administration of, or exposure to, an agent,
b) performing the detection method described herein on a sample obtained from the subject after the administration of, or exposure to, the agent,
c) comparing the amount of the nucleic acid end(s) detected in steps a) and b), wherein the agent causes nucleic acid damage if the amount of the nucleic acid end(s) is greater in step b) than step a),
wherein steps a) and b) can be performed in any order.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The present inventors have surprisingly discovered that the biological material (e.g. a cell) comprising a nucleic acid and a nucleic acid binding protein may undergo a proteinase treatment to increase accessibility of the nucleic acid end(s) without significantly affecting the levels of the nucleic acid binding protein in the biological material. Increased accessibility of the nucleic acid end(s) facilitates the binding of nucleic acid binding molecules to the nucleic acid end(s) which improves the level of detection of the nucleic acid end(s) and nucleic acid binding proteins in the biological material. Thus, the inventors have discovered a more accurate and sensitive method for detection of nucleic acid binding proteins (and the co-localised nucleic acid end(s)) in the biological material.

### DETAILED DESCRIPTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a temperature of "about 25 °C" can include temperatures between (and including) 22.5 °C and 27.5 °C.

The invention provides a method for detection of nucleic acid end(s) (e.g. DNA end(s)) in a biological material containing nucleic acids (e.g. DNA). The invention also provides a method for detection of nucleic acid binding protein(s) in a biological material containing the nucleic acid binding protein(s). The method relies on the presence of a nucleic acid binding protein i) in the proximity of; or ii) bound to a nucleic acid end(s). The method relies on the detection of the event of colocalization of the nucleic acid binding protein and the nucleic acid end(s) in the biological material. Preferably, the nucleic acid binding protein is found in the biological material. That is to say that the method relies on the presence of intrinsic nucleic acid binding proteins in the biological material.

The requirement that "the nucleic acid binding protein is bound to the nucleic acid at the nucleic acid end(s)" means that the nucleic acid binding protein is bound either directly or indirectly in sufficient proximity to the nucleic acid end(s) to allow detection of the co-localisation of the nucleic acid binding protein and the nucleic acid end(s) by the methods described herein. Sufficient proximity is typically within 100 nm of the nucleic acid end(s), preferably within 50 nm of the nucleic acid end(s) and most preferably within 40 nm of the nucleic acid end(s). The nucleic acid binding protein may be bound to the nucleic acid at the nucleic acid end(s) indirectly, e.g. as part of a (multi-protein) complex, which provides sufficient proximity to the nucleic acid end(s) to allow detection. The nucleic acid binding protein may be bound to cytosolic nucleic acid fragments, e.g. in the case of cGAS. As would be readily understood by the skilled person, the term "at the nucleic acid end(s)" as used herein is intended to encompass nucleic acid residues which are at the end of a nucleic acid strand. This encompasses gaps within a nucleic acid strand. Preferably, the nucleic acid end(s) are the result of nucleic acid damage, homologous recombination, or mismatch repair.

The term "intrinsic" in the context of the nucleic acid binding proteins is intended to encompass nucleic acid binding proteins which are found in the biological material. Thus, these proteins are not added to the biological material as part of the methods or kits described herein. The intrinsic nucleic acid binding proteins are naturally found (i.e. naturally occurring) in the biological material. Thus, the biological material (e.g. cell) may comprise a nucleic acid and a nucleic acid binding protein prior to the step of proteinase treatment. The intrinsic nucleic acid binding proteins are not added to the biological material by a user of the method or kit.

Detection based on "co-localization" as used herein (in the context of colocalization of the nucleic acid end(s) and the nucleic acid binding protein) means that the presence of the nucleic acid end(s) and the nucleic acid binding protein are detected based on their proximity to one another. This is typically through a single detection (e.g. fluorescence) signal generated only when the nucleic acid end(s) and the nucleic acid binding protein are in close proximity to one another, i.e. the nucleic acid binding protein is bound to the nucleic acid at the nucleic acid end(s). In the absence of this close proximity no signal is generated. The nucleic acid binding proteins detected according to the invention may thus those that are involved in detection or repair of nucleic acid damage (i.e. damage resulting in detectable nucleic acid end(s), which includes cytosolic chromatin fragments).

The nucleic acid binding proteins may bind to the nucleic acid at the nucleic acid end(s) either directly (i.e. by direct association with the nucleic acid) or indirectly (i.e. by forming a complex molecule with at least one other molecule (e.g. protein) that binds to the nucleic acid directly and therefore acts as an adaptor molecule for indirect binding of a nucleic acid binding protein).

By screening a range of experimental parameters, the inventors discovered optimal conditions for the detection of co-localisation of nucleic acid binding proteins and the nucleic acid end(s) (at which the nucleic acid binding protein binds, directly or indirectly as defined herein). Specifically, the inventors found that proteinase treatment (before the step of detection) increases accessibility of the nucleic acid end(s) in the biological material without significantly affecting the levels of the nucleic acid binding protein to be detected. Preferably, the proteinase is a serine proteinase (especially proteinase K). The inventors found that the treatment with proteinase at a concentration of between 6 µg/mL and 8 µg/mL (e.g. about 7 µg/mL) for less than 2 minutes (preferably 90 seconds or less) at a temperature of about 22 ºC (i.e. room temperature) delivers an optimal combination of increased accessibility of the nucleic acid end(s) and no significant effect on the levels of the nucleic acid binding protein to be detected. The skilled person would understand that these parameters may be altered by e.g. reducing the concentration of proteinase and extending the time of treatment, or by increasing the concentration of proteinase and reducing the time of treatment. The proteinase treatment is preferably shorter when a single stranded nucleic acid end is to be detected in the biological material as compared to detecting double stranded nucleic acid end(s). The methods described herein may comprise a step of incubating the biological material with a proteinase to increase accessibility of the nucleic acid end(s) to the level achieved using proteinase (e.g. proteinase K) at a concentration of 6 µg/mL to 8 µg/mL (e.g. about 7 µg/mL) for less than 2 minutes (preferably 90 seconds or less) (and without significantly affecting the levels of the nucleic acid binding protein to be detected). If the nucleic acid end(s) is single stranded, the methods described herein may comprise a step of incubating the biological material with a proteinase to increase accessibility of the nucleic acid end(s) to the level achieved using proteinase (e.g. proteinase K) at a concentration of 6 µg/mL to 8 µg/mL (e.g. about 7 µg/mL) for less than 90 seconds (preferably about 30 seconds) (and without significantly affecting the levels of the nucleic acid binding protein to be detected). Preferably, the method is performed under conditions such that the levels of nucleic acid binding proteins to be detected are not significantly reduced.

The expressions "without significantly affecting", "not significantly reduced" or "not significantly reducing" as used herein in the context of the levels of nucleic acid binding proteins means that the detectable levels of the nucleic acid binding protein(s) after the treatment with the proteinase remain substantially the same as before the treatment. For example, the detectable levels of nucleic acid binding protein(s) after the treatment may be 100%, at least 99%, at least 98%, at least 97%, at least 96%, at least 95%, at least 94%, at least 93%, at least 92%, at least 91%, at least 90%, at least 85%, at least 80%, at least 75%, at least 70%, at least 65%, at least 60%, or at least 50% of the levels before the treatment. Preferably, the levels of the nucleic acid binding protein remain detectable and, due to the reduction in background proteins, are detected better than in the absence of proteinase treatment.

The biological material may comprise a nucleic acid (e.g. DNA). The biological material may comprise a nucleic acid binding protein (e.g. DNA binding protein).

The invention provides a method for detection of nucleic acid end(s) in a biological material containing nucleic acids, wherein the method comprises the steps of:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention also provides a method for detection of nucleic acid binding protein(s) in a biological material containing the nucleic acid binding protein, wherein the method comprises the steps of:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s); and
d) detecting the nucleic acid binding protein(s) in the biological material by detecting a co-localisation of the nucleic acid binding protein and the nucleic acid end via binding of the two different binding molecules.

As discussed herein, the nucleic acid binding protein may bind to the nucleic acid either directly or indirectly.

Thus, the method for detection of nucleic acid end(s) in a biological material containing nucleic acids may comprise the steps of:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound directly to the nucleic acid at the nucleic acid end(s); and
d) detecting the nucleic acid end(s) in the biological material by detecting a co-localisation of the nucleic acid binding protein and the nucleic acid end via binding of the two different binding molecules.

The method for detection of nucleic acid end(s) in a biological material containing nucleic acids may comprise the steps of:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s) via an adaptor molecule (e.g. adaptor protein); and
d) detecting the nucleic acid end(s) in the biological material by detecting a co-localisation of the nucleic acid binding protein and the nucleic acid end via binding of the two different binding molecules.

The method for detection of nucleic acid end(s) in a biological material containing nucleic acids may comprise the steps of:
a) incubating the biological material with a proteinase to increase accessibility of the nucleic acid end(s) to the level achieved using proteinase (e.g. proteinase K) for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention provides a method for detection of single stranded nucleic acid end(s) in a biological material containing nucleic acids, wherein the method comprises the steps of:
a) incubating the biological material with a proteinase for less than 60 seconds;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the single stranded nucleic acid end(s).

The invention provides a method for detection of double stranded nucleic acid end(s) in a biological material containing nucleic acids, wherein the method comprises the steps of:
a) incubating the biological material with a proteinase for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the double stranded nucleic acid end(s).

The method for detection of nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The invention provides a method for detection of single stranded nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 60 seconds at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the single stranded nucleic acid end(s).

The invention provides a method for detection of double stranded nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules,
wherein the nucleic acid binding protein binds to the double stranded nucleic acid end(s).

The incubation of the biological material with a proteinase may be performed for less than 100 seconds, less than 90 seconds, less than 80 seconds, less than 70 seconds, or less than 60 seconds, preferably 90 seconds or less.

Thus, the method for detection of nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for 90 seconds or less at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The incubation of the biological material with a proteinase may be performed for between 1 second and less than 2 minutes, between 15 seconds and 90 seconds, between 20 seconds and 80 seconds, between 25 seconds and 70 seconds, between 30 seconds and 60 seconds, preferably incubating the biological material with a proteinase is performed for between 30 seconds and 60 seconds.

The methods, kits and uses described herein may focus on the detection of nucleic acid binding protein(s) which bind specifically to single stranded nucleic acid end(s) (i.e. single stranded nucleic acid breaks, e.g. caused by DNA damage).If the nucleic acid binding proteins which bind to single stranded nucleic acid breaks (e.g. PMS2) are to be detected, the incubation may be performed for between 1 second and less than 90 seconds, between 5 seconds and 75 seconds, between 10 seconds and 60 seconds, or between 15 seconds and 45 seconds. Preferably, the incubation is performed for about 30 seconds.

The methods, kits and uses described herein may focus on the detection of nucleic acid binding protein(s) which bind specifically to double stranded nucleic acid end(s) (i.e. double stranded nucleic acid breaks, e.g. caused by DNA damage). If the nucleic acid binding proteins which bind to double stranded nucleic acid breaks (e.g. RAD51 or RPA) are to be detected, the incubation may be performed for between 1 second and less than 2 minutes, between 10 seconds and 90 seconds, between 20 seconds and 80 seconds, or between 30 seconds and 70 seconds. Preferably, the incubation is performed for about 60 seconds.

The shorter proteinase incubation for nucleic acid binding proteins which specifically bind to single stranded nucleic acid end(s) (e.g. PMS1, PMS2, or MLH1) over double stranded nucleic acid end(s) time is preferred to account for a lower amount of these proteins at the site of nucleic acid end(s) (e.g. nucleic acid damage). The time for proteinase incubation is broadly related to the nature of the specific nucleic acid binding protein; for example, in case of double stranded breaks, multiple molecules of RPA and RAD51 are recruited to the nucleic acid end(s). This number is lower in case of PMS2 proteins which are recruited to single stranded breaks.

The incubation of the biological material with a proteinase may be performed for at least 1 second, at least 5 seconds, preferably at least 10 seconds and more preferably at least 15 seconds.

Thus, the method for detection of nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7µg/mL) for between 1 second and less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

Thus, the method for detection of nucleic acid end(s) in a biological material containing nucleic acids may comprise the steps of:
a) incubating the biological material with a proteinase to increase accessibility of the nucleic acid end(s) to the level achieved using proteinase K at a concentration of 6 µg/mL to 8 µg/mL (e.g. about 7 µg/mL) for between 15 second and 90 seconds at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The incubation of the biological material with a proteinase may be performed for about 30 seconds or about 60 seconds. For single stranded nucleic acid end(s) detection, the step of incubating the biological material with a proteinase is preferably 90 seconds or less, 60 seconds or less, and more preferably still about 30 seconds. For double stranded nucleic acid end(s) detection, the step of incubating the biological material with a proteinase is preferably less than 2 minutes, 90 seconds or less, and more preferably still about 60 seconds.

The incubation of the biological material with a proteinase may be performed at a temperature of between 10 ºC and 55 ºC, between 15 ºC and 55 ºC, between 20 ºC and 55 ºC, between 10 ºC and 35 ºC, or between 15 ºC and 30 ºC, preferably between 20 ºC and 25 ºC. More preferably still, the incubation of the biological material with a proteinase (e.g. proteinase K) is performed at a temperature of about 22 ºC (i.e. room temperature).

The proteinase may be in solution. Preferably, the proteinase is at a concentration of between 0.1 µg/mL and 100 µg/mL, between 1 µg/mL and 20 µg/mL, between 3 µg/mL and 15 µg/mL, between 5 µg/mL and 10 µg/mL, or between 6 µg/mL and 8 µg/mL, preferably, between 6 µg/mL and 8 µg/mL. Preferably, the concentration is below 10 µg/mL. The proteinase may be at a concentration of about 7 µg/mL. The solution may comprise a buffer, such as PBS. The solution may comprise SDS (e.g. 0.2 to 1% SDS), and/or urea (e.g. 1 to 4 M urea). The solution may have a pH of between 4.0 and 12.5, preferably between pH 7.0 and 8.0. For example, if the solution comprises PBS, the pH is about 7.4.

The inventors discovered that incubation of the biological material with proteinase K as described herein (i.e. for less than 2 minutes, preferably at a concentration of 6-8 µg/mL) achieves a more accurate detection of nucleic acid end(s) than when using proteinase K for 2.5 minutes at a concentration of 10 µg/mL.

The proteinase may be a broad spectrum proteinase. The expression "broad spectrum proteinase" is intended to encompass proteinases capable of digesting a wide range of native proteins. One particularly preferred example of a broad spectrum proteinase is proteinase K. The predominant site of cleavage for proteinase K is the peptide bond adjacent to the carboxyl group of aliphatic and aromatic amino acids with blocked alpha amino groups. Any other proteinases with identical or similar mode of action would be considered broad spectrum proteinases within the meaning of the present invention.

The proteinase may be an aspartic protease, a glutamic protease, a metalloprotease, a cysteine protease, a serine protease, or a threonine protease. Preferably the protease is a serine protease, such as proteinase K.

For all aspects and embodiments described herein, preferably, the proteinase is proteinase K.

The incubation of the biological material with a proteinase may be stopped by addition of a proteinase inhibitor. Any suitable proteinase inhibitor may be used. For example, the proteinase inhibitor may be phenylmethylsulfonyl fluoride (PMSF), diisopropylfluorophosphate (DFP), 4-(2-aminoethyl)benzenesulfonyl fluoride (AEBSF), or calbiochem, The proteinase inhibitor may be incubated with the proteinase (which is in contact with the biological material) for at least 30 seconds, at least 1 minute, at least 2 minutes, at least 3 minutes, at least 4 minutes or at least 5 minutes. For example, 1 mM of PMSF may be used for 5 minutes to stop the action of proteinase. After incubation, the proteinase inhibitor may be removed by a wash step (e.g. with a buffer, such as PBS). The incubation of the biological material with a proteinase may be stopped by at least one rinse step.

The invention provides a method for detection of nucleic acid end(s) in a biological material containing nucleic acids, wherein the method comprises the steps of:
a) incubating the biological material with a proteinase for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

The biological material may be or may be derived from an animal, a plant, a protozoan, a bacterium, or a virus. The biological material may comprise a cell or a tissue, or a fragment thereof. The biological material may comprise a live cell or a fixed cell. The method may comprise a step of fixation prior to the step of incubation of the biological material with a proteinase. The step of fixation may comprise incubation of the biological material with ethanol (e.g. 70%(v/v) ethanol). The step of fixation may be performed for between 1 h and24 hrs, 2 hrs and 12 hrs, or 3 hrs and 6 hrs. Preferably, the step of fixation is performed for at least 2 hrs. The step of fixation may be performed for at least 8 hrs. The step of fixation may be performed at a temperature of between -80 ºC and 30ºC or between -40 ºC and 25ºC, preferably about -20 ºC. The method may further comprise a step of attaching the biological material to the solid support before the step of incubation of the biological material with a proteinase. The step of attaching the biological material to the solid support may be performed before or after the step of fixation (if present). Preferably, the solid support is a hydrophilic solid support (i.e. a positively charged solid support and/or a negatively charged solid support). By hydrophilic solid support is meant a solid support that comprises at least one hydrophilic outer surface. Thus, a hydrophilic surface is presented to the biological material resulting in attachment of individual cell(s) to the solid support (via the hydrophilic surface). The hydrophilic solid support may be a slide or a coverslip. The hydrophilic nature of the solid support may result from a coating on the solid support or from the composition of the solid support itself. The coating may provide a positive or negative charged surface but is preferably a positive charge. For example, the solid support may be a TOMO^{®} slide or a poly-L-lysine coated slide, both of which provide a positively charged surface to which cells bind. The solid support may be a silicate uncoated glass slide in some embodiments. The biological material (e.g. the cell) may be in suspension.

The step of attaching the biological material (e.g. the cell) may be performed at a temperature of between 10 ºC and 40 ºC, or between 15 ºC and 37 ºC. For example, the step of attaching the biological material (e.g. the cell) may be performed at a temperate of between 20 ºC and 27 ºC, or between 34 ºC and 40 ºC. The step of attaching the biological material (e.g. the cell) may be performed at a temperature of about 25 ºC or about 37 ºC. The step of attaching the biological material (e.g. the cell) may be performed for between 30 min and 4 hours, or between 1 hour and 3 hours. The step of attaching the biological material (e.g. the cell) is preferably performed for about 2 hours. The step of attaching the biological material (e.g. the cell) may be performed for at least 30 min, at least 1 hour, at least 90 minutes, at least 2 hours, at least 2.5 hours or at least 3 hours. The step of attaching the biological material (e.g. the cell) may be performed in a humidity chamber and/or a CO2 incubator. The step of attaching the biological material (e.g. the cell) may be performed under any conditions in which the cell does not dry out during the attaching step. That is to say that the step of attaching the biological material (e.g. the cell) ensures that the cell or elements of the biological material remain substantially surrounded by liquid throughout the step.

In all embodiments described herein, the nucleic acid may be or may comprise DNA and/or RNA, preferably the nucleic acid is DNA. The nucleic acid end(s) may be a DNA end(s), a RNA end(s), or a hybrid DNA/RNA end(s), preferably the nucleic acid end(s) is a DNA end(s).

The method described herein is sensitive and specific enough to detect single stranded and double stranded nucleic acid end(s). Thus, the nucleic acid end(s) may be single-stranded end(s) and/or double-stranded end(s). The nucleic acid end(s) may be a single stranded nucleic acid break or a double stranded nucleic acid break. The nucleic acid end(s) may be a single stranded gap or a single stranded nick. The nucleic acid end(s) may be a double stranded blunt-end break, or a double stranded 3'-protruding break.

The high sensitivity and/or specificity of the method also allows for the detection of a single nucleic acid end in a cell. Thus, the nucleic acid end(s) may be a single nucleic acid end.

The methods described herein rely on the addition of nucleic acid binding molecules to the biological material under conditions such that the nucleic acid binding molecules bind to the nucleic acid end(s) in the biological material.

The nucleic acid binding molecules used in the methods and kits described herein may be any molecules capable of binding to the end of a nucleic acid molecule. The nucleic acid binding molecules used in the methods and kits described herein can also be bound by binding molecules (e.g. antibodies or fragments thereof) which may be attached (or attachable) to an oligonucleotide molecule. Preferably, a first part of or site on the nucleic acid binding molecules interacts with and/or binds to the nucleic acid end(s) and a second part of or site on the nucleic acid binding molecules interacts with and/or is bound by (one of) the binding molecules. The nucleic acid binding molecules may be selected from a group comprising:
(i) halogenated nucleotide or nucleoside molecules, such as BrdU, IdU, CldU,
(ii) DNA precursor analogues such as EdU (5-Ethynyl-2'-deoxyuridine), F-ara-EdU, 5-Ethynyl-2'-deoxycytidine,
(iii) biotinylated nucleotide molecules,
(iv) ADP-ribose molecules,
(v) protein molecules,
(vi) nucleotide, or nucleoside molecules labelled with labels; optionally wherein the labels are selected from a group comprising fluorescent molecules, or chemiluminescent molecules, or radioisotopes, or enzyme substrates, or biotin molecules.

Preferably, the nucleic acid binding molecules are halogenated nucleotide or nucleoside molecules, DNA precursor analogues, and/or biotinylated nucleotide molecules.

Preferably, the nucleic acid binding molecule(s) is not the same as the nucleic acid binding protein.

The nucleic acid binding molecules may be of the same type or of at least two, at least three, at least four or at least five different types. For example, the nucleic acid binding molecules may be halogenated nucleotides of the same type (e.g. BrdU), or halogenated nucleotides of a different type (e.g. BrdU and IdU). The nucleic acid binding molecules may be halogenated nucleotide and biotinylated nucleotide molecules. The nucleic acid binding molecules may be biotinylated nucleotide molecules of different types (e.g. biotin- ATP, biotin-dGTP, biotin- dUTP, and/or biotin dCTP).

The nucleic acid binding molecules may bind to the nucleic acid end(s) by catalytic or noncatalytic means. The catalytic means may comprise non-enzymatic or enzymatic means. The nucleic acid binding molecules may bind to the nucleic acid end(s) by an enzyme catalysed process of addition, for example, using DNA polymerase I, Terminal deoxynucleotidyl transferase (TdT), Klenow fragment, Phu polymerase, Taq polymerase, T4 DNA Polymerase, T7 DNA Polymerase, T4 Polynucleotide Kinase, RNA polymerases. The non-enzymatic means may comprise chemical factors, for example chemical factors with catalytic properties. The noncatalytic means may comprise physical or biochemical factors.

In embodiments in which the nucleic acid end(s) is a single stranded gap or a single stranded nick, the nucleic acid binding molecules may bind to the nucleic acid end(s) with the help of polymerase I. For example, in this embodiment, the nucleic acid binding molecules may be biotinylated nucleotide molecules. In the context of this embodiment, the proteinase treatment is performed for about 30 seconds.

In embodiments in which the nucleic acid end(s) is a double stranded blunt-end break, or a double stranded 3'-protruding break, the nucleic acid binding molecules may bind to the nucleic acid end(s) with the help of TdT (or a TdT reaction). For example, in this embodiment, the nucleic acid binding molecules may be halogenated nucleotide or nucleoside molecules (e.g. BrdU). In the context of this embodiment, the proteinase treatment is performed for about 60 seconds.

The skilled person would understand that depending on the nucleic acid binding molecule type, the conditions under which the nucleic acid binding molecules bind to the nucleic acid end(s) may be different. For example, if the nucleic acid binding molecules are biotinylated nucleotide molecules, the conditions may require conditions optimal for a polymerase I reaction. For example, a temperature of about 37 ºC. In this embodiment, the biological material may undergo a step of blocking of the endogenous biotin before the biotinylated nucleotide molecules are added. If the nucleic acid binding molecules are halogenated nucleotide or nucleoside molecules (e.g. BrdU), the conditions may require conditions optimal for a TdT reaction, for example, a temperature of about 37 ºC.

The methods described herein rely on the addition of two different binding molecules to the biological material under conditions such that one of the binding molecules binds to the nucleic acid binding molecules bound to the nucleic acid end(s) and the other binding molecule binds to a nucleic acid binding protein that is bound to (either directly or indirectly as defined herein) the nucleic acid at the nucleic acid end(s).

Each of the two different binding molecules may be or may comprise an antibody or fragments thereof, a streptavidin molecule, an avidin molecule, a streptavidin analogue, a biotin molecule, a peptide, a protein, a nucleic acid, an azide, or a polymer. The two different binding molecules may be of the same type or of a different type. For example, the two different binding molecules may be or may comprise:
(i) different antibodies or fragments thereof, such as a monoclonal antibody (or a fragment thereof) and a polyclonal antibody (or a fragment thereof);
(ii) different antibodies or fragments thereof, such as a monoclonal antibody (or a fragment thereof) specific to the nucleic acid binding molecule used in the method, and a monoclonal antibody (or a fragment thereof) specific to the nucleic acid binding protein (i.e. the intrinsic nucleic acid binding protein);
(iii) an antibody or a fragment thereof and a streptavidin,
(iv) an antibody or a fragment thereof and an avidin,
(v) an antibody or a fragment thereof and a protein or peptide; or
(vi) an antibody or a fragment thereof and a biotin molecule.

Preferably, the two different binding molecules are (or comprise) two different monoclonal antibodies (or fragments thereof); one specific for the nucleic acid binding molecule used in the method, and the other specific for the nucleic acid binding protein (i.e. the intrinsic nucleic acid binding protein). Preferably, the two different binding molecules are (or comprise) two different monoclonal antibodies (or fragments thereof) from different species (e.g. a rabbit and a mouse).

The two different binding molecules may also be attached to an oligonucleotide molecule. For example, each one of the two different binding molecules may be attached to an oligonucleotide molecule. Preferably, the oligonucleotide molecules of the two different binding molecules are different (e.g. have different sequences). Preferably, the two different binding molecules are attached to a different oligonucleotide molecule. Each oligonucleotide molecule may be covalently linked to each of the the two different binding molecules.

The term "different" as used herein in the context of two different binding molecules means that each of the two binding molecules binds to a different target molecule from the other of the two binding molecule. Specifically, in the methods, kits and uses described herein, one of the two different binding molecules binds to a nucleic acid binding molecule and the other of the two different binding molecules binds to the nucleic acid binding protein. The nucleic acid binding protein and the nucleic acid binding molecule are not the same molecules. The two different binding molecules may be molecules of the same type, e.g. monoclonal antibodies (e.g. mouse monoclonal antibodies), but with specificity for different molecules. Alternatively, the two different binding molecules may be molecules of a different type, e.g. a monoclonal antibody and a biotin molecule. Preferably the two different binding molecules are monoclonal antibodies.

The two different binding molecules may comprise molecular probes. The molecular probes may be fluorophores and/or dyes. The two different binding molecules may comprise components of the Fluorogen Activating Protein (FAP) - Dye Activated by Proximal Anchoring (DAPA) system (Carpenter et al., 2020, "Protein Proximity Observed Using Fluorogen Activating Protein and Dye Activated by Proximal Anchoring (FAP-DAPA) System"). For example, one of the two different binding molecules may comprise a FAP and the other of the two different binding molecules may comprise a HaloTag receptor. The two different binding molecules may further comprise antibodies which specifically bind to either nucleic acid binding molecules or a nucleic acid binding protein. Thus, for example, one of the different binding molecules may be a fusion protein of HaloTag receptor and an antibody against the nucleic acid binding protein, and the other of the different binding molecules may be a fusion protein of FAP and an antibody against the nucleic acid binding molecules. The two different binding molecules may comprise two complementing fragments of the split YFP Venus protein (Harmon et al. 2017, "A Bi-fluorescence complementation system to detect associations between the Endoplasmic reticulum and mitochondria"). The two different binding molecules may further comprise antibodies which specifically bind to either nucleic acid binding molecules or a nucleic acid binding protein. Thus, for example, one of the different binding molecules may be a fusion protein of N-terminal end of the Venus protein and an antibody against the nucleic acid binding protein, and the other of the different binding molecules may be a fusion protein of the C-terminal end of the Venus protein and an antibody against the nucleic acid binding molecules. Each of the two different binding molecules may comprise a nanobody, aptamer or an antibody attached to one of the two oligonucleotide molecules (i.e. one of the two different binding molecules may be attached to one of the two oligonucleotide molecules and the other of the two different binding molecules may be attached to the other oligonucleotide molecule). The two oligonucleotide molecules may serve as a template for a branched DNA signal amplification in a branched proximity hybridization assay. The two different binding molecules may comprise a donor and an acceptor FRET probes, respectively.

The skilled person would understand that depending on the type of the two different binding molecules, the conditions under which the two different binding molecules bind to the nucleic acid binding molecules and nucleic acid binding proteins may be different.

For example, if the two different binding molecules are monoclonal antibodies, the two different binding molecules may be incubated with the biological material for at least 30 minutes, preferably 60 minutes. The two different binding molecules may be incubated with the biological material at a temperature of between 15ºC and 30 ºC, preferably between 20ºC and 25ºC.

The step of adding two different binding molecules to the biological material may include adding the two different binding molecules separately or together. Preferably, the two different binding molecules are added separately. Thus, one of the two different binding molecules may be added first and the other different binding molecule may be added second. Preferably, the binding molecule that binds to the nucleic acid binding protein (i.e. the intrinsic nucleic acid binding protein) is added first and the binding molecules that binds to the nucleic acid binding molecules is added second.

The step of adding two different binding molecules to the biological material may include the steps of:
i. adding one of the two different binding molecules to the biological material
ii. incubating the one of the two different binding molecules with the biological material for at least 30 min, preferably 60 min;
iii. optionally, performing a wash step to remove any unbound binding molecule;
iv. adding the other of the two different binding molecules to the biological material;
v. incubating the other of the two different binding molecules with the biological material for at least 30 min, preferably 60 min; and
vi. optionally, performing a wash step to remove any unbound binding molecule.

The step of adding two different binding molecules to the biological material may include the steps of:
i. adding one of the two different binding molecules to the biological material, wherein the different binding molecule is specific for the nucleic acid binding protein (i.e. the intrinsic nucleic acid binding protein);
ii. incubating the one of the two different binding molecules with the biological material for at least 30 min, preferably 60 min
iii. optionally, performing a wash step to remove any unbound binding molecule
iv. adding the other of the two different binding molecules to the biological material, wherein the different binding molecule is specific for the nucleic acid binding molecules;
v. incubating the other of the two different binding molecules with the biological material for at least 30 min, preferably 60 min; and
vi. optionally, performing a wash step to remove any unbound binding molecule.

The methods described herein rely on the presence of nucleic acid binding proteins in the biological material. Thus, the methods described herein rely on the detection of intrinsic nucleic acid binding proteins bound to the nucleic acids at the nucleic acid end(s). Thus, more specifically, the methods described herein rely on the detection of a co-localization of the intrinsic nucleic acid binding protein and the nucleic acid end in the biological material.

As explained further herein, the nucleic acid binding protein may be bound to the nucleic acid at the nucleic acid end(s) either directly or indirectly. It will be understood by the skilled person that the detection method described herein relies on the presence of a nucleic acid binding protein bound to the nucleic acid at the nucleic acid end(s). In order for the detection to work, the nucleic acid binding protein must be close enough to the nucleic acid at the nucleic acid end(s) so that the detection of a co-localisation of the nucleic acid binding protein and the nucleic acid end via binding of the two different binding molecules can take place.

The nucleic acid binding protein may be bound to the nucleic acid at the nucleic acid end(s) indirectly via interaction with an adaptor protein. The nucleic acid binding protein may be bound to the nucleic acid at the nucleic acid end(s) indirectly as part of a protein complex. For example, XRCC1 protein binds to the nucleic acid indirectly through interactions with PARP1. For example, RAD51 and RPA bind to the nucleic acid directly.

Thus, since the nucleic acid binding protein should have the ability to interact with or bind to the nucleic acid (either directly or indirectly), the nucleic acid binding protein may be a nucleic acid repair protein. The nucleic acid binding protein may be a protein involved in repair of nucleic acid breaks. Preferably, the nucleic acid binding protein is bound to the nucleic acid end(s) (i.e. nucleic acid breaks) in the biological material.

The nucleic acid binding protein may be a protein involved in homologous recombination of the nucleic acid (e.g. DNA) during double strand break repair. Thus, the nucleic acid binding protein may be RAD51.

The nucleic acid binding protein may be a protein involved in responding to nucleic acid (e.g. DNA) damage. Thus, the nucleic acid binding protein may be Replication protein A (RPA) (e.g. RPA70).

The nucleic acid binding protein may be a protein involved in repairing errors in nucleic acid (e.g. DNA) e.g. introduced during DNA replication. Thus, the nucleic acid binding protein may be PMS2, PMS1 or MLH1.

The nucleic acid binding protein may be p53, MSH2, Ataxia telangiectasia and Rad3 related protein, ATM serine/threonine kinase, RAD52, XRCC1, Proliferating cell nuclear antigen, XPC, Ku70, Ku80, Nibrin, DDB2, Bloom syndrome protein, CHEK2, RAD51C, DNA polymerase eta, Rad50, DDB1, RBBP8, FANCB, PALB2, H2AX, DNA repair and recombination protein RAD54-like, PrimPol, REV1, Terminal deoxynucleotidyl transferase, DNA polymerase nu, Fanconi anemia, complementation group C, FANCF, ERCC8, Artemis, Ubiquitin ligase, RNF4, TP53BP1, AP endonuclease, ERCC4, Transcription factor II H, XRCC3, XRCC2, RecA, ERCC6, SLX4, Sirtuin 1, PTEN, Replication protein A2, Replication protein A3, Alkb homolog 3, alpha-ketoglutaratedependent dioxygenase, Exonuclease 5, DNA polymerase alpha catalytic subunit, Cyclin H, or PARP1/2.

The nucleic acid binding protein may comprise at least one post-translational modification. The post-translation modification may be amino acid phosphorylation, amino acid acetylation, amino acid glycosylation, or amino acid methylation. For example, the nucleic acid binding protein may be the phosphorylated form of H2AX (known as yH2AX).

The nucleic acid binding protein may be a nucleic acid (e.g. DNA) repair protein. The nucleic acid binding protein may be ATM, ATR, RPA, RAD51, MRE11, RAD17, RAD9A, RAD1, HUS1, TOPBP1, SMUG1, OGG1, PARP1, PARP2, PARP3, PARG, MGMT, TDP1, TDP2, MSH2, MSH3, MSH6, MLH1, PMS2, MSH4, MSH5, MSH3, PMS1, XPC, XPA, DDB1, DDB2, TFIIH, ERCC3, ERCC2, XRCC1, ERCC1, LIG1, RAD51B, RAD51D, HELQ, RAD52, BRCA1, SHLD1, SHLD2, MUS81, FANCA, FANCB, FANCC, LIG4, XRCC5, XRCC6, Ku70, Ku80, DNPH1, POLA, POLB, POLD, POLE, REV3L, POLO, FEN1, TREX1, TREX2, EXO1, APTX1, HERC2, RNF8, RNF4, H2AX, BLM, WRN, RECOL4, ATRIP, PCNA, TP53, RIF1, TOPBP2.

The nucleic acid binding protein may be a protein involved in replication. The nucleic acid binding protein may be PCNA, RFC, RFA, Topo I, Topo II, polymerase a, polymerase d.

The nucleic acid binding protein may be a DNA repair related factor, expressed by a gene: UNG, SMUG1, MBD4, TDG, OGG1, MUTYH (MYH), NTHL1 (NTH1), MPG, NEIL1, NEIL2, NEIL3, APEX1 (APE1), APEX2, LIG3, XRCC1, PNKP, APLF, HMCES, PARP1 (ADPRT), PARP2 (ADPRTL2), PARP3 (ADPRTL3), PARG, PARPBP, MGMT, ALKBH2 (ABH2), ALKBH3 (DEPC1), TDP1, TDP2 (TTRAP), SPRTN (Spartan), MSH2, MSH3, MSH6, MLH1, PMS2, MSH4, MSH5, MLH3, PMS1, PMS2P3 (PMS2L3), HFM1, XPC, RAD23B, CETN2, RAD23A, XPA, DDB1, DDB2 (XPE), RPA1, RPA2, RPA3, TFIIH, ERCC3 (XPB), ERCC2 (XPD), GTF2H1, GTF2H2, GTF2H3, GTF2H4, GTF2H5 (TTDA), GTF2E2, CDK7, CCNH, MNAT1, ERCC5 (XPG), ERCC1, ERCC4 (XPF), LIG1, ERCC8 (CSA), ERCC6 (CSB), UVSSA (KIAA1530), XAB2 (HCNP), MMS19, RAD51, RAD51 B, RAD51D, HELQ (HEL308), SWI5, SWSAP1, ZSWIM7 (SWS1), SPIDR, PDS5B, DMC1, XRCC2, XRCC3, RAD52, RAD54L, RAD54B, BRCA1, BARD1, ABRAXAS1, PAXIP1 (PTIP), SMC5, SMC6, SHLD1, SHLD2 (FAM35A), SHLD3, SEM1 (SHFM1) (DSS1), RAD50, MRE11A, NBN (NBS1), RBBP8 (CtIP), MUS81, EME1 (MMS4L), EME2, SLX1A (GIYD1), SLX1B (GIYD2), GEN1, FANCA, FANCB, FANCC, BRCA2 (FANCD1), FANCD2, FANCE, FANCF, FANCG (XRCC9), FANCI (KIAA1794), BRIP1 (FANCJ), FANCL, FANCM, PALB2 (FANCN), RAD51C (FANCO), SLX4(FANCP), FAAP20 (C1orf86), FAAP24 (C19orf40), FAAP100, UBE2T (FANCT), XRCC6 (Ku70), XRCC5 (Ku80), PRKDC, LIG4, XRCC4, DCLRE1C (Artemis), NHEJ1 (XLF, Cernunnos), NUDT1 (MTH1), DUT, RRM2B (p53R2), PARK7 (DJ-1), DNPH1, NUDT15 (MTH2), NUDT18 (MTH3), POLA1, POLB, POLD1, POLD2, POLD3, POLD4, POLE (POLE1), POLE2, POLE3, POLE4, REV3L (POLZ), MAD2L2 (REV7), REV1 (REV1L), POLG, POLH, POLI (RAD30B), POLO, POLK (DINB1), POLL, POLM, POLN (POL4P), PRIMPOL, DNTT, FEN1 (DNase IV), FAN1 (MTMR15), TREX1, TREX2, EXO1 (HEX1), APTX (aprataxin), SPO11, ENDOV, DNA2, DCLRE1A (SNM1A), DCLRE1B (SNM1B), EXO5, UBE2A (RAD6A)UBE2B (RAD6B), RAD18, SHPRH, HLTF (SMARCA3), RNF168, RNF8, RNF4, UBE2V2 (MMS2), UBE2N (UBC13), USP1, WDR48, HERC2, H2AX (H2AFX), CHAF1A (CAF1), SETMAR (METNASE), ATRX, BLM, RMI1, TOP3A, WRN, RECOL4, ATM, MPLKIP (TTDN1), RPA4, PRPF19 (PSO4), RECOL (RECQ1), ATR, ATRIP, MDC1, PCNA, RAD1, RAD9A, HUS1, RAD17 (RAD24), CHEK1, CHEK2, TP53, TP53BP1 (53BP1), RIF1, TOPBP1, CLK2, or PER1.

In embodiments in which the nucleic acid end(s) is a single stranded gap or a single stranded nick, the nucleic acid binding molecules may bind to the nucleic acid end(s) via polymerase I catalysis. For example, in this embodiment, the nucleic acid binding molecules may be biotinylated nucleotide molecules and thus the nucleic acid end(s) is/are extended by addition of nucleotide molecules. In this embodiment, for example, the nucleic acid binding protein may be PMS2, PMS1 or MLH1. The two different binding molecules in this example may be an anti-PMS2 (or anti-PMS1 or anti-MLH1) monoclonal antibody and an anti-biotin monoclonal antibody. Thus, the method for detection of nucleic acid end(s) in a biological material containing nucleic acids may comprise the steps of:
a) incubating the biological material with a proteinase for less than 60 seconds;
b) adding biotinylated molecules and polymerase I to the biological material under conditions such that the biotinylated molecules bind to the nucleic acid end(s) in the biological material;
c) adding an anti-PMS2 (or anti-PMS1 or anti-MLH1) monoclonal antibody and an anti-biotin monoclonal antibody to the biological material under conditions such that the anti-PMS2 monoclonal antibody binds to the biotinylated molecules bound to the nucleic acid end(s) and the anti-PMS2 (or anti-PMS1 or anti-MLH1) monoclonal antibody binds to PMS2 (or PMS1 or MLH1) that is (indirectly) bound to the nucleic acid at the nucleic acid end(s); and
d) detecting the nucleic acid end(s) in the biological material by detecting a co-localisation of PMS2 (or PMS1 or MLH1) and the nucleic acid end via binding of the two monoclonal antibodies.

In embodiments in which the nucleic acid end(s) is a double stranded blunt-end break, or a double stranded 3'-protruding break, the nucleic acid binding molecules may bind to the nucleic acid end(s) via Terminal deoxynucleotide Transferase (TdT, or a TdT reaction) catalysis. For example, in this embodiment, the nucleic acid binding molecules may be halogenated nucleotide or nucleoside molecules (e.g. BrdU) and thus the nucleic acid end(s) is/are extended by addition of nucleotide molecules. In this embodiment, for example, the nucleic acid binding protein may be RAD51 or RPA (e.g. RPA70). The two different binding molecules in this example may be an anti-RAD51 (or anti-RPA) monoclonal antibody and an anti-BrdU monoclonal antibody. Thus, the method for detection of nucleic acid end(s) in a biological material containing nucleic acids may comprise the steps of:
a) incubating the biological material with a proteinase for less than 2 minutes;
b) adding BrdU and TdT to the biological material under conditions such that the BrdU binds to the nucleic acid end(s) in the biological material;
c) adding an anti-RAD51 (or anti-RPA) monoclonal antibody and an anti-BrdU monoclonal antibody to the biological material under conditions such that the anti-BrdU monoclonal antibody binds to BrdU bound to the nucleic acid end(s) and the anti-RAD51 (or anti-RPA) monoclonal antibody binds to RAD51 (or RPA) that is bound to the nucleic acid at the nucleic acid end(s); and
d) detecting the nucleic acid end(s) in the biological material by detecting a co-localisation of RAD51 (or RPA) and the nucleic acid end via binding of the two monoclonal antibodies.

The method may further comprise a step of adding further binding molecules that bind to the two different binding molecules after the step of adding the two different binding molecules. In this embodiment, the two different binding molecules may not be attached to oligonucleotide molecules. In this embodiment, the further binding molecules may be attached to an oligonucleotide molecule and thus the further binding molecules link the oligonucleotide molecules to the two different binding molecules. Preferably, the further binding molecules are two different further binding molecules. In the method described herein, each of the two different further binding molecules may be attached to a different oligonucleotide molecule (i.e. an oligonucleotide molecule of a different sequence).

Thus, the method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s);
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein;
e) detecting the nucleic acid end(s) in the biological material by detecting a co-localisation of the nucleic acid binding protein and the nucleic acid end via binding of the two different binding molecules and the two further binding molecules.

As used herein the term "different" in the context of two further binding molecules means that each of the two further binding molecules binds to one but not the other of the binding molecules. Thus, each of the further different molecules may be specific for a different binding molecule. The further binding molecules may be of the same type (e.g. monoclonal binding antibodies, each specific for a different binding molecule), or a different type. Preferably the two different further binding molecules are monoclonal antibodies; one is an antibody that binds to the binding molecule bound to the nucleic acid binding molecules bound to the nucleic acid end(s) and the other is an antibody that binds to the binding molecule bound to the nucleic acid binding protein that is bound to the nucleic acid at the nucleic acid end(s).

The further binding molecules may comprise an antibody or a fragment thereof, a streptavidin molecule, an avidin molecule, a biotin molecule, a protein a peptide, a nucleic acid, an azide, or a polymer. The further binding molecules may be at least two different further binding molecules (e.g. two different antibodies, one of each specific for one of the two different binding molecules). For example, the two different binding molecules that bind to the nucleic acid binding molecule and the nucleic acid binding protein, respectively, may be primary antibodies or fragments thereof and the further binding molecules may be secondary antibodies or fragments thereof that specifically target the primary antibodies. For example, the two different binding molecules that bind to the nucleic acid binding molecule may be mouse and rabbit primary antibodies, and the further binding molecules may be anti-mouse and anti-rabbit secondary antibodies. The further binding molecules may be directly attached (e.g. conjugated) to an oligonucleotide molecule. Preferably, each of the two further binding molecules is directly attached (e.g. conjugated) to a different oligonucleotide molecule (i.e. an oligonucleotide molecule of a different sequence).

The method may further comprise, after the step of adding two different binding molecules (or after the step of adding the further binding molecules), a step of adding further oligonucleotide molecules which hybridise with the oligonucleotide molecules attached to the two different binding molecules (or to the further binding molecules) to form a circular amplification template.

The further oligonucleotide molecules may have the same sequence or at least two different sequences. The further oligonucleotide molecules may hybridize to two oligonucleotide molecules attached to the two different binding molecules (or the further binding molecules) to form a circular template. The two further oligonucleotide molecules may be ligated together to form a circular template. Thus, the method may further comprise a step of ligation of two further oligonucleotide molecules. The method may further comprise a step of extension of the two further oligonucleotide molecules followed by a step of ligation. Preferably, the further oligonucleotide molecules have at least two different sequences. The circular template may comprise or consist of the two further oligonucleotide molecules. The circular template may further comprise an extension product of the two further oligonucleotide molecules. The circular template is suitable for rolling circle amplification. The circular template may be formed by ligation of the further oligonucleotide molecules (or the two different further oligonucleotide molecules).

The method may further comprise a step of performing nucleic acid amplification to produce an amplification product before the step of detecting the nucleic acid end(s). Thus, detecting the nucleic acid end(s) may comprise a step of performing nucleic acid amplification to produce an amplification product that is detected. The nucleic acid amplification may be rolling circle amplification.

Thus, the method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to a different oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to a different oligonucleotide molecule;
d) adding two different further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of about 22 ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to an oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to a different oligonucleotide molecule;
d) adding further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (about 7 µg/mL) for less than 2 minutes at a temperature for about 22 ºC;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s), wherein each of the two different binding molecules is attached to a different oligonucleotide molecule;
d) adding two different further oligonucleotide molecules which hybridise with the oligonucleotide molecules attached to the two different binding molecules to form a circular amplification template;
e) performing nucleic acid amplification to produce an amplification product; and
f) detecting the amplification product to detect the nucleic acid end(s) in the biological material.

Thus, the method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to an oligonucleotide molecule;
e) adding further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to a different oligonucleotide molecule;
e) adding further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) in the biological material without significantly affecting the levels of a nucleic acid binding protein in the biological material;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to a different oligonucleotide molecule;
e) adding two different further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (about 7 µg/mL) for less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to an oligonucleotide molecule;
e) adding further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (about 7 µg/mL) for less than 2 minutes at a temperature of about 22ºC;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to a different oligonucleotide molecule;
e) adding further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

The method for detection of nucleic acid end(s) in a biological material containing nucleic acids may comprise the steps:
a) incubating the biological material with a proteinase to increase accessibility of the nucleic acid end(s) to the level achieved using proteinase K at a concentration of 6 µg/mL to 8 µg/mL (e.g. about 7 µg/mL) for less than 2 minutes at a temperature of about 22ºC (i.e. room temperature);
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s),
d) adding two different further binding molecules to the biological material, wherein one of the two different further binding molecules binds to the binding molecule bound to the nucleic acid binding molecules, and the other of the two different further binding molecules binds to the binding molecules bound to the nucleic acid binding protein, wherein each of the two different further binding molecules is attached to a different oligonucleotide molecule;
e) adding two different further oligonucleotide molecules which hybridise with the oligonucleotide molecules attached to the two different further binding molecules to form a circular amplification template;
f) performing nucleic acid amplification to produce an amplification product; and
g) detecting the amplification product to detect the nucleic acid end(s) in the biological material.

In the methods described herein, the detection of the co-localisation of the nucleic acid binding protein and the nucleic acid end may comprise detection of the amplification product, a proximity ligation assay, or a proximity-driven reaction with fluorophores or dyes. The detection of the co-localisation of the nucleic acid binding protein and the nucleic acid end may comprise detection of a branched probe or a FRET probe.

The step of detecting the nucleic acid end(s) in the biological material (e.g. the detection of the amplification product) may be performed using microscopy, methods of automated analysis of high cell numbers, spectroscopy, fluorescence microscopy (wide field, confocal, multifocal, super-resolution, microscopy with catapulting, laser scanning, high throughput, high content), fluorimetry, transmitted light optical microscopy for absorption detection, flow cytometry, cell sorting (FACS), and/or mass spectrometry. The step of detecting the nucleic acid end(s) in the biological material (e.g. the detection of the amplification product) may include adding a detection molecule, for example, a labelled molecule capable of recognizing and binding to the amplification product, or to two binding molecules or two further binding molecules. For example, the labelled molecule may bind to the further oligonucleotide molecule sequence. The labelled molecule may comprise a nucleic acid. The labelled molecule may comprise a barcode or a tag.

The detection of the co-localisation of the nucleic acid binding protein and the nucleic acid end may by performed by photoactivation of the binding molecules described herein, or by photoactivation of probes attached to the amplification product. The detection of the co-localisation of the nucleic acid binding protein and the nucleic acid end may by performed by immunocytochemical analysis using an antibody (e.g. polyclonal anti-serum) against GFP that recognises both fragments of a split protein. The detection of the co-localisation of the nucleic acid binding protein and the nucleic acid end may by performed by fluorescence analysis of activated fluorophores or fluorescence analysis of probes or tags attached to the amplification product. The detection of the co-localisation of the nucleic acid binding protein and the nucleic acid end may by performed by detection of the amplification product (e.g. by using a tag, e.g. a fluorescent tag). The detection of the co-localisation of the nucleic acid binding protein and the nucleic acid end may by performed by proximity-driven S_{N}Ar reactions of lysine-linked fluorophores (Hymel et al, 2014, "Detection of Protein-Protein Interactions by Proximity-Driven SNAr Reactions of Lysine-Linked Fluorophores"). The detection of the co-localisation of the nucleic acid binding protein and the nucleic acid end may by performed by detection of FRET probes. Thus, one of the two different binding molecules (i.e. the donor probe) may be excited by an external light source and transfer its energy to the other different binding molecule (i.e. the acceptor probe).

The excited acceptor probe may emit light of a different (usually longer) wavelength, which can be detected and measured. The detection of the co-localisation of the nucleic acid binding protein and the nucleic acid end may by performed by a branched proximity hybridization assay to detect branched probes (e.g. DNA probes).

Preferably, in the methods described herein, the detection is performed *in situ.*

The methods described herein may comprise one or more further steps before the step of incubation of the biological material with a proteinase. The method may comprise a step of permeabilization of the biological material (e.g. a cell). The method may comprise a further step of increasing accessibility of nucleic acid end(s). The method may comprise a step of blocking nonspecific binding site(s) for at least one of the molecules used in the method. The method may comprise a step of modification of nucleic acid (DNA) ends in the biological material. The step of permeabilization of the biological material (e.g. a cell) may include chemical and/or physical procedures which compromise the integrity of the biological material (e.g. cell) in order to allow or facilitate access of the molecules used in the method to the nucleic acid and the nucleic acid binding protein in the biological material (e.g. cell).

The further step of increasing accessibility of nucleic acid end(s) may be performed in the methods described herein in addition to the step of incubating the biological material with a proteinase. The further step of increasing accessibility of nucleic acid end(s) may further facilitate access of the molecules used in the methods to the nucleic acid end(s) in the biological material (e.g. cell).

The step of blocking nonspecific binding site(s) for at least one of the molecules used in the method may comprise blocking of all nonspecific binding sites of the molecules used in the method. The term "blocking" means a procedure leading to prevention of binding of critical molecules used in the assay, for instance, antibodies, to molecular targets other than the ones for which the selected molecules exhibit affinity useful in the assay. A typical example of blocking is a procedure used in antibody detection (immunodetection) of a molecule of interest in biological material. The antibody is directed against a well-defined antigen but can also bind to various cell components via weak chemical interactions. This nonspecific binding is minimized by prior addition of blocking agents (such as albumin) that occupy such binding sites. Thus, the methods described herein may further comprise a step of blocking nonspecific binding site(s) of molecules after the step of adding two different binding molecules to the biological material and/or the step of adding further binding molecules.

The step of modification of nucleic acid (DNA) ends in the biological material may be performed after the step of the proteinase treatment and before the step of adding nucleic acid binding molecules. The step of modification of nucleic acid ends may be performed by chemical or physical means. For example, the modification of nucleic acid ends may be performed by T4 polynucleotide kinase.

The methods described herein may comprise one or more steps of washing. For example, the proteinase and/or the proteinase inhibitor may be washed away after the step of incubation of the biological material with proteinase. The step of washing may be performed after the step of adding nucleic acid binding molecules. The step of washing may be performed after the step of adding two different binding molecules. The step of washing may be performed after the step of adding further oligonucleotide molecules. The step of washing may be performed after the step of performing amplification. The step of washing removes unbound material which may produce a clear detection signal and/or less background noise.

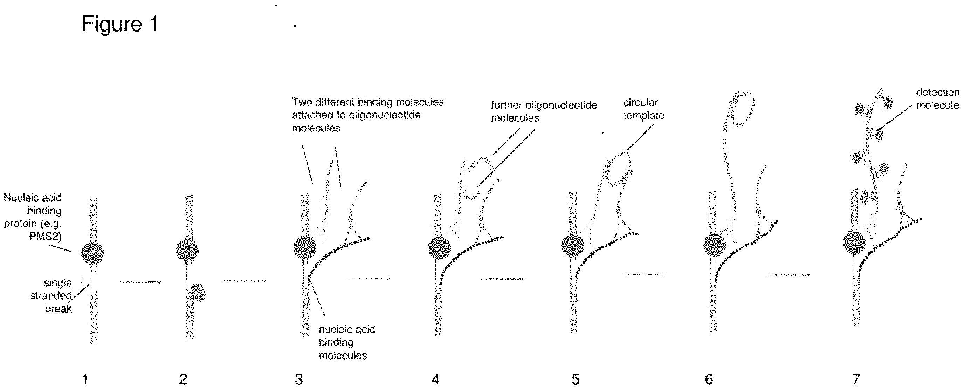

Figure 1 shows an exemplary method of the invention after the step of the proteinase treatment. In step 1, a single stranded nucleic acid (e.g. DNA) break is created. The intrinsic nucleic acid binding protein (e.g. PMS2, PMS1 or MLH1) binds to the nucleic acid at the nucleic acid end to, for example, fix the created break. In step 2, nucleic acid binding molecules binds to the nucleic acid end. This can be done with the help of an enzyme, for example, polymerase I (marked with grey circle in step 2). This creates a chain of the nucleic acid binding molecules (visualised in step 3). In step 3, two different binding molecules (attached to the oligonucleotide molecules) bind to the nucleic acid binding protein and the nucleic acid binding molecules, respectively. In step 4, further oligonucleotide molecules hybridize to the oligonucleotide molecules attached to the two different binding molecules. In step 5, the further oligonucleotide molecules ligate to form a circular template. In step 6, rolling circle amplification is performed on the circular template to produce an amplified product. In step 7, detection molecules (e.g. fluorescent probes) bind to the amplification product to allow detection of the amplification product.

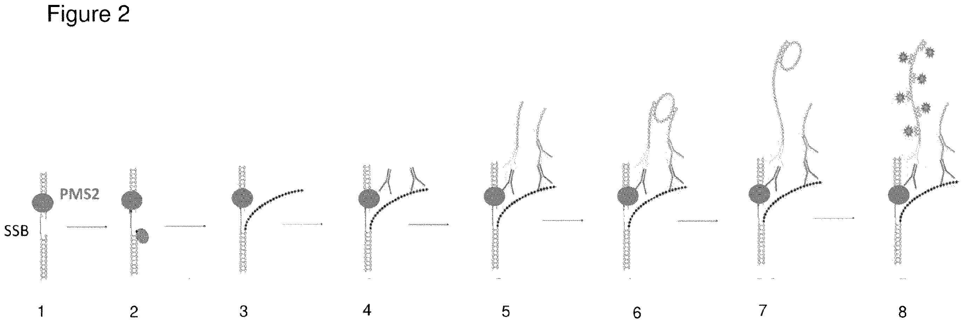

Figure 2 shows another example of the method of the invention. Here, the method steps are as in Figure 1 with the exception that further binding molecules (which are attached to the oligonucleotide molecules) bind to the two different binding molecules (which in this example are not attached to the oligonucleotide molecules).

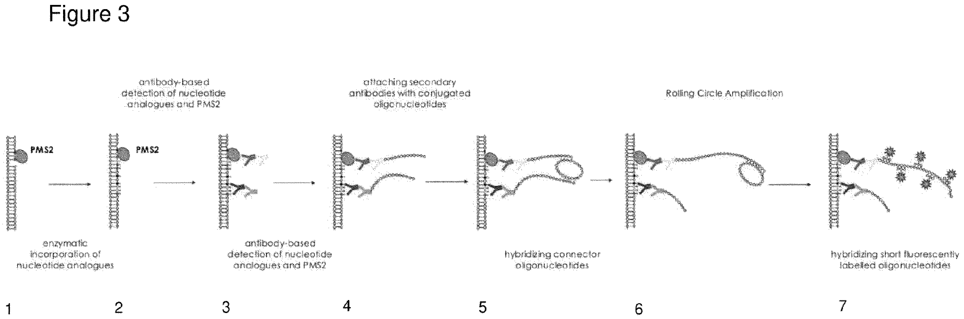

Figure 3 shows another example of the method of the invention. The steps are as in Figure 1. However, here, the nucleic acid binding molecules (e.g. nucleotide analogues) are directly incorporated into the single stranded nucleic acid (e.g. DNA) break. Thus, in this example, a chain of nucleic acid binding molecules is not formed.

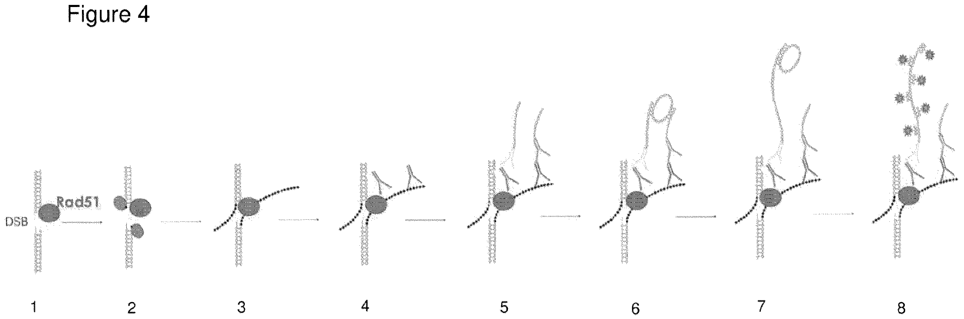

Figure 4 shows another example of the method of the invention. In this example, the nucleic acid break is a double-stranded nucleic acid break. The intrinsic nucleic acid binding protein (e.g. Rad51 or RPA) binds to the nucleic acid at the nucleic acid end to, for example, fix the created break. In step 2, nucleic acid binding molecules binds to the nucleic acid end. This can be done with the help of an enzyme, for example, TdT (marked with grey circle in step 2). This creates a chain of the nucleic acid binding molecules (visualised in step 3). In step 4, two different binding molecules bind to the nucleic acid binding protein and the nucleic acid binding molecules, respectively. In step 5 (which is optional), further binding molecules attached to oligonucleotide molecules bind to the two different binding molecules. In step 6, further oligonucleotide molecules hybridize to the oligonucleotide molecules attached to the further binding molecules and are ligated to form a circular template. In step 7, rolling circle amplification is performed on the circular template to produce an amplified product. In step 8, detection molecules (e.g. fluorescent probes) bind to the amplification product to allow detection of the amplification product.

The method described herein enables direct detection and visualization of nucleic acid end(s). The method described herein enables marking the presence and position of a single nucleic acid strand break. Thus, the method is particularly useful in detecting nucleic acid damage which has broad utility. For example, the method may be used for assessing the quality of DNA in the subject. The method may also be used to study the mechanism of DNA damage induction, sending and repair. In the context of cancer cells, the method may be particularly useful in assessing effectiveness of a therapeutic agent.

Thus, in one aspect, the invention provides a method for assessing effectiveness of a therapeutic agent, the method comprising the steps:
a) performing the detection method as described herein on a sample obtained from a subject before the administration of the therapeutic agent,
b) performing the detection method as described herein on a sample obtained from the subject after the administration of the therapeutic agent,
c) comparing the amount of the nucleic acid end(s) detected in steps a) and b), wherein the therapeutic agent is effective if the amount of the detected nucleic acid end(s) is greater in step a) than step b),
wherein steps a) and b) can be performed in any order.

The subject may be an animal, preferably the subject is human.

Preferably, the nucleic acid end(s) are DNA end(s). More preferably still, the nucleic acid end(s) are single stranded nucleic acid (e.g. DNA) ends.

The therapeutic agent may be a cancer drug or drug candidate. The method may be used to assess therapeutic effectiveness of a drug candidate. The therapeutic agent may target a protein involved in a mismatch repair (MMR) pathway. Thus, the therapeutic agent may target PMS1, PMS2, and/or MLH1. Thus, in the detection methods described herein, the nucleic acid binding protein may be PMS1, PMS2 or MLH1. The therapeutic agent may inhibit a protein involved in a mismatch repair pathway. Thus, once inhibited, the protein will not be able to bind to the nucleic acid end(s) and therefore the detection method shows reduced number of the detected nucleic acid end(s) in the biological material.

The sample may be obtained from the subject at least 1 hour, 6 hours, 12 hours, 18 hours, 24 hours, 2 days, 3 days, 4 days, 5 days, 6 days, 1 week, 2 weeks, 3 weeks or 4 weeks after the administration of the therapeutic agent. The method may include performing the method for detection of nucleic acid end(s) at multiple time points after the administration of the therapeutic agent. This approach can assess a long-term effect of a therapeutic agent.

In one aspect, the invention provides a method for assessing effectiveness of a therapeutic agent, the method comprising the steps:
a) performing the detection method as described herein on a sample obtained from a subject before the administration of the therapeutic agent,
b) performing the detection method as described herein on a sample obtained from the subject after the administration of the therapeutic agent,
c) comparing the amount of the nucleic acid end(s) detected in steps a) and b), wherein the therapeutic agent is effective if the amount of the nucleic acid end(s) is greater in step b) than step a),
wherein steps a) and b) can be performed in any order.

The subject may be an animal, preferably the subject is human.

Preferably, the nucleic acid end(s) are DNA end(s). More preferably still, the nucleic acid end(s) are double stranded nucleic acid (e.g. DNA) ends.

The therapeutic agent may be a cancer drug or drug candidate. The method may be used to assess therapeutic effectiveness of a drug candidate. The therapeutic agent may target a protein involved in repairing errors in nucleic acid (e.g. DNA, or a protein involved in homologous recombination of the nucleic acid (e.g. DNA) during double strand break repair. Thus, the therapeutic agent may target RPA or Rad51. The therapeutic agent may inhibit DNA synthesis, cause nucleic acid damage, and/or inhibits DNA damage repair.

The sample may be obtained from the subject at least 1 hour, 6 hours, 12 hours, 18 hours, 24 hours, 2 days, 3 days, 4 days, 5 days, 6 days, 1 week, 2 weeks, 3 weeks or 4 weeks after the administration of the therapeutic agent. The method may include performing the method for detection of nucleic acid end(s) at multiple time points after the administration of the therapeutic agent. This approach can assess a long-term effect of a therapeutic agent.

The sample may be obtained from a cancer biopsy. The sample may comprise a cancer cell.

The method described herein may also be useful for assessing nucleic acid damage caused by an agent (e.g. a chemical or an environmental agent).

Thus, in an aspect, the invention a method for assessing nucleic acid damage caused by an agent, the method comprising the steps:
a) performing the detection method as described herein on a sample obtained from a subject before administration of, or exposure to, an agent,
b) performing the detection method as described herein on a sample obtained from the subject after the administration of, or exposure to, the agent,
c) comparing the amount of the nucleic acid end(s) detected in steps a) and b), wherein the agent causes nucleic acid damage if the amount of the nucleic acid end(s) is greater in step b) than step a),
wherein steps a) and b) can be performed in any order.

The nucleic acid damage may be a single strand nucleic acid break or a double strand nucleic acid break. Preferably, the nucleic acid end(s) are DNA end(s). Thus, the nucleic acid damage maybe a single stranded DNA break or a double-stranded DNA break. In the context of single stranded breaks, preferably, the method relies on the detection of PMS1, PMS2 or MLH1 as nucleic acid binding proteins. In the context of double stranded breaks, preferably, the method relies on the detection of Rad51 or RPA as nucleic acid binding proteins.

The agent may be a therapeutic agent, a chemical agent or an environmental agent.

In the context of a therapeutic agent, the method described herein is able to detecte nucleic acid damage caused by the therapeutic agent which may have a side effects. For example, the therapeutic agent may show therapeutic effect in respect of a specific disease or disorder and, at the same time, may cause nucleic acid damage in certain cell types. Thus, the method described herein is able to identify whether the therapeutic agent causes nucleic acid (e.g. DNA) damage.

The chemical agent may be a toxin, an ink, an adhesive, paint, oil, a lubricant, a hair dye, a laboratory reagent, a welding fume, a hazardous medicinal product or a cleaning fluid.

The environmental agent may be a pollutant, a bacterium (or a part thereof), a virus (or a part thereof) or a radioactive substance. The pollutant may be an air pollutant (e.g. a particulate matter, ozone, nitrogen dioxide, carbon monoxide, or sulphur dioxide), a water pollutant (e.g. bacteria, viruses, parasites, fertilisers, pesticides, pharmaceutical products, nitrates, phosphates, plastics, or faecal waste) or a soil pollutant (e.g. pesticides, petroleum products, radon, asbestos, lead, chromated copper arsenate or creosote).

The term "administration" as used herein refers to any type of administration of an agent to the subject. For example, the agent may be administered to the subject by any acceptable route of administration including, but not limited to, oral, topical (including transdermal) and parenteral modes of administration.

The term "exposure to" an agent as used herein is intended to encompass any type of exposure. For example, the subject may be exposed to the agent by inhaling the agent from the air, consuming the agent, absorbing the agent through the skin, eye or a membrane in the subject's body (e.g. nasal membrane or vaginal membrane), or by fluid communication with the agent (e.g. in case of a wound).

The sample may be obtained from the subject at least 1 hour, 6 hours, 12 hours, 18 hours, 24 hours, 2 days, 3 days, 4 days, 5 days, 6 days, 1 week, 2 weeks, 3 weeks, 4 weeks, 2 months, 3 months, 6 months, or 1 year after the administration of or the exposure to the agent. The method may include performing the method for detection of nucleic acid end(s) at multiple time points after the administration of or the exposure to the agent. This approach can assess a long-term effect of the agent on the health status of the subject.

The methods described herein may be performed using the kits described herein. The skilled person would understand that all molecules, reagents and approaches described in the context of the methods are equally applicable to the kit aspects of the invention. Nonetheless, certain preferred embodiments are also highlighted in the context of the kit aspects of the invention.

The invention provides a kit for detection of nucleic acid end(s) in a biological material, the kit comprising:
a) nucleic acid binding molecules;
b) a binding molecule that binds to the nucleic acid binding molecules; and
c) a binding molecule that binds to a nucleic acid binding protein.

The kit may comprise a proteinase, preferably proteinase K.

The kit for detection of nucleic acid end(s) in a biological material may comprise:
a) nucleic acid binding molecules;
b) a monoclonal antibody that binds to the nucleic acid binding molecules; and
c) a monoclonal antibody that binds to a nucleic acid binding protein.

The kit for detection of nucleic acid end(s) in a biological material may comprise:
a) proteinase (e.g. proteinase K);
b) nucleic acid binding molecules;
c) a binding molecule that binds to the nucleic acid binding molecules;
d) a binding molecule that binds to a nucleic acid binding protein; and
e) further binding molecules.

The kit for detection of nucleic acid end(s) in a biological material may comprise:
a) a hydrophilic solid support;
b) nucleic acid binding molecules;
c) a binding molecule that binds to the nucleic acid binding molecules; and
d) a binding molecule that binds to a nucleic acid binding protein.

The kit for detection of nucleic acid end(s) in a biological material may comprise:
a) nucleic acid binding molecules;
b) a binding molecule that binds to the nucleic acid binding molecules;
c) a binding molecule that binds to a nucleic acid binding protein;
d) reagents for performing amplification.

The kit for detection of nucleic acid end(s) in a biological material may comprise:
a) nucleic acid binding molecules;
b) a binding molecule that binds to the nucleic acid binding molecules;
c) a binding molecule that binds to a nucleic acid binding protein; and
d) further oligonucleotide molecules.

Preferably, the hydrophilic solid support is a positively charged solid support. For example, the hydrophilic solid support may be TOMO^{®} slide or a poly-L-lysine coated slide.

The nucleic acid binding molecules may comprise halogenated nucleotide or nucleoside molecules, DNA precursor analogues, and/or biotinylated nucleotide molecules. The kit may comprise at least one type of nucleic acid binding molecules. The kit may comprise at least two, at least three or at least four types of nucleic acid binding molecules.

The binding molecules that bind to the nucleic acid binding molecules may be antibodies or fragments thereof, streptavidin molecules, avidin molecules, streptavidin analogues, biotin molecules, peptides, proteins, nucleic acids, azides, or polymers.

The two different binding molecules may be of the same time or of a different type. For example, the two different binding molecules may be:
(i) different antibodies or fragments thereof, such as a monoclonal antibody (or a fragment thereof) and a polyclonal antibody (or a fragment thereof);
(ii) different antibodies or fragments thereof, such as a monoclonal antibody (or a fragment thereof) specific to a first nucleic acid binding molecule used in the method, and a monoclonal antibody (or a fragment thereof) specific to a second nucleic acid binding molecule used in the method;
(iii) an antibody or a fragment thereof and a streptavidin,
(iv) an antibody or a fragment thereof and an avidin,
(v) an antibody or a fragment thereof and a protein or peptide; or
(vi) an antibody or a fragment thereof and a biotin molecule.

Preferably, the two different binding molecules are (or comprise) two different monoclonal antibodies (or fragments thereof); one specific for the nucleic acid binding molecule used in the method, and the other specific for the nucleic acid bind protein (i.e. the intrinsic nucleic acid binding protein). Preferably, the two different binding molecules are (or comprise) two different monoclonal antibodies (or fragments thereof) from different species (e.g. a rabbit and a mouse).

In one embodiment, the two different binding molecules may be attached to an oligonucleotide molecule. Preferably, each one of the two different binding molecules is attached to an oligonucleotide molecule. The oligonucleotide molecules attached to each of the two different binding molecules are preferably different (i.e. have a different sequence).

In another embodiment, the kit may further comprise further binding molecules that are attached to an oligonucleotide molecule. The further binding molecules may be of the same type or of a different type. Preferably, the further binding molecules are of a different type. For example, the further binding molecules may be two (different) antibodies (or fragments thereof). The further binding molecules may be specific and/or selective for binding to the two different binding molecules.

The further oligonucleotide molecules are, preferably, able to hybridize to the oligonucleotide molecules attached to the binding molecules or further binding molecules present in the kit. The further oligonucleotide molecules may comprise the same or different type of oligonucleotides (e.g. have the same or different sequences). Preferably, the further oligonucleotide molecules comprise at least two types of oligonucleotides (i.e. at least two oligonucleotide sequences). The further binding molecules may be ligated to form a circular template.

The reagents for performing amplification may comprise a polymerase (e.g. a phi29 polymerase), a dNTP mix, a primer (e.g. a hexamer primer), and/or a nuclease-free water.

The kit may further comprise reagents for fixing the biological material (e.g. the cell). For example, the reagent for fixing the biological material may be 70%(v/v) ethanol.

The kit may further comprise a polymerase, and/or a ligase. The kit may comprise DNA polymerase I, TdT, Klenow fragment, Phu polymerase, Taq polymerase, T4 DNA Polymerase, T7 DNA Polymerase, T4 Polynucleotide Kinase, and/or RNA polymerase.

The invention also provides the kit described herein for use in the methods described herein.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

The invention is further disclosed in the following clauses:
1. A method for detection of nucleic acid end(s) in a biological material containing nucleic acids, wherein the method comprises the steps of:
   a) incubating the biological material with a proteinase to increase accessibility of the nucleic acid end(s) in the biological material without significantly affecting the levels of a nucleic acid binding protein in the biological material;
   b) adding nucleic acid binding molecules to the biological material under conditions such that the nucleic acid binding molecules bind to the nucleic acid end(s) in the biological material;
   c) adding two different binding molecules to the biological material under conditions such that one of the binding molecules binds to the nucleic acid binding molecules bound to the nucleic acid end(s) and the other binding molecule binds to the nucleic acid binding protein that is bound to the nucleic acid at the nucleic acid end(s); and
   d) detecting the nucleic acid end(s) in the biological material by detecting a co-localisation of the nucleic acid binding protein and the nucleic acid end via binding of the two different binding molecules.
2. The method for detection of nucleic acid end(s) in a biological material containing nucleic acids may comprise the steps of:
   a) incubating the biological material with a proteinase to increase accessibility of the nucleic acid end(s) to the level achieved using proteinase (e.g. proteinase K) at a concentration of 6 µg/mL to 8 µg/mL for less than 2 minutes;
   b) adding nucleic acid binding molecules to the biological material under conditions such that the nucleic acid binding molecules bind to the nucleic acid end(s) in the biological material;
   c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
   d) detecting the nucleic acid end(s) in the biological material by detecting a co-localisation of the nucleic acid binding protein and the nucleic acid end via binding of the two different binding molecules.
3. A method for detection of nucleic acid end(s) in a biological material containing nucleic acids, wherein the method comprises the steps of:
   a) incubating the biological material with a proteinase for less than 2 minutes;
   b) adding nucleic acid binding molecules to the biological material under conditions such that the nucleic acid binding molecules bind to the nucleic acid end(s) in the biological material;
   c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
   d) detecting the nucleic acid end(s) in the biological material by detecting a co-localisation of the nucleic acid binding protein and the nucleic acid end via binding of the two different binding molecules.
4. The method of any one of clauses 1-3, wherein the biological material is or is derived from an animal, a plant, a protozoan, a bacterium, or a virus.
5. The method of any one of clauses 1-4, wherein the biological material comprises a cell or a fragment thereof.
6. The method of any one of clauses 1-5, wherein the biological material comprises fixed cells.
7. The method of any one of clauses 1-6, wherein the nucleic acid comprises or is DNA and/or RNA, preferably DNA.
8. The method of any one of clauses 1-7, wherein the nucleic acid end(s) is a DNA end(s) or a RNA end(s), preferably a DNA end(s).
9. The method of any one of clauses 1-8, wherein the nucleic acid end(s) is a single stranded nucleic acid break or a double stranded nucleic acid break.
10. The method of any one of clauses 1-9, wherein the nucleic acid end(s) is a single stranded gap, a double stranded blunt-end break, a double stranded 3'-protruding break, or a single stranded nick.
11. The method of any one of clauses 1-10, wherein the proteinase is a broad spectrum proteinase.
12. The method of any one of clauses 1-11, wherein the proteinase is an aspartic protease, a glutamic protease, a metalloprotease, a cysteine protease, a serine protease, or a threonine protease, preferably wherein the protease is a serine protease, such as proteinase K.
13. The method of any one of clauses 1-12, wherein incubating the biological material with a proteinase is performed for at least 5 seconds, preferably at least 10 seconds and more preferably at least 15 seconds.
14. The method of any one of clauses 1-13, wherein incubating the biological material with a proteinase is performed for between 1 second and less than 2 minutes, between 15 seconds and 90 seconds, between 20 seconds and 80 seconds, between 25 seconds and 70 seconds, between 30 seconds and 60 seconds, preferably incubating the biological material with a proteinase is performed for between 30 seconds and 60 seconds.
15. The method of any one of clauses 1-14, wherein incubating the biological material with a proteinase is performed for less than 100 seconds, less than 90 seconds, less than 80 seconds, less than 70 seconds, or less than 60 seconds, preferably 90 seconds or less.
16. The method of any one of clauses 1-15, wherein incubating the biological material with a proteinase is performed for about 30 seconds or about 60 seconds.
17. The method of any one of clauses 1-16, wherein the step of incubating the biological material with a proteinase is stopped by addition of a proteinase inhibitor.
18. The method of any one of clauses 1-17, wherein incubating the biological material with a proteinase is performed at a temperature of between 15ºC and 30 ºC, preferably between 20ºC and 25ºC.
19. The method of any one of clauses 1-18, wherein the proteinase is in solution, preferably at a concentration of between 1 µg/mL and 20 µg/mL, between 3 µg/mL and 15 µg/mL, between 5 µg/mL and 10 µg/mL, or between 6 µg/mL and 8 µg/mL, preferably about 7 µg/mL .
20. The method of clause 19, wherein the solution comprises a buffer, urea, and/or SDS.
21. The method of any one of clauses 1-20, wherein the nucleic acid binding molecules are selected from a group comprising:
   (i) halogenated nucleotide or nucleoside molecules, such as BrdU, IdU, CldU,
   (ii) DNA precursor analogues such as EdU (5-Ethynyl-2'-deoxyuridine), F-ara-EdU, 5-Ethynyl-2'-deoxycytidine,
   (iii) biotinylated nucleotide molecules,
   (iv) ADP-ribose molecules,
   (v) protein molecules,
   (vi) nucleotide, or nucleoside molecules labelled with labels; optionally wherein the labels are selected from a group comprising fluorescent molecules, or chemiluminescent molecules, or radioisotopes, or enzyme substrates, or biotin molecules.
22. The method of any one of clauses 1-21, wherein the nucleic acid binding molecules are halogenated nucleotide or nucleoside molecules, DNA precursor analogues, and/or biotinylated nucleotide molecules.
23. The method of any one of clauses 1-22, wherein the nucleic acid binding molecules bind to the nucleic acid end(s) in the biological material by an enzyme catalysed process of addition, for example, using DNA polymerase I, TdT, Klenow fragment, Phu polymerase, Taq polymerase, T4 DNA Polymerase, T7 DNA Polymerase, T4 Polynucleotide Kinase, or RNA polymerase.
24. The method of any one of clauses 1-23, wherein the two different binding molecules may be or comprise two different monoclonal antibodies (or fragments thereof).
25. The method of any one of clauses 1-24, wherein the two different binding molecules are incubated with the biological material for at least 30 minutes, preferably 60 minutes.
26. The method of any one of clauses 1-27, wherein the two different binding molecules are incubated with the biological material at a temperature of between 15ºC and 30 ºC, preferably between 20ºC and 25ºC.
27. The method of any one of clauses 1-26, wherein the step of adding two different binding molecules to the biological material may include adding the two different binding molecules separately or together, preferably the two different binding molecules are added separately, preferably one of the two different binding molecules is added first and the other different binding molecule is added second.
28. The method of any one of clauses 1-27, wherein the step of adding two different binding molecules to the biological material comprises the steps of:
   a. adding one of the two different binding molecules to the biological material
   b. incubating the one of the two different binding molecules with the biological material for at least 30 min, preferably 60 min
   c. optionally, performing a wash step to remove any unbound binding molecule
   d. adding the other of the two different binding molecules to the biological material
   e. incubation the other of the two different binding molecules with the biological material for at least 30 min, preferably 60 min
   f. optionally, performing a wash step to remove any unbound binding molecule
29. The method of any one of clauses 1-28, wherein the nucleic acid binding protein is a protein found in the biological material.
30. The method of any one of clauses 1-29, wherein the nucleic acid binding protein is bound to the nucleic acid at the nucleic acid end(s) either directly or indirectly.
31. The method of clauses 30, wherein the nucleic acid binding protein is bound to the nucleic acid at the nucleic acid end(s) indirectly via interaction with an adaptor protein.
32. The method of any one of clauses 1-31, wherein the nucleic acid binding protein is a nucleic acid repair protein, preferably a nucleic acid end (e.g. break) repair protein.
33. The method of any one of clauses 1-32, wherein the nucleic acid binding protein is RAD51, RPA (e.g. RPA70), PMS2, MLH1, PMS1, p53, MSH2, Ataxia telangiectasia and Rad3 related protein, ATM serine/threonine kinase, RAD52, XRCC1, Proliferating cell nuclear antigen, XPC, Ku70, Ku80, Nibrin, DDB2, Bloom syndrome protein, CHEK2, RAD51C, DNA polymerase eta, Rad50, DDB1, RBBP8, FANCB, PALB2, H2AX, yH2AX DNA repair and recombination protein RAD54-like, PrimPol, REV1, Terminal deoxynucleotidyl transferase, DNA polymerase nu, Fanconi anemia, complementation group C, FANCF, ERCC8, Artemis, Ubiquitin ligase, RNF4, TP53BP1, AP endonuclease, ERCC4, Transcription factor II H, XRCC3, XRCC2, RecA, ERCC6, SLX4, Sirtuin 1, PTEN, Replication protein A2, Replication protein A3, Alkb homolog 3, alpha-ketoglutaratedependent dioxygenase, Exonuclease 5, DNA polymerase alpha catalytic subunit, Cyclin H, PARP1/2
34. The method of any one of clauses 1-33, wherein the nucleic acid binding protein comprises post-translational modifications.
35. The method of any one of clauses 1-34, wherein each of the two different binding molecules is attached to an oligonucleotide molecule.
36. The method of any one of clauses 1-35, wherein each of the two different binding molecules is attached to a different oligonucleotide molecule.
37. The method of any one of clauses 1-36, wherein the method further comprises a step of adding further binding molecules that respectively bind to the two different binding molecules after the step of adding the two different binding molecules.
38. The method of clauses 37, wherein the further binding molecules are two different further binding molecules.
39. The method of clause 37 or 38, wherein the further binding molecules are attached to an oligonucleotide molecule.
40. The method of any one of clauses 37-39, wherein each of the two different further binding molecules is attached to a different oligonucleotide molecule.
41. The method of any one of clauses 1-40, wherein the method further comprises, after the step of adding two different binding molecules (or after the step of adding the further binding molecules when dependent from clauses 37-40) a step of adding further oligonucleotide molecules which hybridise with the oligonucleotide molecules attached to the two different binding molecules (or to the further binding molecules) to form a circular template.
42. The method of any one of clauses 1-41, wherein the method further comprises, after the step of adding two different binding molecules (or after the step of adding the further binding molecules), a step of adding two different further oligonucleotide molecules which hybridise with the oligonucleotide molecules attached to the two different binding molecules (or to the further binding molecules) to form a circular template.
43. The method of clause 41 or 42, wherein the circular amplification template is formed by ligation of the further oligonucleotide molecules (or the two different further oligonucleotide molecules).
44. The method of any one of clauses 1-43, wherein the method further comprises a step of blocking nonspecific binding site(s) of molecules after the step of adding two different binding molecules to the biological material and/or the step of adding further binding molecules.
45. The method of any one of clauses 1-44, wherein detecting the nucleic acid end(s) comprises a step of performing nucleic acid amplification to produce an amplification product that is detected.
46. The method of clause 45, wherein the nucleic acid amplification is rolling circle amplification.
47. The method of any one of clauses 1-46, wherein detecting the co-localisation of the nucleic acid binding protein and the nucleic acid end comprises:
   a. a proximity ligation assay;
   b. a branched proximity hybridization assay;
   c. a FRET detection; or
   d. a proximity-driven reaction with fluorophores or dyes.
48. A kit for detection of nucleic acid end(s) in a biological material, the kit comprising:
   a) nucleic acid binding molecules;
   b) a binding molecule that binds to the nucleic acid binding molecules; and
   c) a binding molecule that binds to a nucleic acid binding protein.
49. The kit of clause 48, wherein the kit comprises a proteinase, preferably proteinase K
50. A kit for detection of nucleic acid end(s) in a biological material, the kit comprising:
   a) nucleic acid binding molecules;
   b) a monoclonal antibody that binds to the nucleic acid binding molecules; and
   c) a monoclonal antibody that binds to a nucleic acid binding protein.
51. A method for assessing effectiveness of a therapeutic agent, the method comprising the steps:
   a) performing the method of any one of clauses 1-47 on a sample obtained from a subject before the administration of the therapeutic agent,
   b) performing the method of any one of clauses 1-47 on a sample obtained from the subject after the administration of the therapeutic agent,
   c) comparing the amount of the nucleic acid end(s) detected in steps a) and b), wherein the therapeutic agent is effective if the amount of the detected nucleic acid end(s) is greater in step a) than step b),
   wherein steps a) and b) can be performed in any order.
52. The method of clause 51, wherein the therapeutic agent targets a protein involved in a mismatch repair (MMR) pathway.
53. The method of clause 51 or 52, wherein the therapeutic agent targets PMS1, PMS2 or MLH1.
54. A method for assessing effectiveness of a therapeutic agent, the method comprising the steps:
   a) performing the method of any one of clauses 1-47 on a sample obtained from a subject before the administration of the therapeutic agent,
   b) performing the method of any one of clauses 1-47 on a sample obtained from the subject after the administration of the therapeutic agent,
   c) comparing the amount of the nucleic acid end(s) detected in steps a) and b), wherein the therapeutic agent is effective if the amount of the nucleic acid end(s) is greater in step b) than step a),
   wherein steps a) and b) can be performed in any order.
55. The method of clause 54, wherein the therapeutic agent targets RPA or RAD51.
56. The method of clause 54 or 55, wherein the sample is a cancer biopsy.
57. The method of any one of clauses 54-56, wherein the sample comprises a cancer cell.
58. The method of any one of clauses 54-57, wherein the therapeutic agent is a cancer drug.
59. The method of any one of clauses 54-58, wherein the therapeutic agent inhibits DNA synthesis, causes nucleic acid damage, and/or inhibits DNA damage repair.
60. A method for assessing nucleic acid damage caused by an agent, the method comprising the steps:
   a) performing the method of any one of clauses 1-47 on a sample obtained from a subject before administration of, or exposure to, an agent,
   b) performing the method of any one of clauses 1-47 on a sample obtained from the subject after the administration of, or exposure to, the agent,
   c) comparing the amount of the nucleic acid end(s) detected in steps a) and b), wherein the agent causes nucleic acid damage if the amount of the nucleic acid end(s) is greater in step b) than step a),
   wherein steps a) and b) can be performed in any order.
61. The method for detection of single stranded nucleic acid end(s) in a biological material containing nucleic acids may comprise the steps of:
   a) incubating the biological material with a proteinase to increase accessibility of the nucleic acid end(s) to the level achieved using proteinase (e.g. proteinase K) at a concentration of 6 µg/mL to 8 µg/mL for less than 60 seconds;
   b) adding nucleic acid binding molecules to the biological material under conditions such that the nucleic acid binding molecules bind to the nucleic acid end(s) in the biological material;
   c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
   d) detecting the nucleic acid end(s) in the biological material by detecting a co-localisation of the nucleic acid binding protein and the nucleic acid end via binding of the two different binding molecules.
62. The method of clauses 61, wherein the nucleic acid binding protein binds to single stranded nucleic acid end(s).
63. The method of clause 61 or 62, wherein the nucleic acid binding protein is a protein involved in MMR.
64. The method of any one of clauses 61-63, wherein the nucleic acid binding protein is PMS1, PMS2, and/or MLH1.
65. The method for detection of double stranded nucleic acid end(s) in a biological material containing nucleic acids may comprise the steps of:
   a) incubating the biological material with a proteinase to increase accessibility of the nucleic acid end(s) to the level achieved using proteinase (e.g. proteinase K) at a concentration of 6 µg/mL to 8 µg/mL for less than 2 minutes;
   b) adding nucleic acid binding molecules to the biological material under conditions such that the nucleic acid binding molecules bind to the nucleic acid end(s) in the biological material;
   c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
   d) detecting the nucleic acid end(s) in the biological material by detecting a co-localisation of the nucleic acid binding protein and the nucleic acid end via binding of the two different binding molecules.
66. The method of clauses 65, wherein the nucleic acid binding protein binds to double stranded nucleic acid end(s).
67. The method of clause 65 or 66, wherein the nucleic acid binding protein is a protein involved in HR or double stranded DNA damage repair.
68. The method of any one of clauses 65-67, wherein the nucleic acid binding protein is RAD51 and/or RAD70.

These and other aspects of the invention will now be described with reference to the accompanying Figures, in which:

### DESCRIPTION OF THE FIGURES

Figure 1 shows an exemplary method of the present invention with PMS2 as the nucleic acid binding protein.
Figure 2 shows an exemplary method of the present invention with PMS2 as the nucleic acid binding protein and further binding molecules.
Figure 3 shows an exemplary method of the present invention with PMS2 as the nucleic acid binding protein and direct incorporation into a single stranded DNA strand of the nucleic acid binding molecules.
Figure 4 shows an exemplary method of the present invention with Rad51 as the nucleic acid binding protein.
Figure 5A shows results for Sample 3 - NT cells sSTRIDE MMR (Example 1) staining.
Figure 5B shows results for Sample 4 - 6TG treated cells, sSTRIDE MMR (Example 1) staining.
Figure 5C shows results of analysis - antigen retrieval 1 (proteinase K 2 min) with 6TG treatment.
Figure 6A shows results for Sample 5 - non-treated U2OS WT, sSTRIDE MMR staining.
Figure 6B shows results for Sample 6 - non-treated U2OS WT cells, sSTRIDE MMR staining negative control (only anti-biotin antibody).
Figure 6C shows results for Sample 7 - non-treated U2OS WT cells, sSTRIDE MMR staining negative control (only PMS2 antibody).
Figure 6D shows results for Sample 8 - non-treated U2OS WT cells, sSTRIDE standard assay.
Figure 7 shows results of analysis - antigen retrieval 2 (proteinase K 1 min)
Figure 8 shows comparison between sSTRIDE MMR assay and the negative controls.
Figure 9A shows results for Sample 9 - non-treated U2OS WT cells, sSTRIDE MMR standard assay.
Figure 9B shows results for Sample 10 - non-treated U2OS WT cells, sSTRIDE MMR assay negative control (PMS2 antibody only).
Figure 9C shows results for Sample 11 - non-treated U2OS WT cells, sSTRIDE MMR assay positive control (standard procedure).
Figure 9D shows results of analysis - antigen retrieval 3 (no proteinase K).
Figure 10A shows comparison of three antigen retrieval methods (U2OS WT non-treated cells). Representative images showing the result of labelling performed with sSTRIDE-MMR assay with different antigen retrieval conditions.
Figure 10B shows results of analysis - dependence on antigen retrieval.
Figure 11 shows comparison of the foci intensity and frequency. In the upper photos sSTRIDE MMR foci are shown, in lower sSTRIDE assay foci are shown.
Figure 12A shows Foci intensity and frequency - (U2OS WT cells, 48h treatment, antigen retrieval 2).
Figure 12B shows Antigen retrieval methods comparison - Abeam antibody. Sample 27-30 - U2OS WT cells treated 48h by 6TG in different concentration, sSTRIDE MMR assay positive control (antigen retrieval method v2).

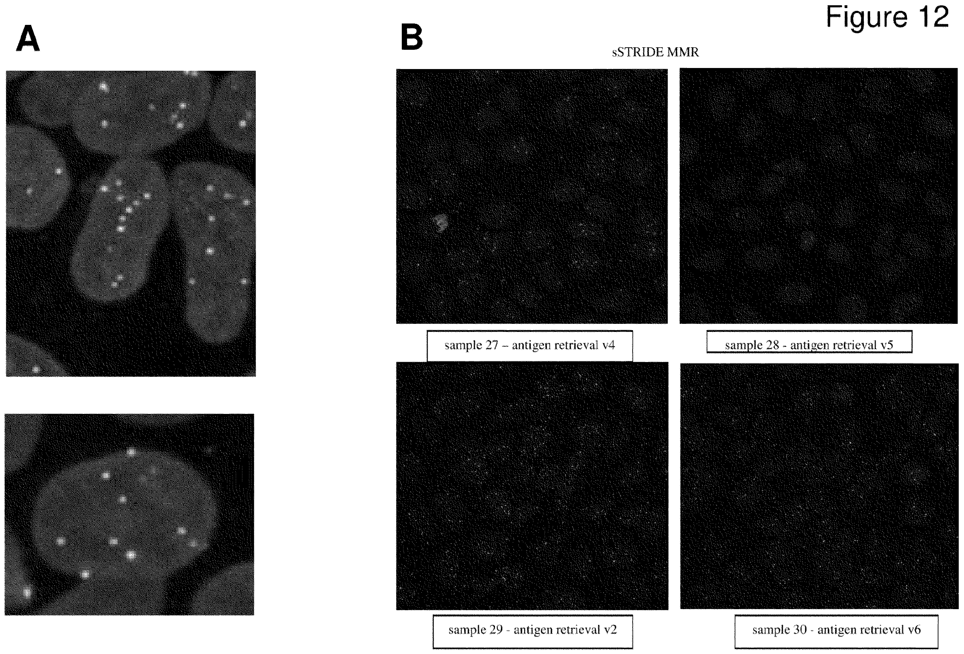

Figure 12A shows ntigen retrieval methods comparison - results of quantitative analysis - comparison of antigen retrieval methods.

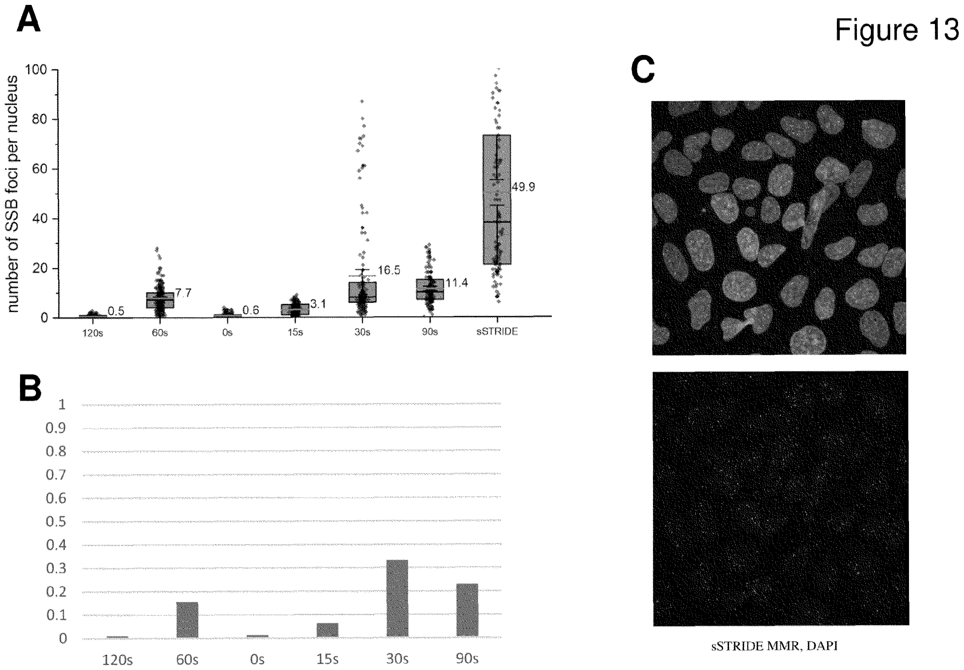

Figure 13B shows results of analysis - ratio between sSTRIDE MMR and sSTRIDE normal procedure.
Figure 13C shows results for Sample 29 - U2OS WT non-treated cells - antigen retrieval method v5.

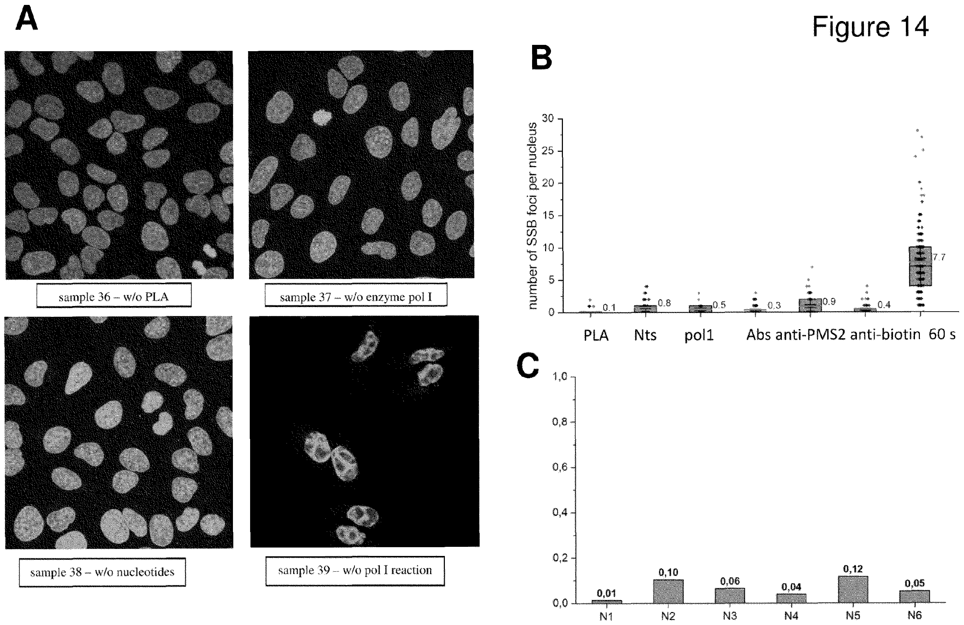

Figure 14A shows negative controls - Abeam antibody. Sample 36-39 - U2OS WT non-cells treated 48h, sSTRIDE MMR assay negative controls (antigen retrieval method v2). In each enzymatic reaction marked agent was omitted
Figure 14B shows results of quantitative analysis - negative controls (antigen retrieval 2). Results of quantitative analysis - comparison of antigen retrieval methods.
Figure 14C shows results of analysis - ratio between negative controls and sSTRIDE MMR. In control sample omitted were: - N1 - PLA, N2 - nucleotides (nts), N3 - pol I reaction, N4 - antibodies (abs), N5 - anti-PMS2, N6 - anti-biotin.

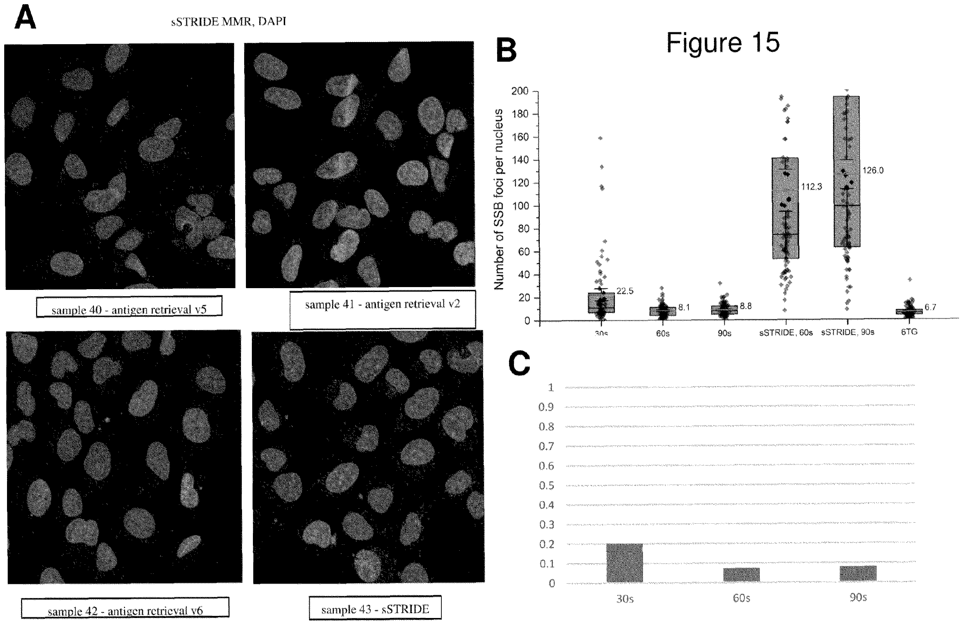

Figure 15A shows results for Sample 40-43 - untreated U2OS WT cells labelled with sSTRIDE-MMR (40-42) and sSTRIDE (43), different antigen retrieval methods were tested.
Figure 15B shows results of quantitative analysis - antigen retrieval. Results of analysis - ratio between sSTRIDE normal procedure and sSTRIDE MMR.
Figure 15C shows results for different antigen retrievals.

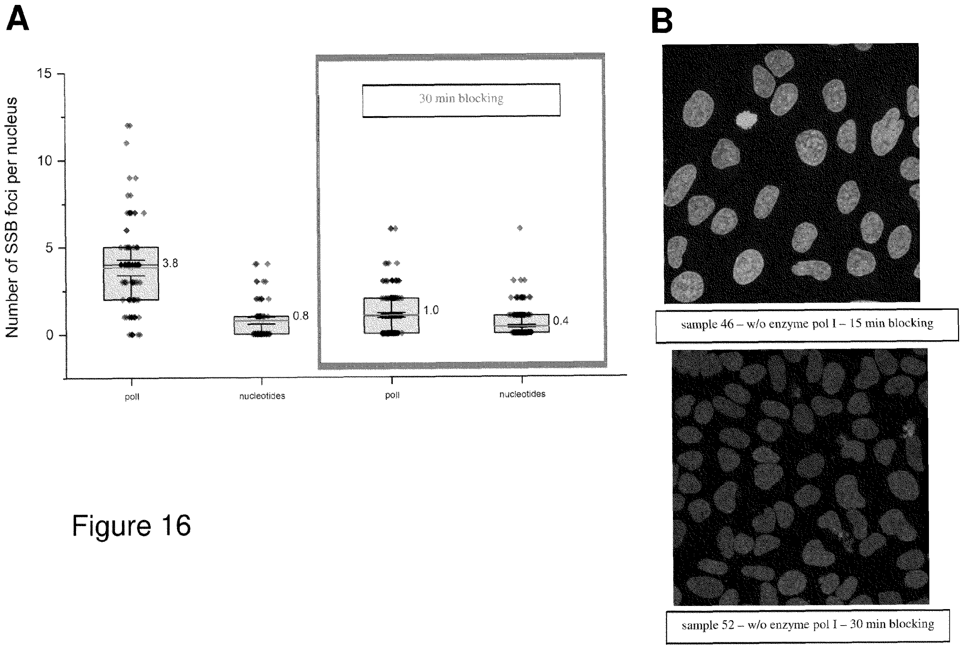

Figure 16A shows negative controls - impact of endogenous biotin time blocking for the redout level.
Results of quantitative analysis - comparison of controls with different biotin blocking time - in red frame 30 min blocking time, without frame 15 min blocking time.
Figure 16B shows results for Samples 46 and 52 - U2OS WT cells, sSTRIDE MMR assay negative controls. Difference between readout level depending on blocking time.

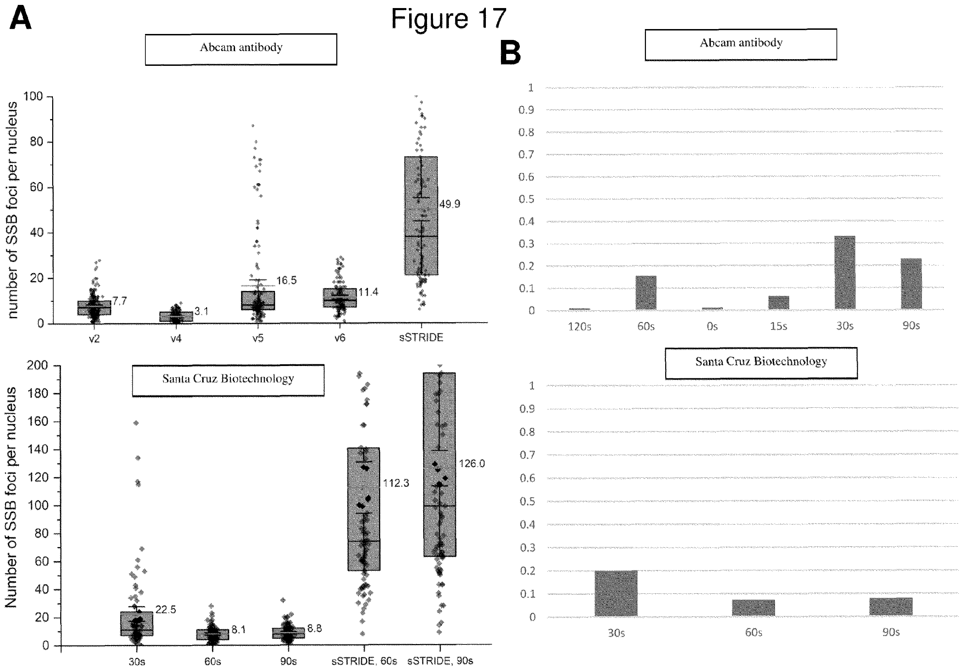

Figure 17A shows comparison - antigen retrieval methods. v2 - 60s, v4 - 15s, v5 - 30s, v6 - 90s.
Figure 17B shows comparison between ratio sSTRIDE MMR/sSTRIDE

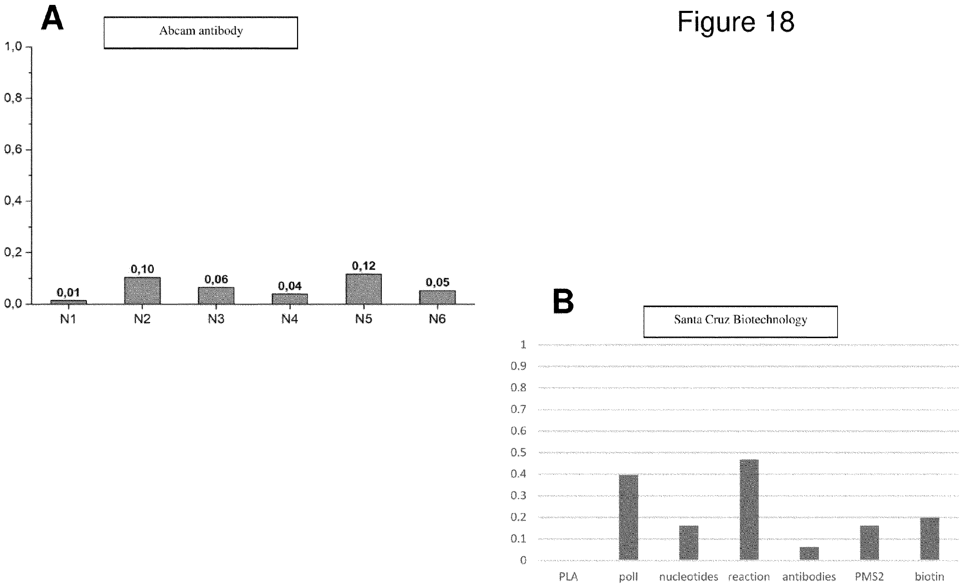

Figure 18A shows negative control ratio comparison sSTRIDE MMR/sSTRIDE (abeam antibody). In control omitted: - N1 - PLA, N2 - nucleotides, N3 - pol I reaction, N4 - antibodies, N5 - anti-PMS2, N6 - anti-biotin.
Figure 18 B shows negative control ratio comparison sSTRIDE MMR/sSTRIDE (Santa Cruz Biotech antibody).

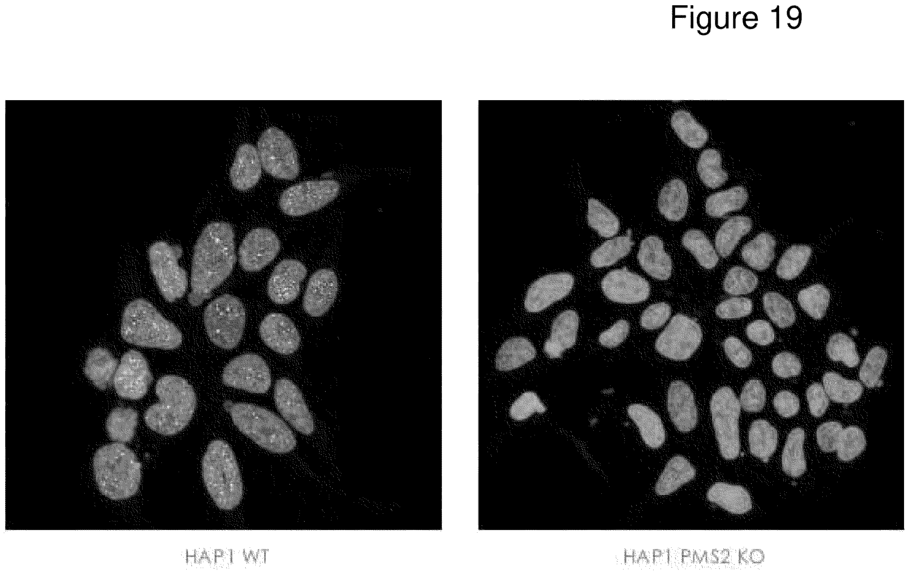

Figure 19 shows HAP1 parental vs PMS2 KO cell line (IF assessment of the staining (Abcam antibody). Representative images show an overlay of DAPI and PMS2 signals.

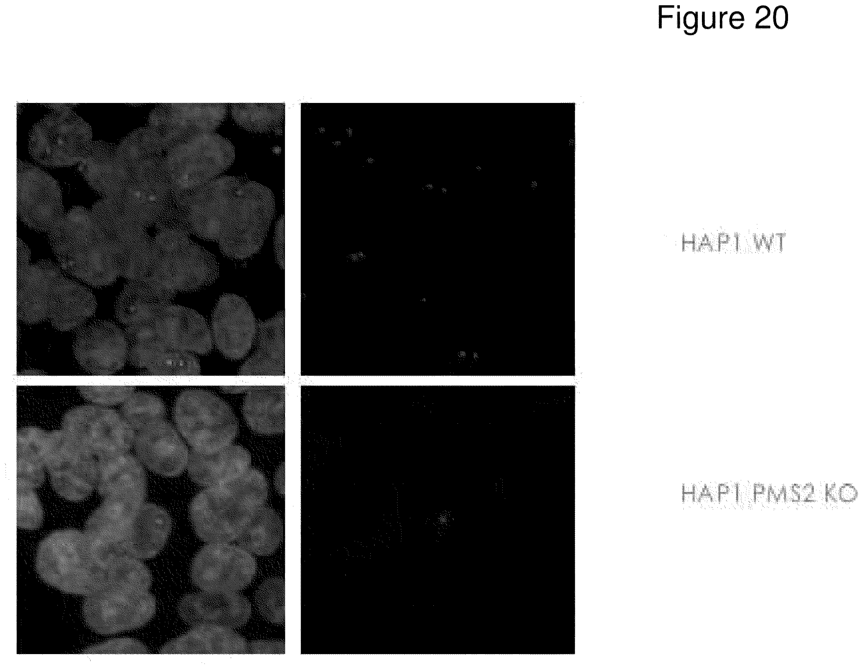

Figure 20 shows HAP1 parental vs PMS2 KO with sSTRIDE-MMR labelling.

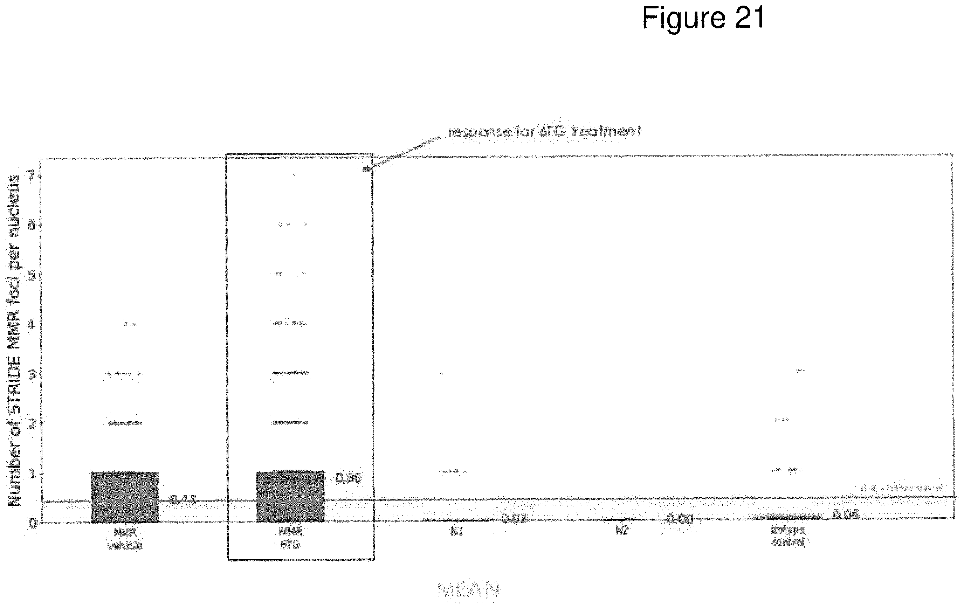

Figure 21 shows HAP1 parental: untreated, positive control and negative technical controls (*6TG 1uM 48 h treatment).

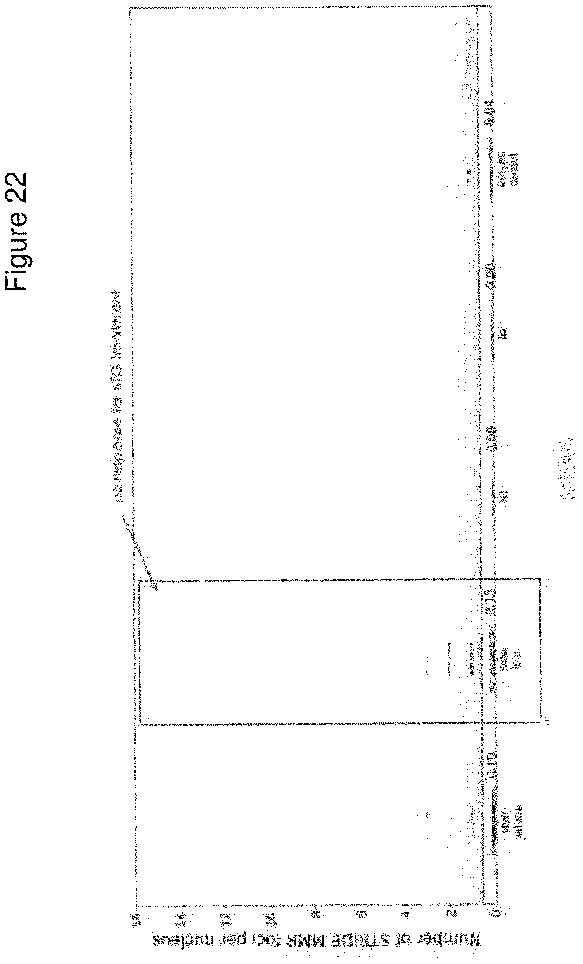

Figure 22 shows HAP1 PMS2-KO: untreated, positive control and negative technical controls (*6TG 1uM 48 h treatment).

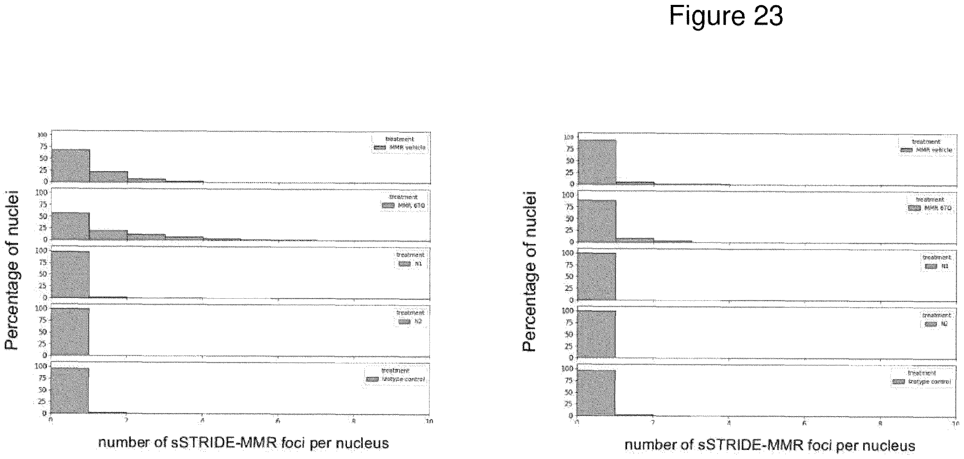

Figure 23 shows HAP1 PMS2-KO: untreated, positive control and negative technical controls vis a vis different treatment.

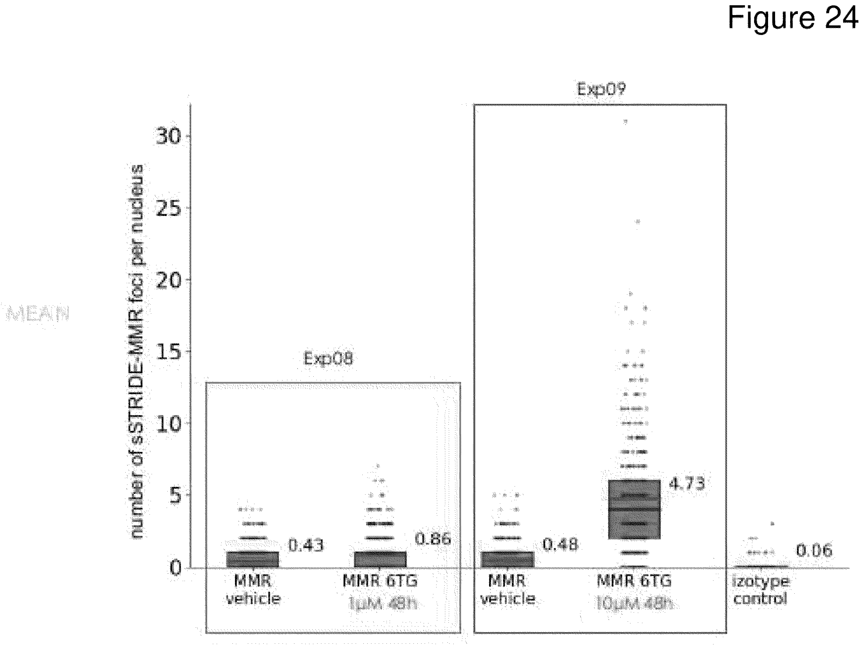

Figure 24 shows HAP1 parental: increased 6TG concentration control.

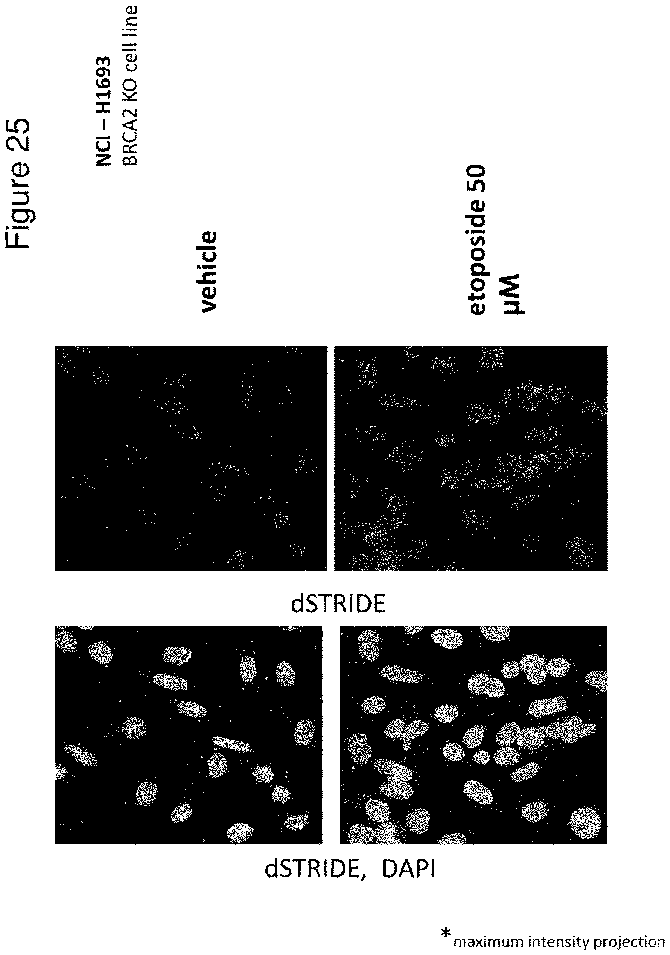

Figure 25 shows dSTRIDE imagining with and without etoposide.

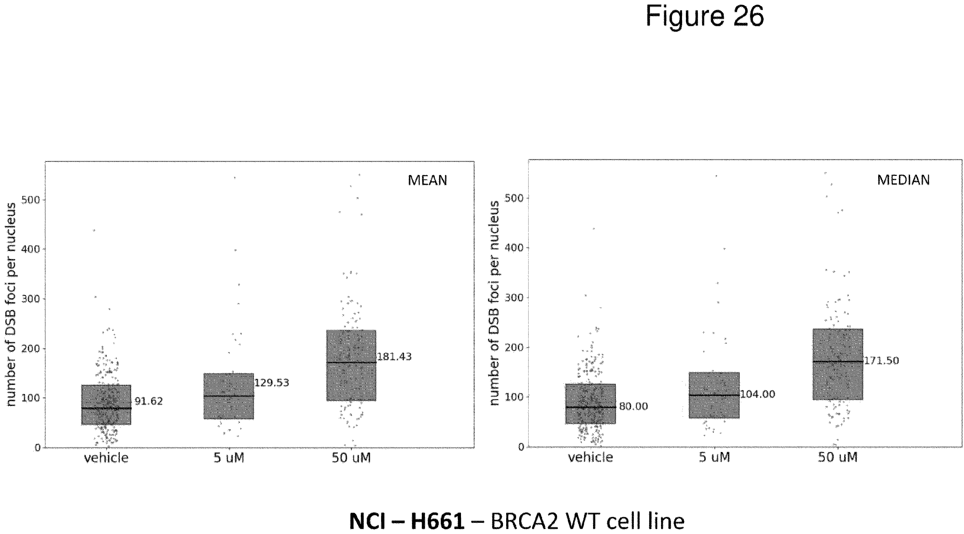

Figure 26 shows results of dSTRIDE signals data analysis, measurement: level of double-strand DNA breaks after the treatment with etoposide.

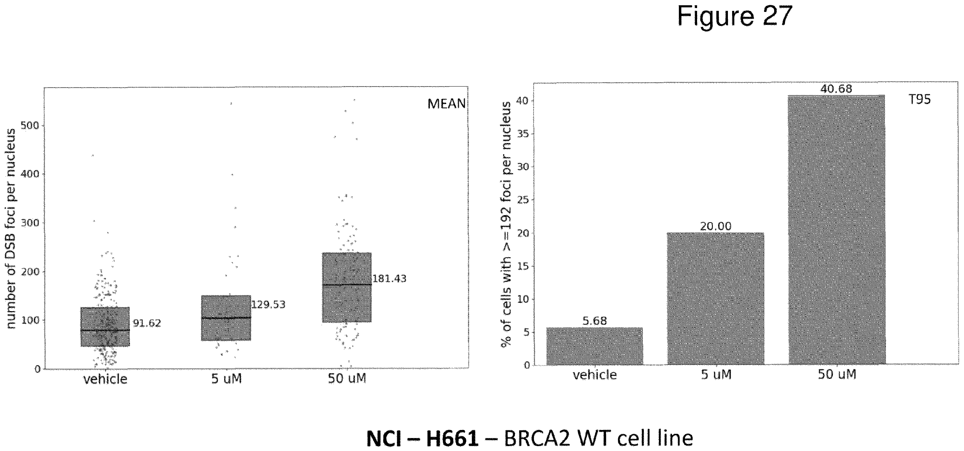

Figure 27 shows Results of dSTRIDE signals data analysis, measurement: level of double-strand DNA breaks after the treatment with etoposide. *in vehicle condition 5% of most damage cells have >=192 foci.

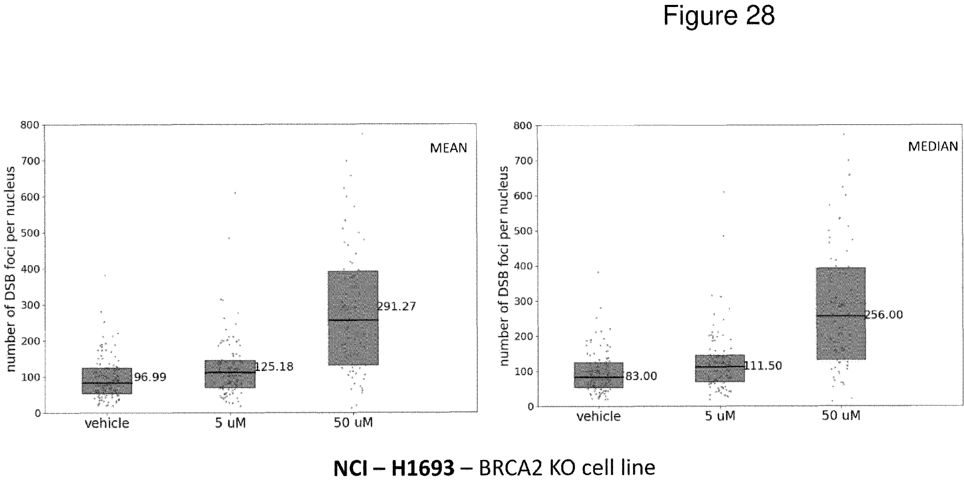

Figure 28 shows results of dSTRIDE signals data analysis, measurement: level of double-strand DNA breaks after the treatment with etoposide.

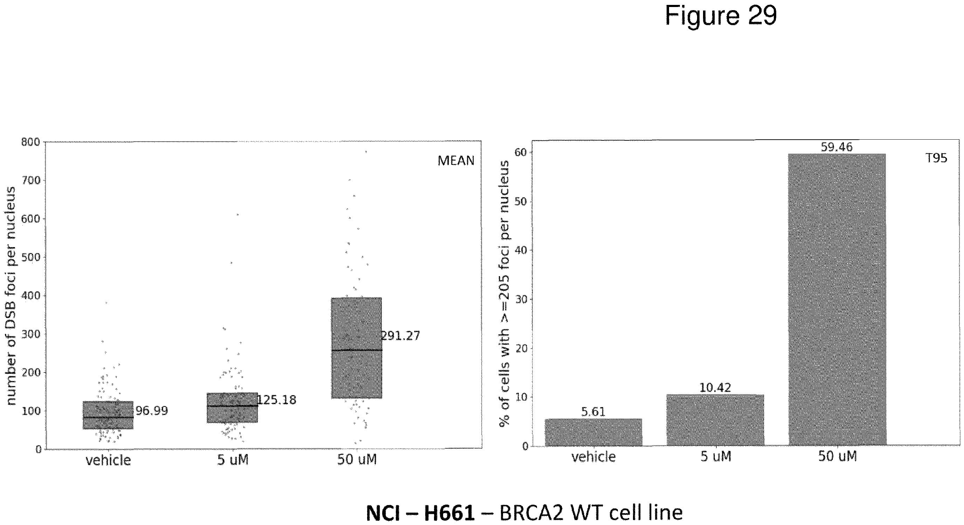

Figure 29 shows results of dSTRIDE signals data analysis, measurement: level of double-strand DNA breaks after the treatment with etoposide. *in vehicle condition 5% of most damage cells have >=205 foci.

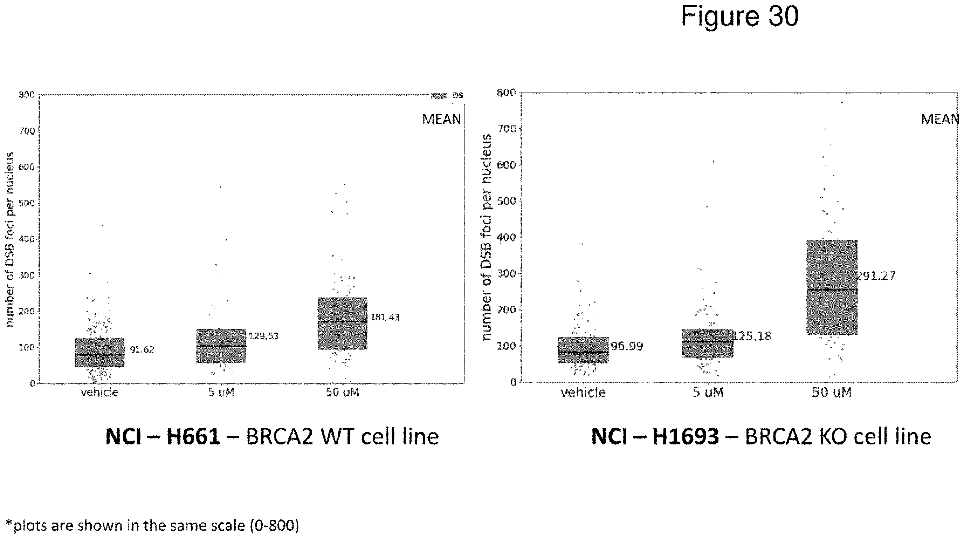

Figure 30 shows results of dSTRIDE signals data analysis, cell line comparison.

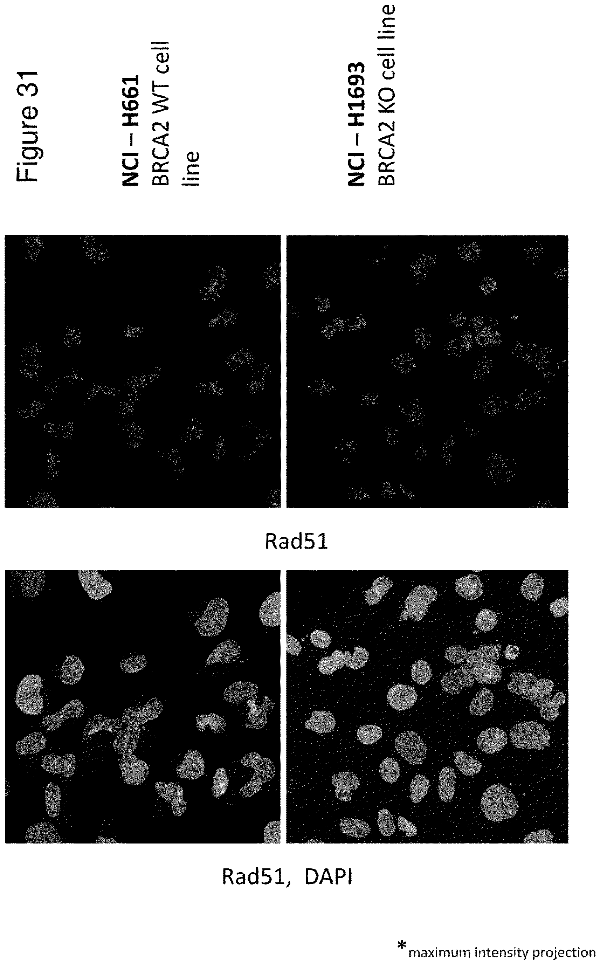

Figure 31 shows Rad51-dSTRIDE staining in HR defective (termed "BRCA2 KO") and WT cell line.

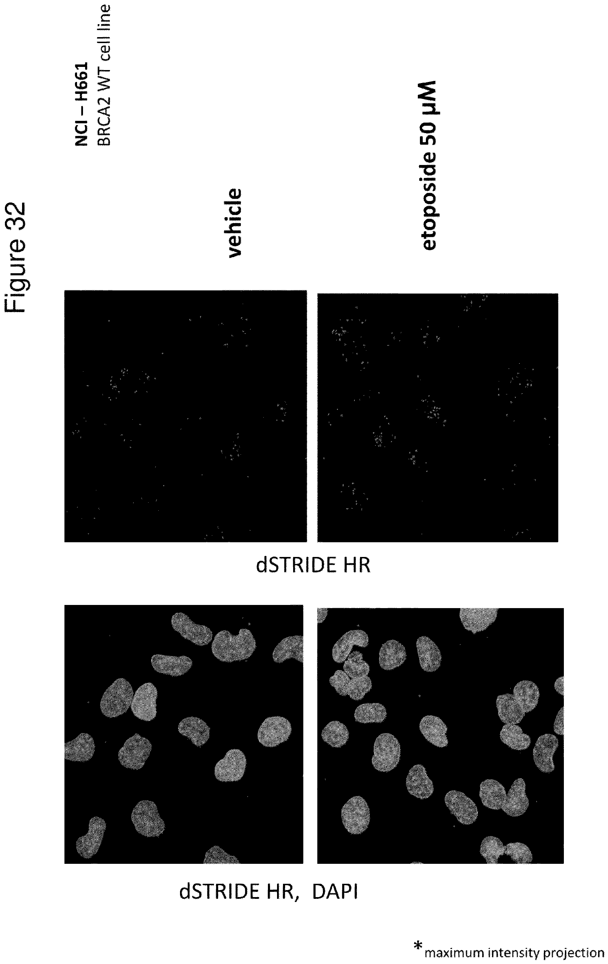

Figure 32 shows Rad51-dSTRIDE staining in BRCA2 WT cell line with and without etoposide.

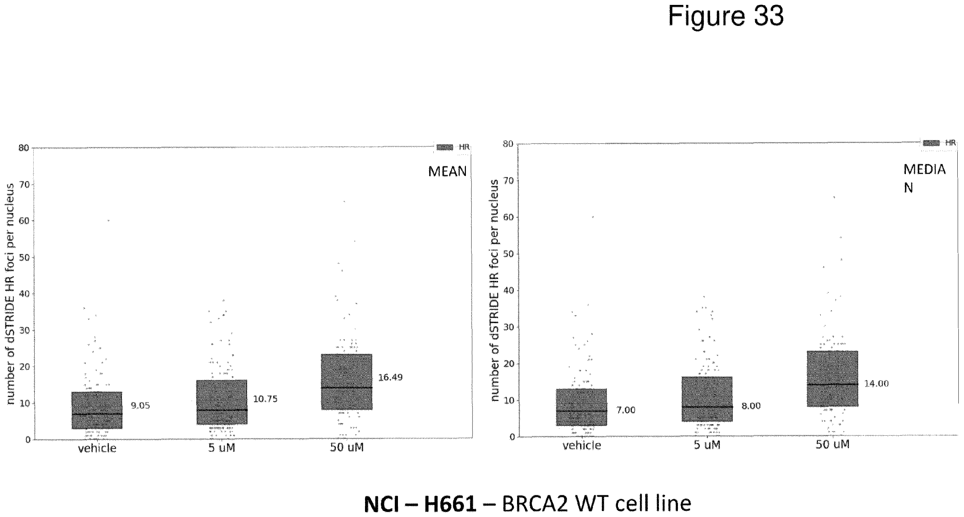

Figure 33 shows results of dSTRIDE HR signals data analysis, measurement: level of double-strand DNA breaks associated with Rad51 after the treatment with etoposide.

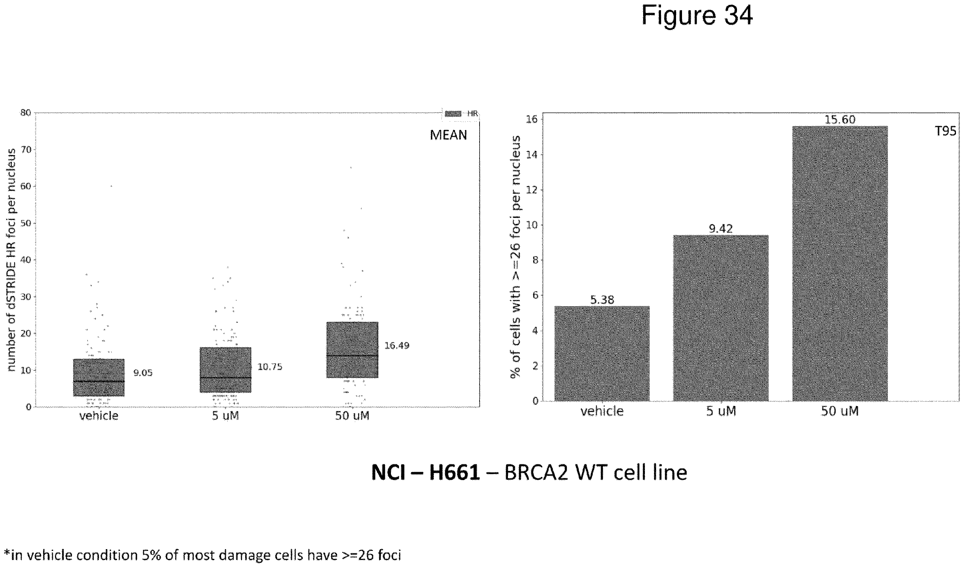

Figure 34 shows results of dSTRIDE HR signals data analysis, measurement: level of double-strand DNA breaks associated with Rad51 after the treatment with etoposide.

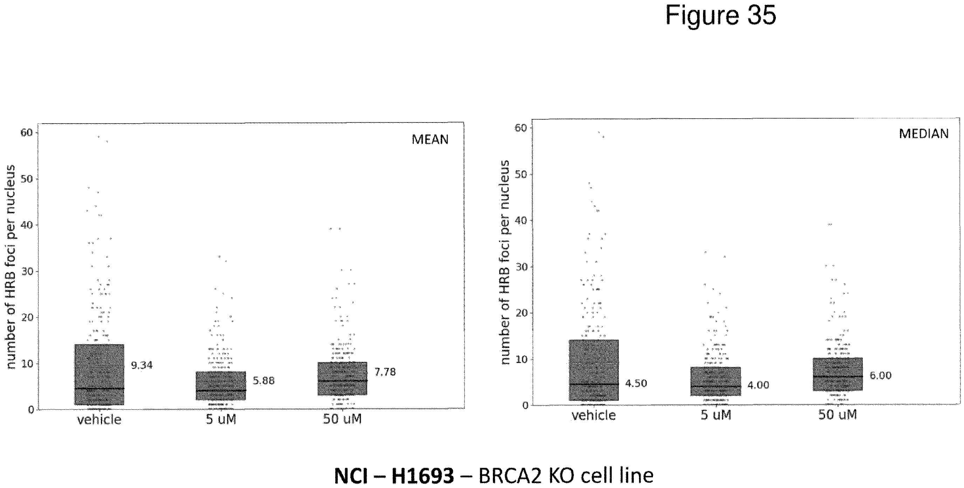

Figure 35 shows results of dSTRIDE HR signals data analysis, measurement: level of double-strand DNA breaks associated with Rad51 after the treatment with etoposide.

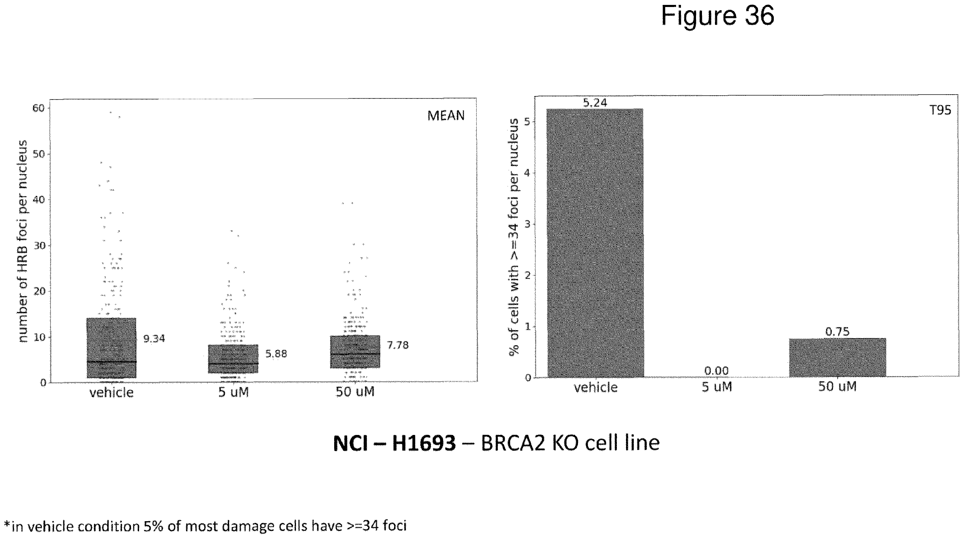

Figure 36 shows results of dSTRIDE HR signals data analysis, measurement: level of double-strand DNA breaks associated with Rad51 after the treatment with etoposide.

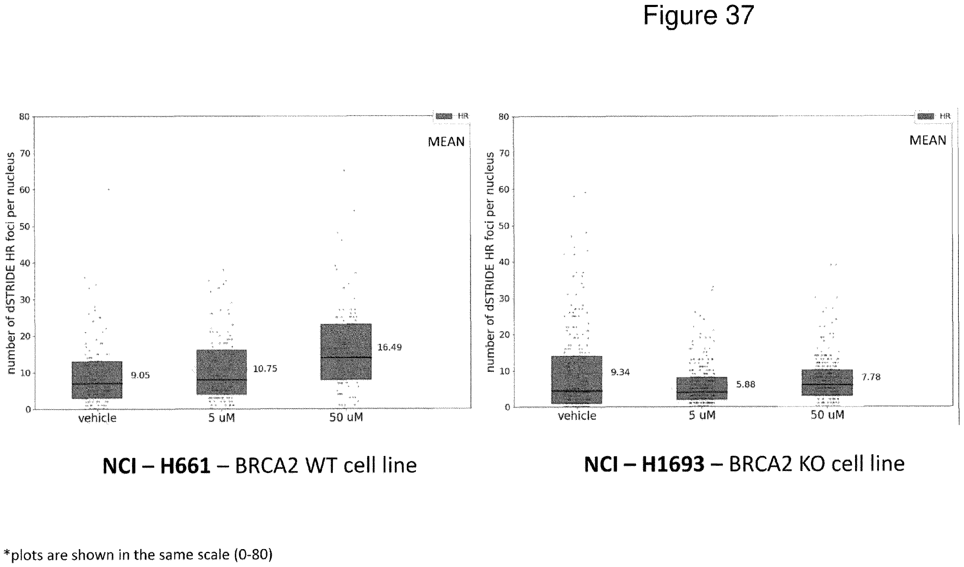

Figure 37 shows results of dSTRIDE HR signals data analysis, cell line comparison.

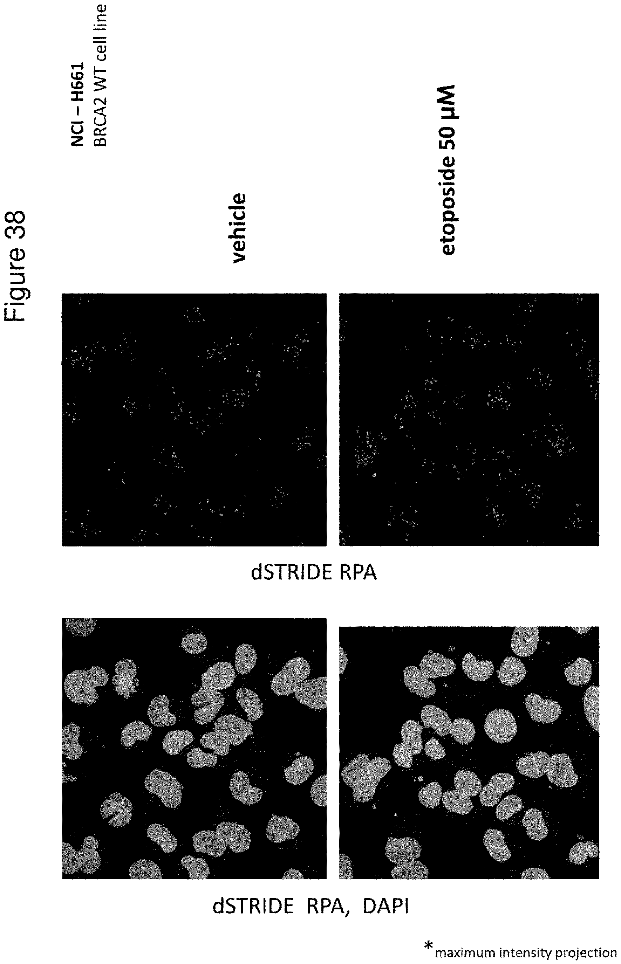

Figure 38 shows dSTRIDE-RPA staining in BRCA2 WT cell line with and without etoposide.

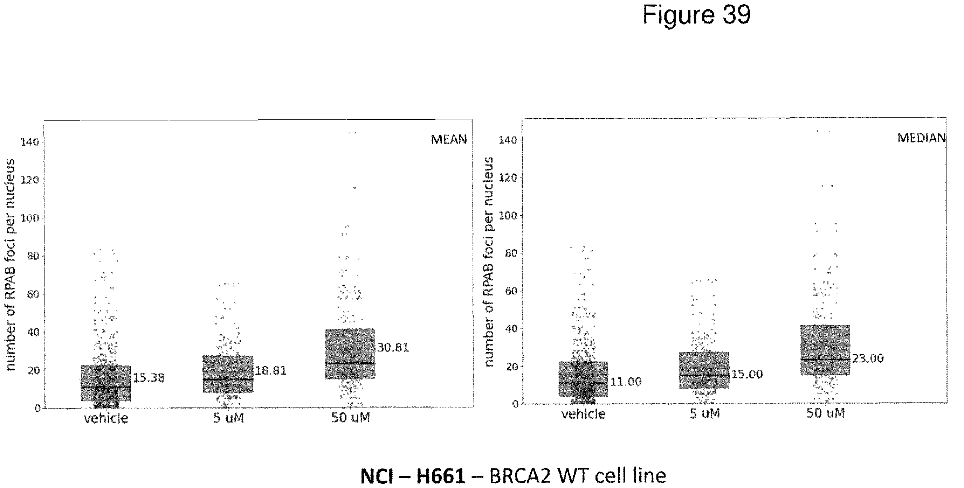

Figure 39 shows results of dSTRIDE RPA signals data analysis, measurement: level of double-strand DNA breaks associated with RPA70 after the treatment with etoposide.

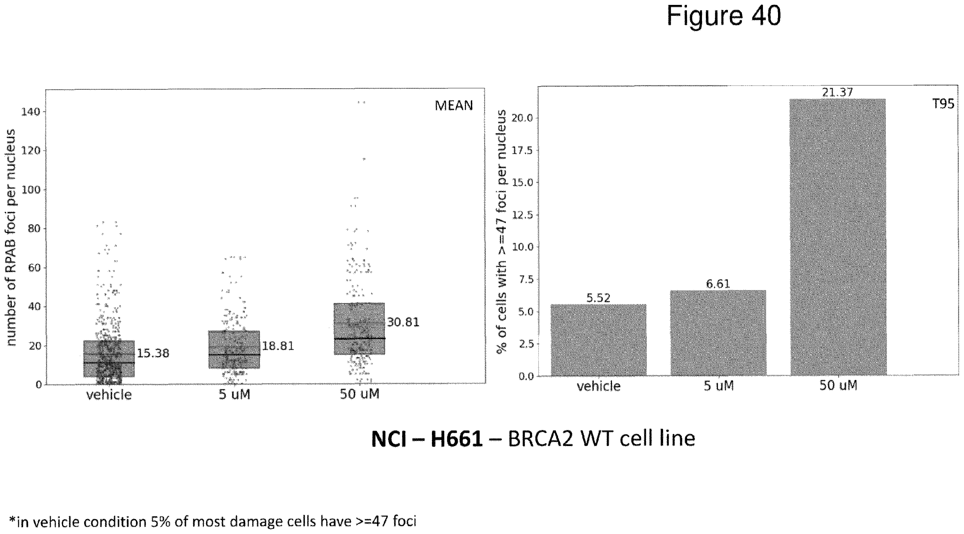

Figure 40 shows results of dSTRIDE RPA signals data analysis, measurement: level of double-strand DNA breaks associated with RPA70 after the treatment with etoposide.

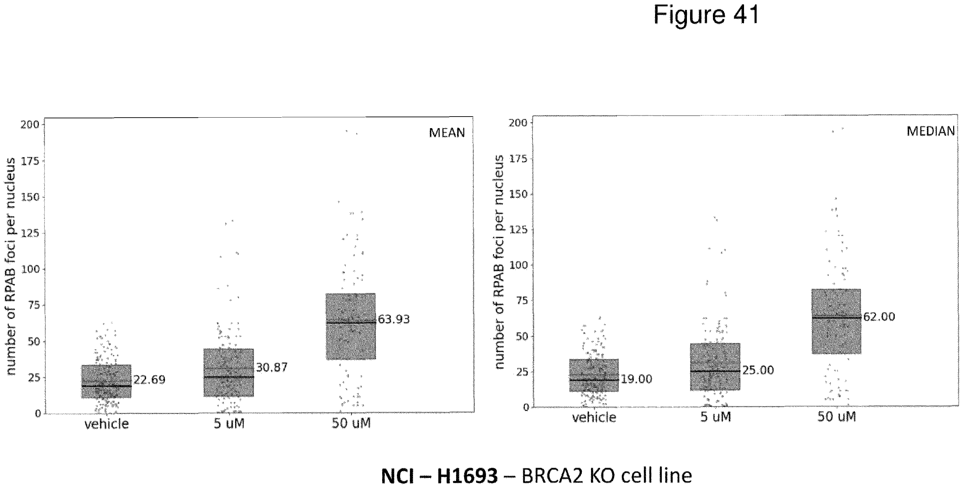

Figure 41 shows results of dSTRIDE RPA signals data analysis, measurement: level of double-strand DNA breaks associated with RPA70 after the treatment with etoposide.

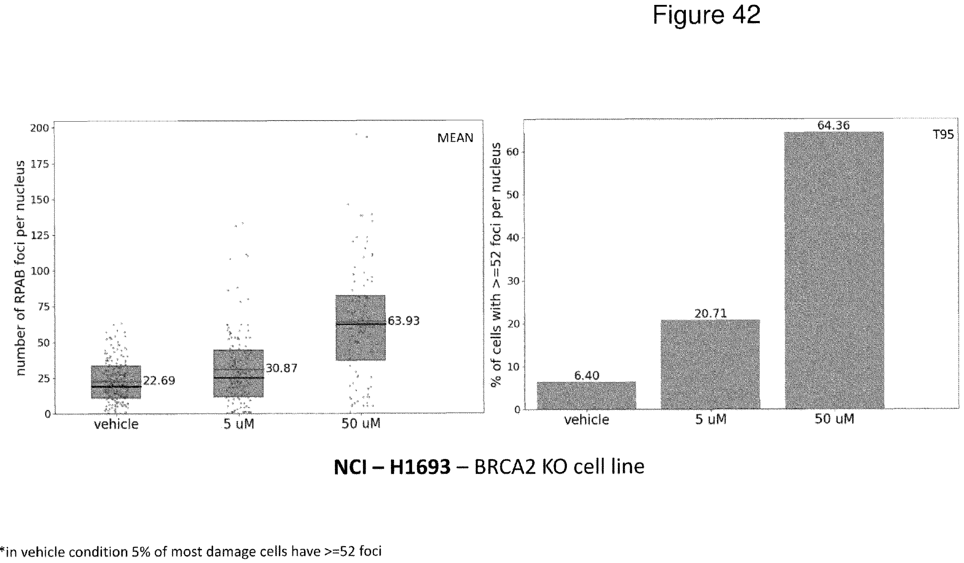

Figure 42 shows results of dSTRIDE RPA signals data analysis, measurement: level of double-strand DNA breaks associated with RPA70 after the treatment with etoposide.

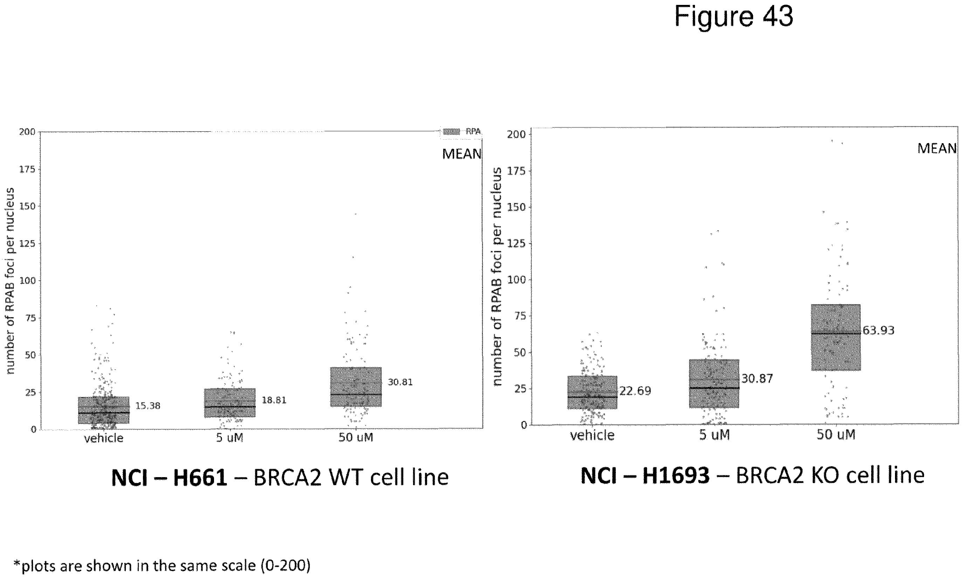

Figure 43 shows results of dSTRIDE RPA signals data analysis, cell line comparison.

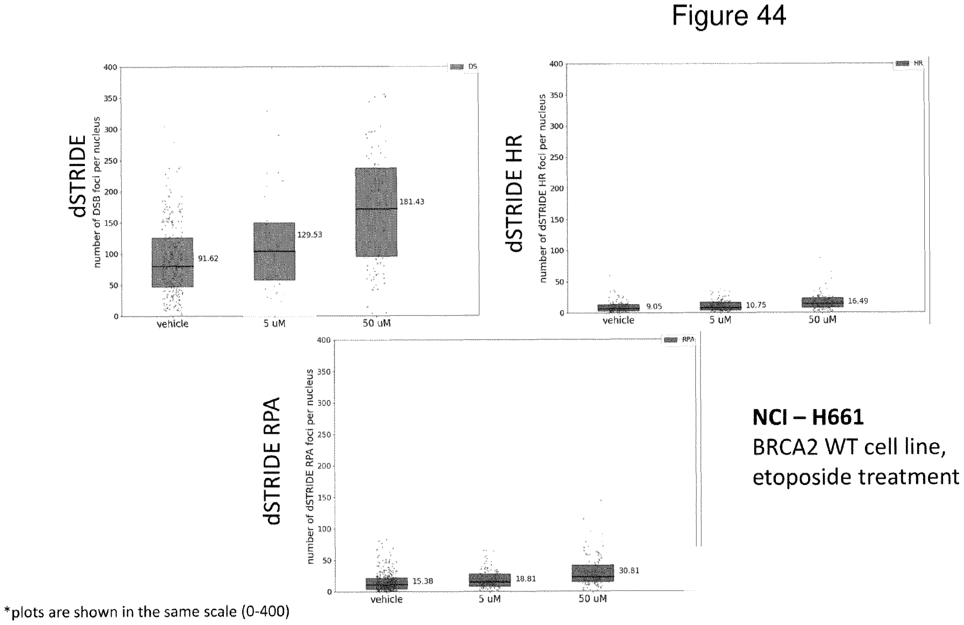

Figure 44 shows dSTRIDE, dSTRIDE-HR (Rad51) and dSTRIDE-RPA comparison in BRCA2 WT cell line with etoposide treatment.

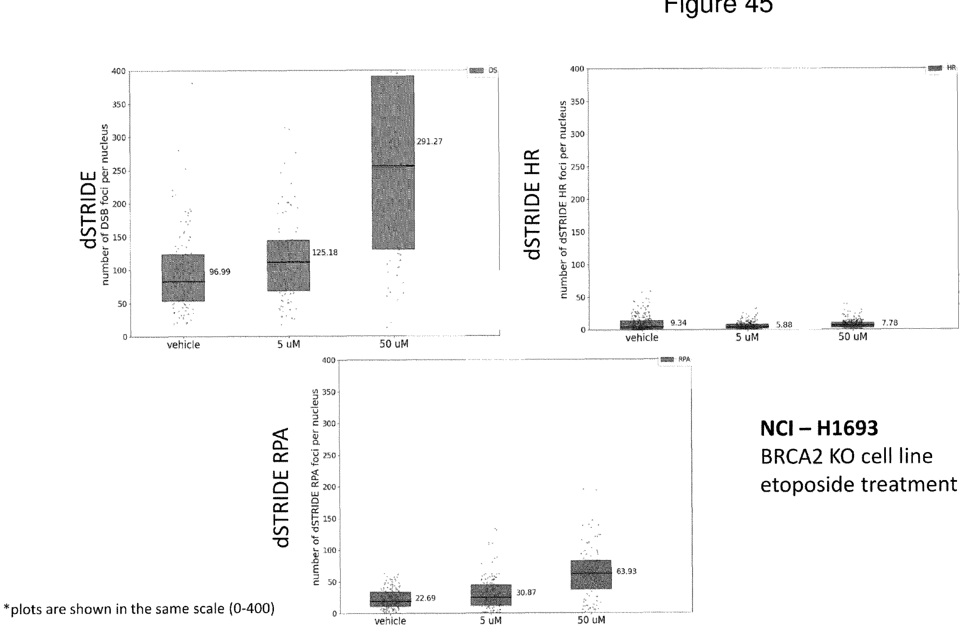

Figure 45 shows dSTRIDE, dSTRIDE-HR (Rad51) and dSTRIDE-RPA comparison in HR defective ("BRCA2 KO") cell line with etoposide treatment.

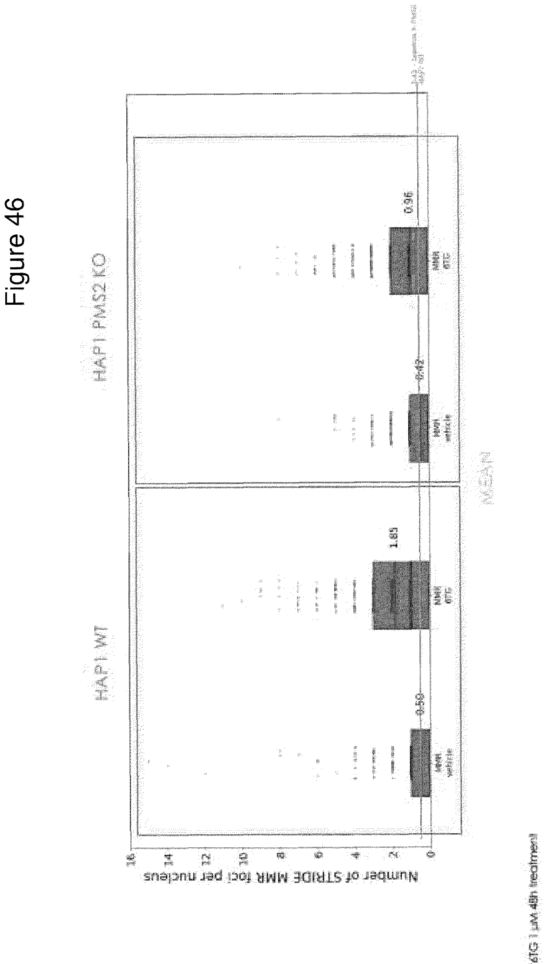

Figure 46 shows results of quantitative analysis in HAP1 cells (WT and KO) for sSTRIDE-MLH1.

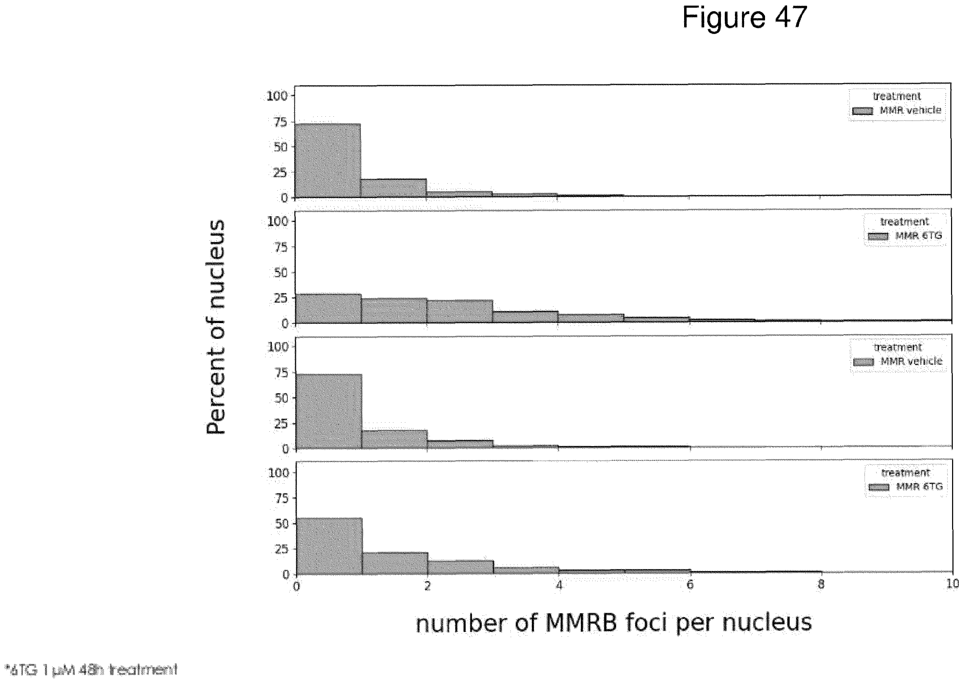

Figure 47 shows results of quantitative analysis in HAP1 cells for sSTRIDE-MLH1 with or without 6TG treatment.

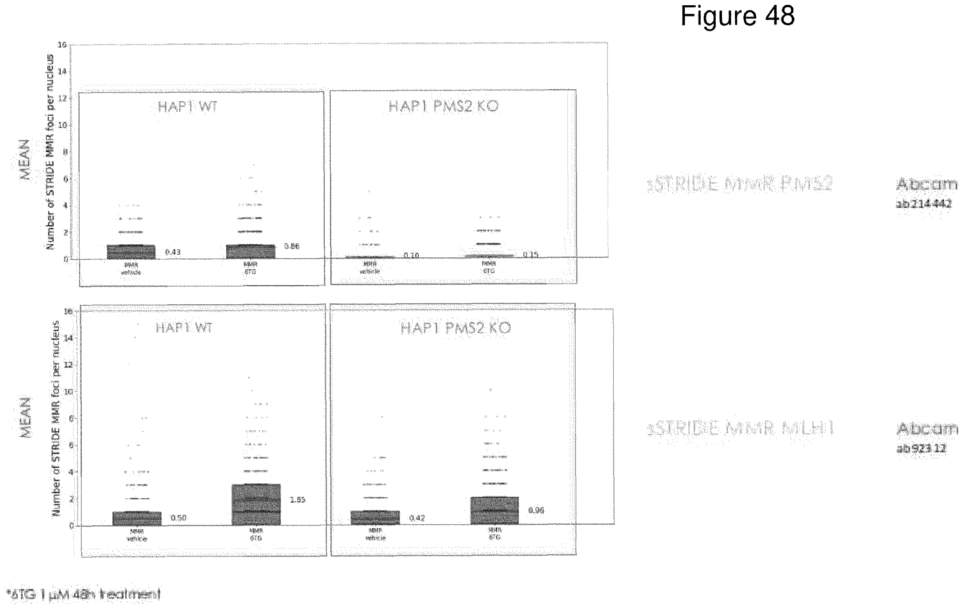

Figure 48 shows comparison between PMS2 and MLH1 results.

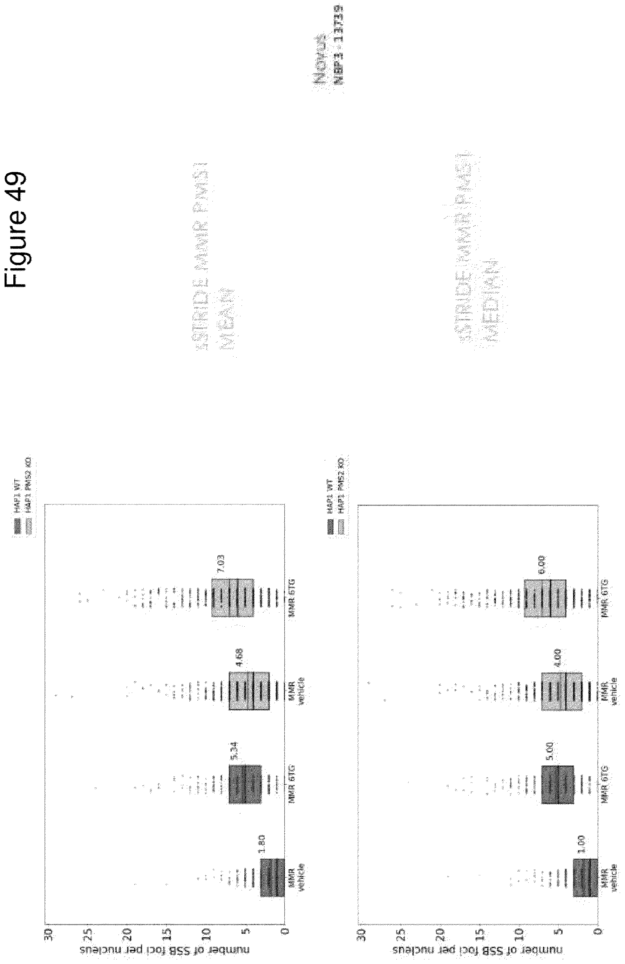

Figure 49 shows results of quantitative analysis in HAP1 cells (WT and KO) for sSTRIDE-PMS1.

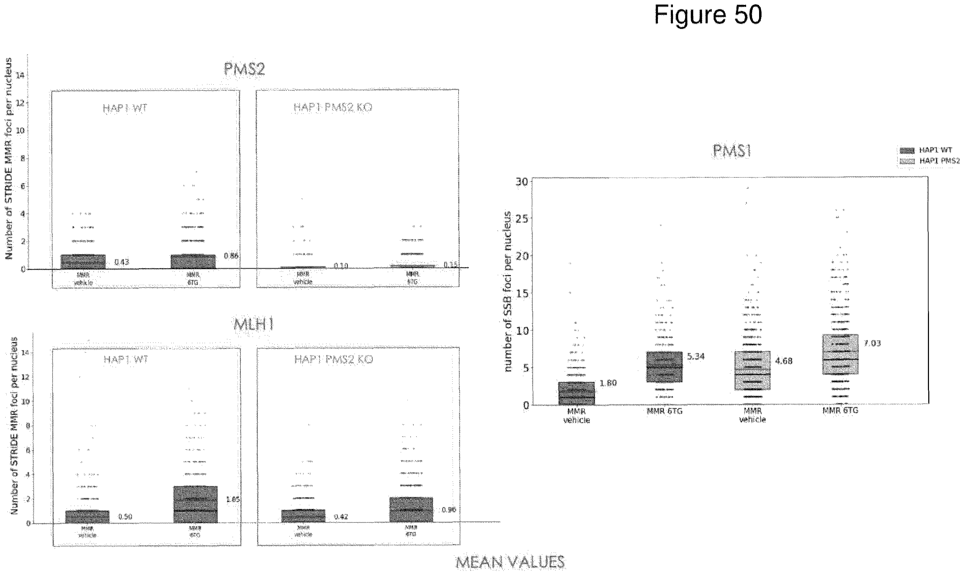

Figure 50 shows comparison between PMS2, PMS1 and MLH1 results.

### EXAMPLES

The present invention is explained more in detail with the aid of the following examples which are not intended to limit the scope of the present invention in any manner.

Examples are described on the basis of general protocols which concern main embodiments presented in the description.

### Example 1 - PMS1, PMS2, or MLH1 detection protocol

### Sample pre-treatment - optional

### (optional) fixation of biological material in 70% (v/v) ethanol

### Proteinase treatment

1. Proteinase K treatment of the biological material for 30 seconds (at a concentration of about 7 µg/mL(1:350 dilution of proteinase K stock concentration of 2.5 mg/mL), solution in PBS) at about 22 ºC (i.e. room temperature)
2. (optional) incubation with 1mM PMSF in PBS for 5 minutes at about 22 ºC
3. (optional) rinse with PBS

### DNA ends modification - optional

### 1. (optional) T4 PNK reaction

### Endogenous biotin blocking step - optional

1. (optional) 30 min incubation in the Streptavidin Blocking Solution at about 22 ºC
2. (optional) rinse with PBS
3. (optional) 30 min incubation in the Biotin Blocking Solution at about 22 ºC
4. (optional) rinse with PBS

### Biotin-modified nucleotides binding

5. Polymerase I reaction at 37 ºC with polymerase I enzyme, biotin-7-ATP, biotin-16-dUTP, biotin-16-dCTP, and dGTP
6. (optional) rinse with buffer

### Antibodies binding

1. (optional) 60 min incubation in Navinci Blocking Buffer at 37ºC
2. 60 min incubation in 1:100 rabbit monoclonal anti-PMS2 (or PMS1 or MLH1) antibody in Navinci Primary Antibody Diluent at about 22ºC
3. (optional) PBS rinse
4. 60 min incubation in 1:100 mouse monoclonal anti-biotin antibody in Navinci Primary Antibody Diluent at about 22ºC
5. (optional) PBS rinse

### PLA procedure

1. PLA probes binding reaction at 37ºC (Navinci PLA reagents and standard protocol)
2. Reaction A at 37ºC
3. Reaction B at 37ºC
4. Reaction C at 37ºC

### Reagent list - as used in Examples 1-3

**Table 1. Reagents details**

| **step** | **reagent** | **producer** | **serial number** |
|---|---|---|---|
| **proteinase** | Proteinase K | Novus Biological | NB900-66727 |
| | PMSF | Sigma-Aldrich | P7626 |
| **T4 PNK** | T4 PNK buffer | New England BioLabs | B0201S |
| | T4 PNK enzyme | New England BioLabs | M0201 |
| **Blocking** | Sheptavidyn | Invitrogen | 21390 |
| | Biotin | Invitrogen | E21390 |
| **Pol I reaction** | Pol I [3U] | New England BioLabs | M0209L |
| | Buffer [NEB2] | New England BioLabs | B7002S |
| | Biotin-7-dATP | Jena Bioscience | NU-835-BIO-L |
| | Biotin-16-dUTP | Jena Bioscience | NU-803-BIO-L |
| | Biotin-16-dCTP | Jena Bioscience | NU-809-BIO-16-L |
| | dGTP | Jena Bioscience | NU-1003S |
| **Blocking** | Blocking Buffer Navinci | Navinci | NaveniFlex MR |
| **PLA** | Probe Diluent | | |
| | Probe M1 | | |
| | Probe R2 | | |
| | Enzyme A | | |
| | Buffer A | | |
| | Enzyme B | | |
| | Buffer B | | |
| | Enzyme C | | |
| | Buffer C | | |

**Table 2. Antibody details**

| **antibody** | **Producer** | **serial number** |
|---|---|---|
| Anti-Biotin antibody [Hyb-8] Ms mAb to Biotin | Abcam | ab201341 |
| Rb pAb to Biotin | Abcam | ab53494 |
| Anti-BrdU antibody [MoBu-1] | Abcam | ab8039 |
| Rb pAb to BrdU | Abcam | ab152095 |
| Recombinant Anti-PMS2 antibody [EPR3947] | Abcam | ab214442 |
| Recombinant Anti-Rad51 antibody [EPR4030[3]] | Abcam | ab221796 |
| Recombinant Anti-RPA70 antibody [EPR3472] | Abcam | ab79398 |

### Example 2 - Rad51 detection protocol

### Sample pre-treatment - optional

### (optional) fixation of biological material in 70% (v/v) ethanol

### Proteinase treatment

1. Proteinase K treatment of the biological material for 60 seconds (at a concentration of about 7 µg/mL (1:350 dilution of proteinase K stock concentration of 2.5 mg/mL), solution in PBS) at about 22 ºC (i.e. room temperature)
2. (optional) incubation with 1mM PMSF in PBS for 5 minutes at about 22 ºC
3. (optional) rinse with PBS

### DNA ends modification - optional

### 1. (optional) T4 PNK reaction

### TdT reaction - BrdU incorporation

1. TdT reaction at 37ºC with BrdU and TdT enzyme
2. (optional) rinse with PBS

### Antibodies binding

6. (optional) 60 min incubation in Navinci Blocking Buffer at 37ºC
7. 60 min incubation in 1:100 rabbit monoclonal anti-Rad51 antibody in Navinci Primary Antibody Diluent at about 22ºC
8. (optional) PBS rinse
9. 60 min incubation in 1:100 mouse monoclonal anti-BrdU antibody in Navinci Primary Antibody Diluent at about 22ºC
10.(optional) PBS rinse

### PLA procedure

1. PLA probes binding reaction at 37ºC (Navinci PLA reagents and standard protocol)
2. Reaction A at 37ºC
3. Reaction B at 37ºC
4. Reaction C at 37ºC

### Example 3- RPA70 detection protein

### Sample pre-treatment - optional

### 1. (optional) fixation of biological material in 70% (v/v) ethanol

### Proteinase treatment

1. Proteinase K treatment of the biological material for 60 seconds (at a concentration of about 7 µg/mL (1:350 dilution of proteinase K stock concentration of 2.5 mg/mL, solution in PBS) at about 22 ºC (i.e. room temperature)
2. (optional) incubation with 1mM PMSF in PBS for 5 minutes at about 22 ºC
3. (optional) rinse with PBS

### DNA ends modification - optional

### 1. (optional) T4 PNK reaction

### TdT reaction - BrdU incorporation

1. TdT reaction at 37ºC with BrdU and TdT enzyme
2. (optional) rinse with PBS

### Antibodies binding

1. (optional) 60 min incubation in Navinci Blocking Buffer at 37ºC
2. 60 min incubation in 1:100 rabbit monoclonal anti-RPA70 antibody in Navinci Primary Antibody Diluent at about 22ºC
3. (optional) PBS rinse
4. 60 min incubation in 1:100 mouse monoclonal anti-BrdU antibody in Navinci Primary Antibody Diluent at about 22ºC
5. (optional) PBS rinse

### PLA procedure

1. PLA probes binding reaction at 37ºC (Navinci PLA reagents and standard protocol)
2. Reaction A at 37ºC
3. Reaction B at 37ºC
4. Reaction C at 37ºC

### Example 4 - optimisation of protease treatment

### Aim of the experiments

Experimental setup and optimization: detection and quantitative analysis of PMS2-active DNA single strand breaks in human cells U2OS WT.

### Methods

### U2OS cells

USOS cells were cultured in T25 Falcons (Nunc) in DMEM HG medium (Gibco) containing 10% FBS (Gibco) and 100 U/mL penicillin and 100 µg/mL streptomycin (Gibco).

### Antigen retrieval method - proteinase K treatment

Proteinase K were used from freshly opened vial. Number of antigen retrieval methods corresponds to the numbers in the table below.
1. v1 proteinase K - 2 min incubation,
2. v2 proteinase K - 1 min incubation,
3. v3 no proteinase,
4. v4 proteinase K - 15s incubation,
5. v5 proteinase K - 30s incubation,
6. v6 proteinase K - 90s incubation,
7. v7 proteinase K - 180s incubation.

### PMS2 antibody

Rabbit Recombinant Anti-PMS2 antibody [EPR3947] - BSA and Azide free (ab214442) Mouse monoclonal Anti-PMS2 Antibody (B-3): sc-25315

### Tested condition - five optimization experiments

| **sample** | **cell line** | **assay** | **compound** | **treatment time (h)** | **antigen retrieval** |
|---|---|---|---|---|---|
| EXP1 | | | | | |
| 1 | U2OS WT | IF PMS2 | vehicle | 0 | X |
| 2 | U2OS WT | IF PMS2 | 6TG | 48h | X |

| EXP2 | | | | | |
|---|---|---|---|---|---|
| 3 | U2OS WT | As in Example 1 | vehicle | 0 | 1 |
| 4 | U2OS WT | As in Example 1 | 6TG | 48h | 1 |

| EXP3 | | | | | |
|---|---|---|---|---|---|
| 5 | U2OS WT | As in Example 1 | vehicle | 0 | 2 |
| 6 | U2OS WT* | As in Example 1 | vehicle | 0 | 2 |
| 7 | U2OS WT* | As in Example 1 | vehicle | 0 | 2 |
| 8 | U2OS WT^ | As in Example 1 | vehicle | 0 | 2 |

| EXP4 | | | | | |
|---|---|---|---|---|---|
| 9 | U2OS WT | As in Example 1 | vehicle | 0 | 3 |
| 10 | U2OS WT* | As in Example 1 | vehicle | 0 | 3 |
| 11 | U2OS WT^ | sSTRIDE | vehicle | 0 | 3 |
| 12 | U2OS WT | As in Example 1 | 6TG | 48 | 3 |
| 13 | U2OS WT* | As in Example 1 | 6TG | 48 | 3 |
| 14 | U2OS WT^ | sSTRIDE | 6TG | 48 | 3 |

| | | | | | |
|---|---|---|---|---|---|
| * negative controls ^ positive controls | | | | | |

In sSTRIDE method all steps of Example 1 are performed with the exception that the method does not rely on the detection of the intrinsic nucleic acid binding molecules, and instead focuses on detection of nucleic acid end(s) only. Thus, in the sSTRIDE method, the standard procedure is to apply proteinase K treatment for 3 min (same concentration, same temperature as in Example 1). The (optional) step of endogenous biotin blocking is performed for 15 min (each) instead of 30 min (each). Instead of anti-PMS2 antibodies and anti-biotin antibodies, two anti-biotin antibodies are used (each having different affinity for and/or different binding site on the biotin molecule).

In each experiment the same reagents were used (see reagents of Example 1).

Streptavidin/biotin endogenous biotin kit were the same in each experiment.

The same proteinase K batch was used to each sample.

### Staining procedure Abcam antibody

Samples were proceeded according to sSTRIDE (as above) standard protocols with following modifications:
1. Different antigen retrieval methods were tested.
2. One of the biotin antibodies were replaced by PMS2 antibody - assay sSTRIDE MMR.
3. Positive control - standard sSTRIDE assays were performed.
4. Negative controls were performed - one of the antibodies was discarded. Incubation was proceeded on 1% BSA solution without antibody.

**Table 3. Method particulars. Rb - rabbit antibody (host), Ms - mouse antibody (host)**

| | |
|---|---|
| **Experiment code** | sSTRIDE MMR optimization |
| **Design** | in vitro |
| **STRIDE** | **sSTRIDE MMR PMS2** |
| **LB1** | 45 min |
| **EDTA 10mM** | 15 min |
| **EDTA 50mM** | 7 min |
| **Proteinase conditions** | table below - differs between experiments |
| **Biotin/streptavidin** | 15 min |
| **mono Ab anti-PMS2 Rb** | 1:100, 60 min (Abcam) |
| **poly Ab anti-biotin Ms** | 1:100, 60 min (Abeam) |
| **Counterstaining** | DAPI 500 nM, 15 min |
| **Treatment** | 6TG 100nM 48h, vehicle |
| **Additional staining** | x |

### Staining procedure Santa Cruz Biotechnology antibody

Samples were proceeded according to sSTRIDE standard protocols with following modifications:
1. Different antigen retrieval methods were tested.
2. One of the biotin antibodies were replaced by PMS2 antibody - assay sSTRIDE MMR.
3. Positive control - standard sSTRIDE assays were performed.
4. Negative controls were performed - one of the antibodies was discarded. Incubation was proceeded on 1% BSA solution without antibody.

**Table 4. Method particulars. Rb - rabbit antibody (host), Ms - mouse antibody (host)**

| | |
|---|---|
| **Experiment code** | sSTRIDE MMR optimization |
| **Design** | in vitro |
| **STRIDE** | **sSTRIDE MMR PMS2** |
| **LB1** | 45 min |
| **EDTA 10mM** | 15 min |
| **EDTA 50mM** | 7 min |
| **Proteinase conditions** | table below - differs between experiments |
| **Biotin/streptavidyn** | 15 min |
| **mono Ab anti-PMS2 Ms** | 1:100, 60 min (Santa Cruz Biotechnology) |
| **poly Ab anti-biotin Rb** | 1:100, 60 min (Abcam) |
| **Counterstaining** | DAPI 500 nM, 15 min |
| **Treatment** | 6TG 100nM 48h, vehicle |
| **Additional staining** | x |

### Results, part 1

**Table 5. Tested conditions**

| EXP2 | | | | | |
|---|---|---|---|---|---|
| nr | sample | treatment | 1°antibody | 2°antibody | antigen retrieval |
| 3 | As in Example 1 - sSTRIDE MMR | vehicle | PMS2 Rb | Biotin Ms | 1 |
| 4 | As in Example 1 - sSTRIDE MMR | 6TG | PMS2 Rb | Biotin Ms | 1 |

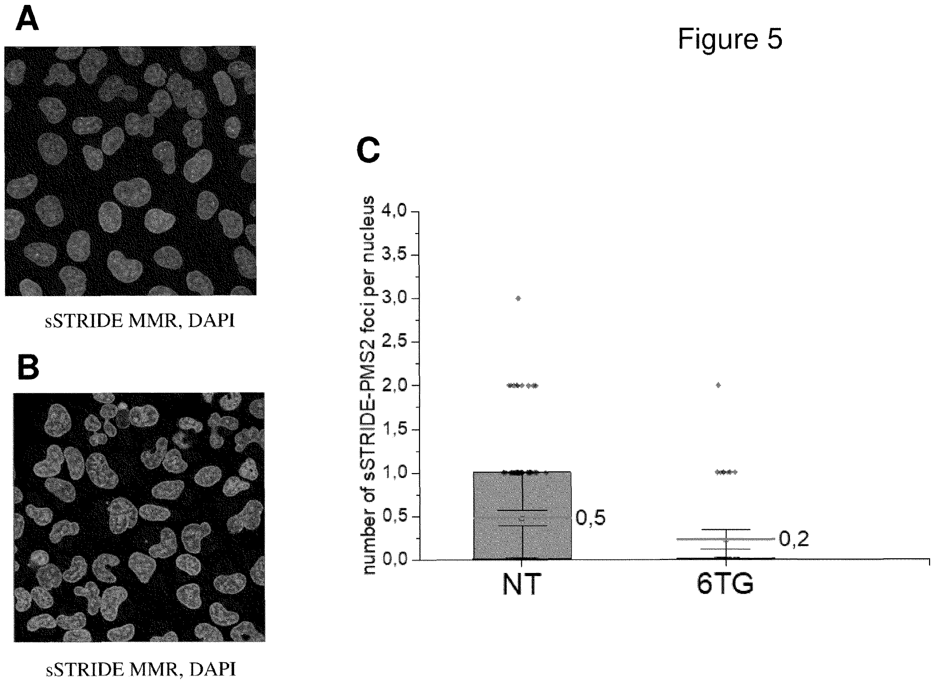

Figure 5A - Sample 3 - NT cells sSTRIDE MMR (Example 1) staining. Low amount of the foci was observed, maximum number is 3 foci per nucleus (antigen retrieval 1).

Figure 5B - Sample 4 - 6TG treated cells, sSTRIDE MMR (Example 1) staining. Low amount of the foci was observed, maximum number is 3 foci per nucleus (antigen retrieval 1).

Figure 5C- Results of analysis - antigen retrieval 1 (proteinase K 2 min). Quantitative image analysis shows that the number of foci detected is low and no response after 6TG treatment is observed.

**Table 6. Tested conditions**

| EXP3 | | | | | |
|---|---|---|---|---|---|
| nr | sample | treatment | 1°antibody | 2°antibody | antigen retival |
| 5 | As in Example 1 - sSTRIDE MMR | vehicle | PMS2 Rb | Biotin Ms | 2 |
| 6 | As in Example 1 - sSTRIDE MMR* | vehicle | BSA | Biotin Ms | 2 |
| 7 | As in Example 1 - sSTRIDE MMR * | vehicle | PMS2 Rb | BSA | 2 |
| 8 | As in Example 1 - sSTRIDE MMR ^ | vehicle | Biotin Rb | Biotin Ms | 2 |

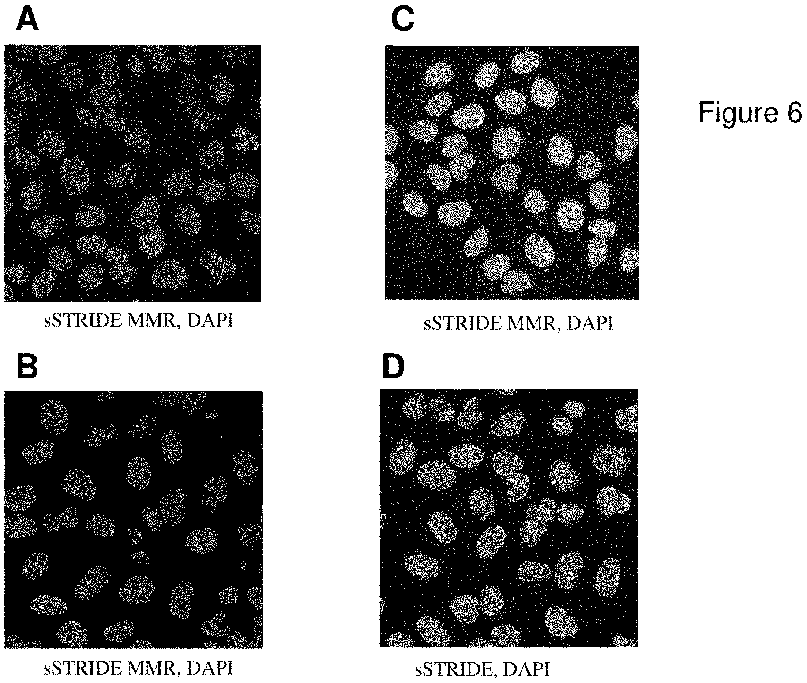

Figure 6A - Sample 5 - non-treated U2OS WT, sSTRIDE MMR staining. More foci are observed in antigen retrieval method 2 comparing to method 1. Low amount of the background signal is detected (antigen retrieval 2).

Figure 6B - Sample 6 - non-treated U2OS WT cells, sSTRIDE MMR staining negative control (only anti-biotin antibody). Single foci can be detected (antigen retrieval 2).

Figure 6C - Sample 7 - non-treated U2OS WT cells, sSTRIDE MMR staining negative control (only PMS2 antibody). No foci is detected, high background signal is visible (antigen retrieval 2).

Figure 6D - Sample 8 - non-treated U2OS WT cells, sSTRIDE standard assay. Lots of foci can be detected in single nucleus - more than 20 per nucleus (antigen retrieval 2).

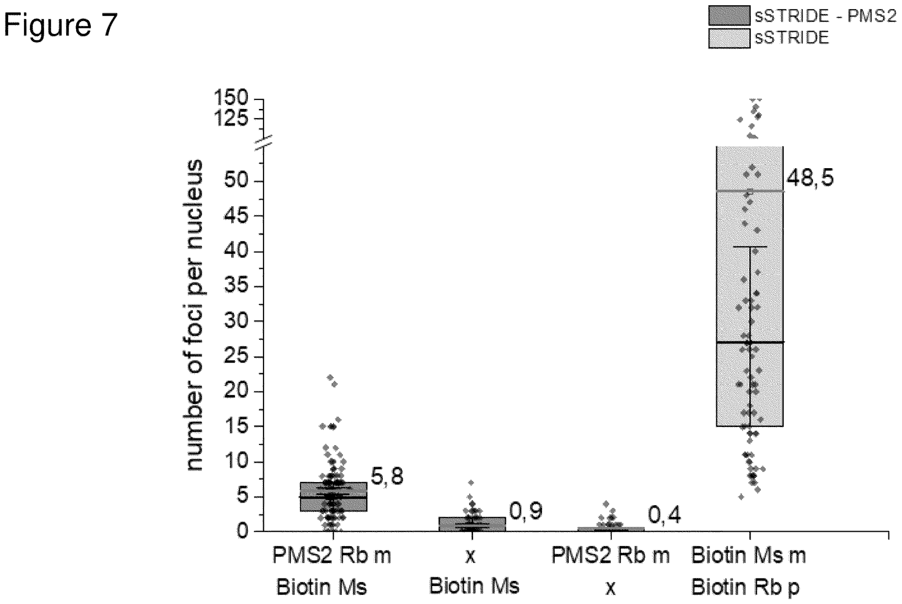

Figure 7 - Results of analysis - antigen retrival 2 (proteinase K 1 min). Quantitative image analysis showing comparison of readouts in U2OS cells after labelling with sSTRIDE and sSTRIDE-MMR assays and negative controls. The results show that sSTRIDE-MMR foci constitute approximately 12% of total SSBs, while the signals in negative controls are kept at a low level.

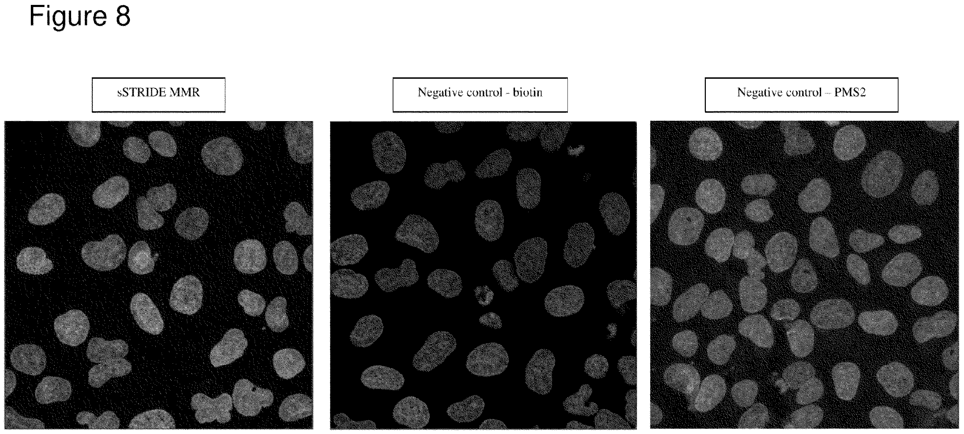

Figure 8 - Comparison between sSTRIDE MMR assay and the negative controls. Samples were stained and imaged in the same day. Comparison of the foci intensity and frequency (U2OS WT cells, NT, antigen retrieval 2). The images show that very low numbers of foci were detected in the negative controls when one of the antibodies (targetin biotin or PMS2) was absent.

**Table 7. Tested conditions**

| EXP4 | | | | | |
|---|---|---|---|---|---|
| nr | sample | treatment | 1°antibody | 2°antibody | antigen retival |
| 9 | As in Example 1 - sSTRIDE MMR | Vehicle | PMS2 Rb | Biotin Ms | 3 |
| 10 | sSTRIDE MMR | Vehicle | PMS2 Rb | BSA | 3 |
| 11 | sSTRIDE | Vehicle | Biotin Rb | Biotin Ms | 3 |
| 12 | sSTRIDE MMR | 6TG | PMS2 Rb | Biotin Ms | 3 |
| 13 | sSTRIDE MMR | 6TG | PMS2 Rb | BSA | 3 |
| 14 | sSTRIDE | 6TG | Biotin Rb | Biotin Ms | 3 |

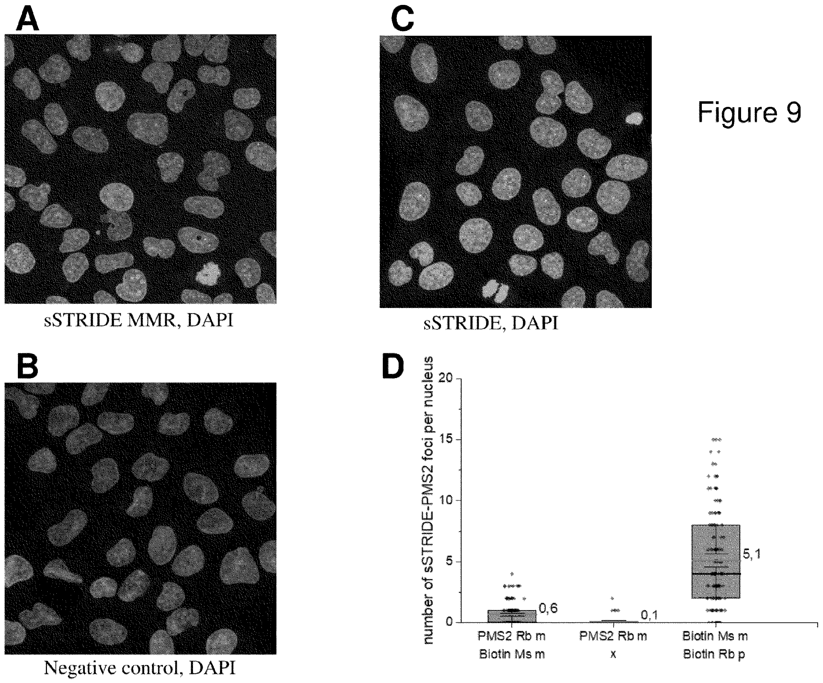

Figure 9A - Sample 9 - non-treated U2OS WT cells, sSTRIDE MMR standard assay. Single foci can be detected in the nucleus, high background intensity is observed (antigen retrieval 3).

Figure 9B - Sample 10 - non-treated U2OS WT cells, sSTRIDE MMR assay negative control (PMS2 antibody only). No signals can be detected (antigen retrieval 3).

Figure 9C - Sample 11 - non-treated U2OS WT cells, sSTRIDE MMR assay positive control (standard procedure). More than 10 signals per nucleus can be detected (antigen retrieval 3).

Figure 9D - Results of analysis - antigen retrieval 3 (no proteinase K). Results of quantitative image analysis shows that in the lack of proteinase treatment the number of foci detected in sSTRIDE-MMR (PMS2) and sSTRIDE is low.

### Summary - part 1

In the studies the best antigen retrieval method for sSTRIDE MMR PMS2 turned up to be method 2 (1min incubation with proteinase K). In case of the method 1 and the method 3 to less foci were detected.

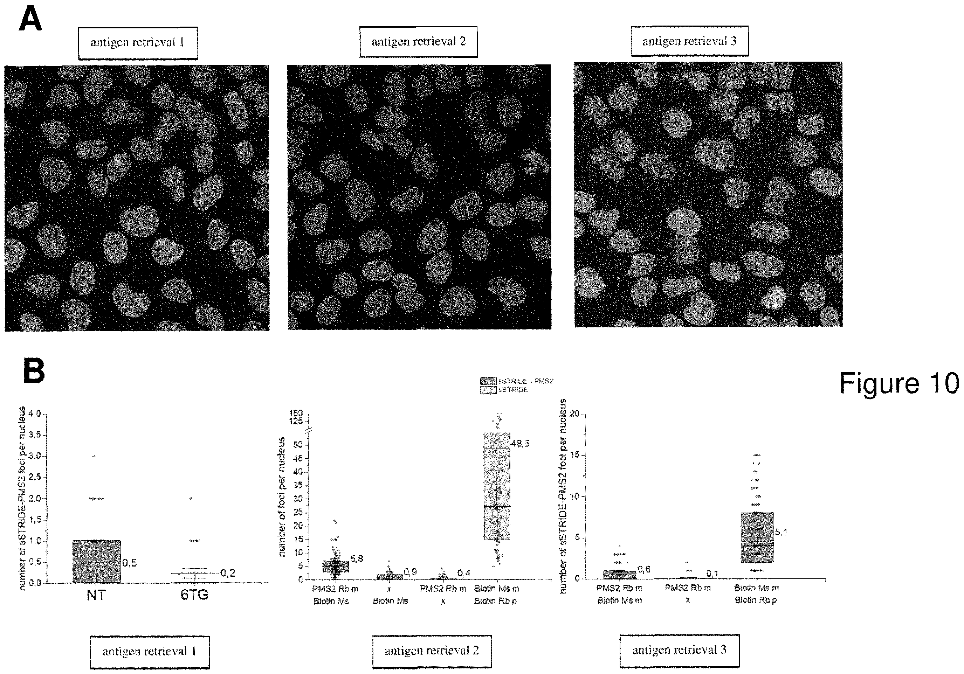

Figure 10 A - Comparison of three antigen retrieval methods (U2OS WT non-treated cells). Representative images showing the result of labelling performed with sSTRIDE-MMR assay with different antigen retrieval conditions. Different number of fluorescence foci are visible for various antigen retrieval conditions with the most foci for antigen retrieval v2.

Figure 10 B - Results of analysis - dependence on antigen retrieval. Quantitative image analysis confirms that the highest number of foci is detected when method 2 is applied.

sSTRIDE assay without proteinase (antigen retrieval 3) is less effective than assay with proteinase condition. The amount of foci per nucleus is 90% lower than after 1 min proteinase treatment (antigen retrieval 3).

The background level from PMS2 antibody is neglectable in the assay in each condition.

The background level from Biotin antibody cannot be neglectable in the assay. The average count of foci per nucleus is 0,9, however in some nucleus the count level is higher than 5.

Differences between foci number and morphology were detected through the assays.

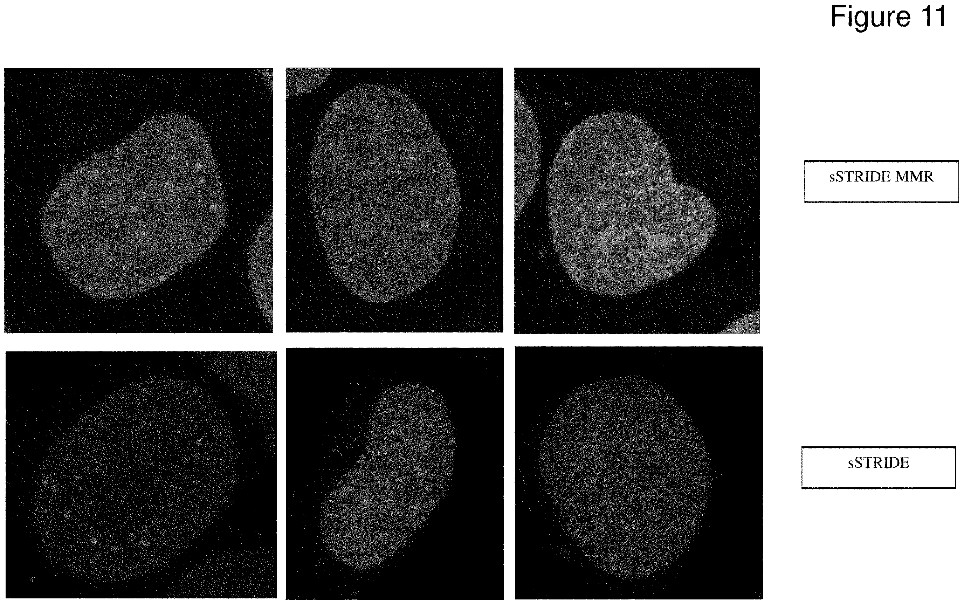

Figure 11- Comparison of the foci intensity and frequency. In the upper photos sSTRIDE MMR foci are shown, in lower sSTRIDE assay foci are shown. sSTRIDE MMR foci are more intensive but low frequent (U2OS WT cells, NT, antigen retrieval 1).

### Conclusions, part 1

1. Antigen retrieval method has high impact of sSTRIDE normal assay and sSTRIDE MMR2 assay.
2. Antigen retrieval step is relevant in assay sSTRIDE MMR PMS2 - without proteinase assay does not work.
3. The best tested way of antigen retrieval is condition - proteinase K 1 min.
4. The background level from biotin antibody is not neglectable - the negative control with biotin antibody is relevant in each sSTRIDE MMR PMS2 staining.

### Results, part 2

**Table 8. Tested conditions**

| EXP5 | Abeam Ab | | | | |
|---|---|---|---|---|---|
| nr | sample | treatment | 1°antibody | 2°antibody | antigen retival |
| 15 | As in Example 1 - sSTRIDE MMR | 6TG, 250 nM, 24h | PMS2 | biotin | v2 |
| 16 | As in Example 1 - sSTRIDE MMR | 6TG, 500 nM, 24h | PMS2 | biotin | v2 |
| 17 | As in Example 1 - sSTRIDE MMR | 6TG, 750 nM, 24h | PMS2 | biotin | v2 |
| 18 | As in Example 1 - sSTRIDE MMR | 6TG, 1 µM, 24h | PMS2 | biotin | v2 |
| 19 | As in Example 1 - sSTRIDE MMR | 6TG, 5 µM, 24h | PMS2 | biotin | v2 |
| 20 | As in Example 1 - sSTRIDE MMR | 6TG, 10 µM, 24h | PMS2 | biotin | v2 |
| 21 | As in Example 1 - sSTRIDE MMR | 6TG, 250 nM, 48h | PMS2 | biotin | v2 |
| 22 | As in Example 1 - sSTRIDE MMR | 6TG, 500 nM, 48h | PMS2 | biotin | v2 |
| 23 | As in Example 1 - sSTRIDE MMR | 6TG, 750 nM, 48h | PMS2 | biotin | v2 |
| 24 | As in Example 1 - sSTRIDE MMR | 6TG, 1 µM, 48h | PMS2 | biotin | v2 |
| 25 | As in Example 1 - sSTRIDE MMR | 6TG, 5 µM, 48h | PMS2 | biotin | v2 |
| 26 | As in Example 1 - sSTRIDE MMR | 6TG, 10 µM, 48h | PMS2 | biotin | v2 |
| 27 | As in Example 1 - sSTRIDE MMR | vehicle | PMS2 | biotin | v4 |
| 28 | As in Example 1 - sSTRIDE MMR | vehicle | PMS2 | biotin | v5 |
| 29 | As in Example 1 - sSTRIDE MMR | vehicle | PMS2 | biotin | v2 |
| 30 | As in Example 1 - sSTRIDE MMR | vehicle | PMS2 | biotin | v6 |
| 31 | sSTRIDE | vehicle | biotin | biotin | v2 |
| 32 | sSTRIDE | vehicle | biotin | biotin | v7 |
| 33 | As in Example 1 - sSTRIDE MMR | vehicle | w/o PLA | | v2 |
| 34 | As in Example 1 - sSTRIDE MMR | vehicle | w/o polymerase I | | v2 |
| 35 | As in Example 1 - sSTRIDE MMR | vehicle | w/o nukleotides | | v2 |
| 36 | As in Example 1 - sSTRIDE MMR | vehicle | w/o polymerase I reaction | v2 | |
| 37 | As in Example 1 - sSTRIDE MMR | vehicle | w/o antibodies | v2 | |

Samples 15-18 and 21 were not imagined in 3D because maximum amount of observed foci per nucleus were 2-3, so this condition cannot be the positive control of the assay.

### Conclusions, part 2

6TG treated cells did not respond in anticipated way. The readouts level is lower than in the non-treated samples. In each condition antigen retrieval method 2 were used. However, in some cells, foci were very bright and big, which can lead to conclusion that MMR is more intensive in specific spots and detected foci are multiple.

Figure 12A - Foci intensity and frequency - (U2OS WT cells, 48h treatment, antigen retrieval 2). The morphology of the foci is as expected with a high signal to noise ratio.

Figure 12B - Antigen retrieval methods comparison - Abeam antibody. Sample 27-30 - U2OS WT cells treated 48h by 6TG in different concentration, sSTRIDE MMR assay positive control (antigen retrieval method v2). Treatment did not significantly increase number of foci in the nucleus.

Figure 13A - Antigen retrieval methods comparison. Results of quantitative analysis - comparison of antigen retrieval methods.

Figure 13B - Results of analysis - ratio between sSTRIDE MMR and sSTRIDE normal procedure. The most promising antigen retrieval method is v5 - 30s. In this sample the distribution of the signal is the other - some group of nucleus has more than 40 foci per nucleus.

Figure 13C - Sample 29 - U2OS WT non-treated cells - antigen retrieval method v5. Antigen retrieval v5 was chosen as the best antigen retrieval method.

Figure 14A- Negative controls - Abeam antibody. Sample 36-39 - U2OS WT non-cells treated 48h, sSTRIDE MMR assay negative controls (antigen retrieval method v2). In each enzymatic reaction marked agent was omitted.

Figure 14B - Results of quantitative analysis - negative controls (antigen retrieval 2). Results of quantitative analysis - comparison of antigen retrieval methods.

In the graph the result of counts from sample 37 is not shown. The number of SSB per nucleus is 3.8. The number of foci in most controls can be neglectable. However, the number of SSB in negative controls with polymerase I can lead to conclusion, that in the assay endogenous biotin blocking time should be increased.

The ratio between sSTRIDE MMR assay and procedure also show significant amount of signals in negative control with omitted polymerase I enzyme (no shown in the plot - ratio is 0.49).

Figure 17C- Results of analysis - ratio between negative controls and sSTRIDE MMR. In control sample omitted were: - N1 - PLA, N2 - nucleotides (nts), N3 - poll reaction, N4 - antibodies (abs), N5 - anti-PMS2, N6 - anti-biotin.

### Results, part 3

**Table 9. Tested conditions**

| EXP6 | Santa Cruz Biotechnology antibody | | | | |
|---|---|---|---|---|---|
| Nr | Sample | treatment | 1°antibody | 2°antibody | antigen retrieval |
| 38 | As in Example 1 - sSTRIDE MMR | 6TG, 1 µM, 48h | PMS2 Ms | Biotin Rb | v2 |
| 39 | As in Example 1 - sSTRIDE MMR | 6TG, 10 µM, 48h | PMS2 Ms | Biotin Rb | v2 |
| 40 | As in Example 1 - sSTRIDE MMR | vehicle | PMS2 Ms | Biotin Rb | v5 |
| 41 | As in Example 1 - sSTRIDE MMR | vehicle | PMS2 Ms | Biotin Rb | v2 |
| 42 | As in Example 1 - sSTRIDE MMR | vehicle | PMS2 Ms | Biotin Rb | v6 |
| 43 | sSTRIDE | vehicle | Biotin Ms | Biotin Rb | v2 |
| 44 | sSTRIDE | vehicle | Biotin Ms | Biotin Rb | v7 |
| 45 | As in Example 1 - sSTRIDE MMR | vehicle | w/o PLA | | v2 |
| 46 | As in Example 1 - sSTRIDE MMR | vehicle | w/o enzyme polimerase I | | v2 |
| 47 | sSTRIDE As in Example 1 - sSTRIDE MMR | vehicle | w/o nucleotides | | v2 |
| 48 | As in Example 1 - sSTRIDE MMR | vehicle | w/o polymerase I reaction | | v2 |
| 49 | As in Example 1 - sSTRIDE MMR | vehicle | w/o antibodies | | v2 |
| 50 | As in Example 1 - sSTRIDE MMR | vehicle | x | Biotin Rb | v2 |
| 51 | As in Example 1 - sSTRIDE MMR | vehicle | PMS2 Ms | x | v2 |
| 52 | As in Example 1 - sSTRIDE MMR | vehicle | w/o nucleotides | | v2 |
| 53 | As in Example 1 - sSTRIDE MMR | vehicle | w/o polymerase I reaction | | v2 |

Sample 39 was not imaged in 3D because maximum amount of observed foci per nucleus were 2-3, so this condition cannot be the positive control of the assay.

Samples 52 and 53 were stained by Abeam antibody. In the procedure endogenous biotin blocking time was changed to 30 minutes (15 minutes longer than in previous experiments).

Figure 15A -Sample 40-43 - untreated U2OS WT cells labelled with sSTRIDE-MMR (40-42) and sSTRIDE (43), different antigen retrieval methods were tested.

Figure 15B - Results of quantitative analysis - antigen retrieval. Results of analysis - ratio between sSTRIDE normal procedure and sSTRIDE MMR.

Figure 15C - In the experiment 05 the most promising versions of antigen retrieval procedure were tested. The antigen retrieval v5 gives the highest amount of the signal per nucleus (the same version was chosen in experiments with Abeam antibody).

Figure 16A - Negative controls - impact of endogenous biotin time blocking for the redout level. Results of quantitative analysis - comparison of controls with different biotin blocking time - in red frame 30 min blocking time, without frame 15 min blocking time.

Figure 16B - Samples 46 and 52 - U2OS WT cells, sSTRIDE MMR assay negative controls. Difference between readout level depending on blocking time.

Figure 171 A - Comparison - antigen retrieval methods. v2 - 60s, v4 - 15s, v5 - 30s, v6 - 90s. The highest readout is seen for 30 seconds of treatment with proteinase K. Higher readouts are seen for the Santa Cruz Biotechnology antibody.

Figure 17B - Comparison between ratio sSTRIDE MMR/sSTRIDE. The ratio is the highest for both antibodies for antigen retrieval v5 - 30 seconds of treatment with proteinase K.

Figure 18 A - Negative control ratio comparison sSTRIDE MMR/sSTRIDE (abcam antibody). In control omitted: - N1 - PLA, N2 - nucleotides, N3 - poll reaction, N4 - antibodies, N5 - anti-PMS2, N6 - anti-biotin. The ratio for all negative controls is at a very low level confirming the specificity of the assay.

Figure 18 B - Negative control ratio comparison sSTRIDE MMR/sSTRIDE (Santa Cruz Biotech antibody). The ratio is higher than in the case of the Abeam antibody which show that the Santa Cruz Biotechnology antibody is characterized by more non-specific binding.

### Conclusions, part 3

Both Abeam and Santa Cruz Biotechnology antibodies can be used in the sSTRIDE-MMR assay. Highest readouts are obtained for Santa Cruz Biotechnology, however, this antibody also results in higher readouts in negative controls. Increasing the time of endogenous biotin blocking may help decrease the background seen in negative controls.

### Example 5- Development of sSTRIDE-MMR (PMS2) assay variant

### A. Cell culture conditions HAP1 cells

HAP1 cell line is a human near-haploid cell line derived from the chronic myelogenous leukemia (CML). The advantages of HAP1 cell line in assay development research are: one copy of any particular allel, rapid doubling time and ease of transfection. HAP1 parental and HAP1 PMS2 knockout cell line are offered as the isogenic pair. Knockout of PMS2 protein was edited by CRISPR/Cas to contain a 1bp insertion in a coding exon of PMS2.

HAP1 cells and HAP1 PMS2 KO cells were purchased from Horizon Discovery cell culture collection (18.05.2022). The detailed cell culture description is listed on Table10. Cells were grown at 37°C with 5% CO2 in T75 flasks with IMDM + 10% FBS + 1% Penicillin - Streptomycin Solution (10,000 units ea.).

The master bank collection was prepared form #1 passage performed in intoDNA laboratory.

Banking procedure was performed as described below. Cell suspension was centrifuged at room temperature (1000 rpm, 5 min). Then medium was removed and sterile mixture 60% medium, 30% FBS and 10% DMSO was added. The mixture was mixed gently and pipetted to cryotubes (in one bank 800k cells is stored). Cryotube was transferred to -80°C in Nalgene Mr. Frosty Freezing Container for the slow freezing (decreasing the temperature approximately 1 °C per minute).

Experiments included in the report were performed from the cell bank prepared at 06.06.2022.

**Table 10. A list of cell banks purchased from ATCC.**

| **Cell line** | **Cell line origin** | **Batch number** | **Lot number** |
|---|---|---|---|
| HAP1 parental | Human near haploid cell line | 51523 | 29281 |
| HAP1 PMS2 KO | Human near haploid cell line | 43674 | 29280 |

### B. Seeding and treatment protocol

### Seeding protocol

When cell confluence reached 90%, cells were washed with sterile PBS (without Ca²⁺ and Mg²⁺) and incubated for 2 min with 2 mL of 0.25% trypsin solution with EDTA. Trypsin was inactivated with the culture medium containing FBS and the collected cells were counted in the Burker chamber. Next, cells were seeded on cover glasses in 12-well plates at the densities listed in Table 11. (also included passage number). Cells were then left to rest for 24h in the incubator.

**Table 11. A list of cell seeding densities. The same densities were used for sSTRIDE and sSTRIDE MMR samples. The passage number is the passage number performed in intoDNA laboratory.**

| **HD name** | **Modification** | **Passage #** | **Seeding density (#cells/well)** |
|---|---|---|---|
| HAP1 | parental | #4-#6 | 30k |
| HAP1 | PMS2 KO | #3-#5 | 50k |

### Treatment protocol

After 24h of rest the operation medium was changed to a fresh one with supplementation of 1 µM or 10 µM 6-Tioguanine (6TG) in DMSO and left to rest for the incubation time 48h (final DMSO concentration was 0,1% in medium in each experimental condition) . In vehicle conditions the operation medium was changed to a fresh one with supplementation of 0,1% sterile DMSO and left to rest for the incubation time. Next, all samples were fixed with ice-cold 70% EtOH and stored at -20°C.

*6TG stock was freshly prepared from powder. The powder was suspended in sterile DMSO to final stock concentration 100mM. 6TG in DMSO was stored in singlequotes in -20°C in the darkness.

### C. Reagents

**Table 12. A list of reagents used in cell culture procedures.**

| **Reagent** | **Producer** | **Serial number** |
|---|---|---|
| Fetal Bovine Serum | Gibco | 16140071 |
| IMDM | Gibco | 1244053 |
| Penicillin-Streptomycin Solution (10,000 units ea.) | Gibco | 15140130 |
| Trypsin-EDTA (0.25%), phenol red | Gibco | 25200056 |
| PBS, pH 7.2 | Gibco | 20012027 |
| Dimethyl Sulfoxide Bioreaqent | Thermo Scientific | J66650.AD |
| DAPI Solution | Thermo Scientific | 62248 |
| 6-Tioguanine (6TG) | Sigma Aldrich | A4882-1G |

### D. Antibodies list

**Table 13. A list of antibodies used in assay validation.**

| **Antibody** | **Producer** | **Serial number** | **Dilution** |
|---|---|---|---|
| Anti-Biotin antibody [Hyb-8] Ms mAb to Biotin | Abeam | ab201341 | 1:100 |
| Rabbit pAb to Biotin | Abeam | ab53494 | 1:100 |
| Recombinant Anti-PMS2 antibody [EPR3947] | Abeam | ab214442 | 1:100 |
| Mouse PMS2 Antibody (B-3) | SCB | sc-25315 | 1:100 |
| Recombinant Anti-MLH1 antibody [EPR3894] | Abeam | ab92312 | 1:100 |
| PMS1 Antibody (PCRP-PMS1-2E11) | Novus | NBP3-13739 | 1:100 |

### In assays with MLH1 and PMS1 antigen retrieval 30s was tested

### E. Cell culture conditions U2OS cells

U2OS cells were purchased by intoDNA from ATCC cell culture collection. Cells were grown at 37°C with 5% CO2 in T25 flasks with DMEM HG + 10% FBS + 1% Penicillin - Streptomycin Solution (10,000 units ea.).

Banking procedure was performed as described below. Cell suspension was centrifuged at room temperature (1000 rpm, 5 min). Then medium was removed and sterile mixture 60% medium, 30% FBS and 10% DMSO was added. The mixture was mixed gently and pipetted to cryotubes (in one bank 800k cells is stored). Cryotube was transferred to -80°C in Nalgene Mr. Frosty Freezing Container for the slow freezing (decreasing the temperature approximately 1 °C per minute).

### F. Seeding and treatment protocol

### Seeding protocol

When cell confluence reached 90%, cells were washed with sterile PBS (without Ca²⁺ and Mg²⁺) and incubated for 4 min with 1 mL of 0.25% trypsin solution with EDTA. Trypsin was inactivated with the culture medium containing FBS and the collected cells were counted in the Burker chamber. Next, cells were seeded on cover glasses in 12-well plates at the densities listed in Table 14. (also included passage number). Cells were then left to rest for 24h in the incubator.

**Table 14. A list of cell seeding densities. The same densities were used for sSTRIDE and sSTRIDE MMR samples. The passage number is the passage number performed in intoDNA labolatory.**

| **ATCC name** | **Treatment time** | **Passage #** | **Seeding density (#cells/well)** |
|---|---|---|---|
| U2OS | 48h | #4-#9 | 75k |
| U2OS | 24h | #4-#9 | 120k |

### Treatment protocol

After 24h of rest the operation medium was changed to a fresh one with supplementation* of 6-Tioguanine (6TG)** in DMSO and left to rest for the incubation time 24h or 48h (final DMSO concentration was 0,1% in medium in each experimental condition). In vehicle conditions the operation medium was changed to a fresh one with supplementation of 0,1% sterile DMSO and left to rest for the incubation time. Next, all samples were fixed with ice-cold 70% EtOH and stored at -20°C.
*6TG concentration range from 250 nM to 50 µM. In each well diluent (DMSO) concentration in medium was 0,1%.
**6TG stock was freshly prepared from powder. The powder was suspended in sterile DMSO to final stock concentration 100mM. 6TG in DMSO was stored in singlequotes in -20°C in the darkness.

### G. Reagents

**Table 15. A list of reagents used in cell culture procedures.**

| **Reagent** | **Producer** | **Serial number** |
|---|---|---|
| Fetal Bovine Serum | Gibco | 16140071 |
| DMEM HG | Gibco | 10313021 |
| Penicillin-Streptomycin Solution (10,000 units ea.) | Gibco | 15140130 |
| Trypsin-EDTA (0.25%), phenol red | Gibco | 25200056 |
| PBS, pH 7.2 | Gibco | 20012027 |
| Dimethyl Sulfoxide Bioreaqent | Thermo Scientific | J66650.AD |
| DAPI Solution | Thermo Scientific | 62248 |
| 6-Tioguanine (6TG) | Sigma Aldrich | A4882-1G |

### H. sSTRIDE-MMR (PMS2) labelling

The labelling was performed according to the protocol described in Example 1

### I. Results

Figure 19 shows HAP1 parental vs PMS2 KO cell line (IF assessment of the staining (Abcam antibody). Representative images show an overlay of DAPI and PMS2 signals. The lack or very weak signal from the PMS2 channel confirm the KO status of one of the cell lines.

Figure 20 shows HAP1 parental vs PMS2 KO with sSTRIDE-MMR labelling. Fluorescence foci are visible in HAP1 WT cells while the majority of HAP1 PMS2 KO cells contains no foci.

Figure 21 shows HAP1 parental: untreated, positive control and negative technical controls (*6TG 1uM 48 h treatment). Quantitative images analysis shows that 6TG treatment results in an increase in the number of detected fluorescence foci while very low number of foci is detected in negative control samples.

Figure 22 shows HAP1 PMS2-KO: untreated, positive control and negative technical controls (*6TG 1uM 48 h treatment). Quantitative images analysis shows that 6TG treatment does not result in a significant increase in the number of fluorescence foci in HAP1 PMS2 KO cells while very low number of foci is detected in negative control samples.

Figure 23 shows HAP1 WT and PMS2-KO cells: untreated, positive control and negative technical controls. Frequency distribution histograms highlight the difference in sSTRIDE-MMR readouts between the cell lines.

Figure 24 shows HAP1 parental: increased 6TG concentration control. Quantitative images analysis shows that treatment with 6TG at a higher concentration results in an elevated level of sSTRIDE-MMR signals.

### Conclusions

1) Comparison of sSTRIDE-MMR (PMS2) readouts between HAP1 WT and PMS2 KO cells shows a significant difference in the number of foci detected in these cell lines. As expected, lower number of foci were detected in HAP1 PMS2 KO cell line.
2) The low number of foci detected in HAP1 PMS2 KO cells confirms the specificity of the assay.
3) Treatment with 6TG results in a statistically significant increase in the number of sSTRIDE-MMR foci only in HAP1 WT cells.

### Example 6 - Comparison of dSTRIDE, dSTRIDE-RAD51 and dSTRIDE-RPA readouts in NCI-H661 and NCI-H1693 cell lines

### Cell culture conditions

NCI-H1693 and NCI-H661 were purchased from ATCC cell culture collection (18.05.2022). The detailed cell culture description is listed on Table 16. Cells were grown at 37°C with 5% CO2 in T75 flasks with RPMI 1640 + 10% FBS + 1% Penicillin - Streptomycin Solution (10,000 units ea.). The master bank collection was prepared form #1 passage.

Banking procedure was performed as described below. Cell suspension was centrifuged at room temperature (1000 rpm, 5 min). Then medium was removed and sterile mixture 60% medium, 30% FBS and 10% DMSO was added. The mixture was mixed gently and pipetted to cryotubes (in one bank 800k cells is stored). Cryotube was transferred to -80°C in Nalgene Mr. Frosty Freezing Container for the slow freezing (decreasing the temperature approximately 1 °C per minute). After 120 minutes the cryotube was transferred to the liquid nitrogen.

### Experiments included in the report were performed from the cell bank prepared at 30.05.2022.

**Table 16. A list of cell banks purchased from ATCC.**

| **Cell line** | **Cell line origin** | **ATCC name** | **Lot number** |
|---|---|---|---|
| NCI-H1693 | Human adenocarcinoma lung | CRL-5887 | 70015981 |
| NCI-H661 | Lung carcinoma human | HBT-183 | 70007203 |

### Seeding and treatment protocol

### Seeding protocol

When cell confluence reached 90%, cells were washed with sterile PBS (without Ca²⁺ and Mg²⁺) and incubated for 2 min with 2 mL of 0.25% trypsin solution with EDTA. Trypsin was inactivated with the culture medium containing FBS and the collected cells were counted in the Burker chamber. Next, cells were seeded on cover glasses in 12-well plates at the densities listed in Table 17. (also included passage number). Cells were then left to rest for 24h in the incubator.

**Table 17. A list of cell seeding densities. The same densities were used for dSTRIDE, dSTRIDE HR and dSTRIDE RPA in each treatment condition.**

| **ATCC name** | **Cell line** | **Passage #** | **Seeding density (#cells/well)** |
|---|---|---|---|
| CRL-5887 | NCI-H1693 | #3 | 80k |
| HBT-183 | NCI-H661 | #5 | 70k |

### Treatment protocol

After 24h of rest the operation medium was changed to a fresh one with supplementation of 5 µM or 50 µM etoposide* and left to rest for the incubation time 48h (final DMSO concentration was 0,1% in medium in each experimental condition) . In vehicle conditions the operation medium was changed to a fresh one with supplementation of 0,1% sterile DMSO and left to rest for the incubation time. Next, all samples were fixed with ice-cold 70% EtOH and stored at -20°C.

*Etoposide stock (100mM in DMSO) is stored in -20°C in the darkness.

### Reagents

**Table 18. A list of reagents used in cell culture procedures.**

| **Reagent** | **Producer** | **Serial number** |
|---|---|---|
| Fetal Bovine Serum | Gibco | 16140071 |
| RPMI 1640 | Gibco | 11875101 |
| Penicillin-Streptomycin Solution (10,000 units ea.) | Gibco | 15140130 |
| Trypsin-EDTA (0.25%), phenol red | Gibco | 25200056 |
| PBS, pH 7.2 | Gibco | 20012027 |
| Dimethyl Sulfoxide Bioreagent | Thermo Scientific | J66650.AD |
| DAPI Solution | Thermo Scientific | 62248 |
| Etoposide | Torcis Chemicals | 6A/24772 |

### dSTRIDE / dSTRIDE-RAD51 / dSTRIDE-RPA labelling

The labelling was performed according to protocols described in Examples 2 or 3.

### Results

1) In both cell lines etoposide treatment resulted in a dose-dependent increase in the number of double-strand DNA breaks detected by dSTRIDE.
2) The baseline level of DSBs (dSTRIDE) and the increase in DSBs after 5 um etoposide treatment is comparable in both cell lines.
3) 50 uM etoposide results in a more profound increase in the number of DSBs (dSTRIDE) in NCI-H1693 cell line.
4) In both cell lines etoposide treatment resulted in a dose-dependent increase in the number of RPA-associated double-strand DNA breaks detected by dSTRIDE-RPA.
5) 50 uM etoposide treatment resulted in a significant increase in the number of RAD51-associated double-strand DNA breaks detected by dSTRIDE-RAD51 in NCI-H661.
6) Etoposide treatment did not results in a detectable increase in the number of RAD51-associated double-strand DNA breaks detected by dSTRIDE-RAD51 in NCI-H1693.

Figure 25 shows dSTRIDE imaging with and without etoposide. Representative images show the increase in the number of double-strand DNA breaks detected by dSTRIDE and represented as fluorescence foci in NCI-H1693 after etoposide treatment.

Figure 26 shows results of dSTRIDE signals data analysis, measurement: level of double-strand DNA breaks after the treatment with etoposide. Quantitative image analysis shows a dose-dependent increase in the number of DSBs after etoposide treatment.

Figure 27 shows Results of dSTRIDE signals data analysis, measurement: level of double-strand DNA breaks after the treatment with etoposide. *in vehicle condition 5% of most damage cells have >=192 foci.

Figure 28 shows results of dSTRIDE signals data analysis, measurement: level of double-strand DNA breaks after the treatment with etoposide. Quantitative image analysis shows a dose-dependent increase in the number of DSBs after etoposide treatment.

Figure 29 shows results of dSTRIDE signals data analysis, measurement: level of double-strand DNA breaks after the treatment with etoposide. *in vehicle condition 5% of most damage cells have >=205 foci.

Figure 30 shows results of dSTRIDE signals data analysis, cell line comparison. Quantitative image analysis shows that after treatment with etoposide at 50 uM concentration more DSBs are induced in NCI-H1693 than in NCI-H661 cell lines.

Figure 31 shows RAD51 IF staining in NCl-H661 and NCI-H1693 cell lines. No clear difference between the cell lines is visible.

Figure 32 shows -dSTRIDE-HR (RAD51) staining in BRCA2 WT cell line with and without etoposide. Etoposide treatment results in an increase in the number of visible fluorescence foci.

Figures 33-36 show results of dSTRIDE HR signals data analysis, measurement: level of double-strand DNA breaks associated with Rad51 after the treatment with etoposide. While in NCI-H661 there is a dose-dependent increase in the number of detected dSTRIDE-HR (RAD51) foci after etoposide treatment, no response is visible in NCl-H1693 cell line.

Figure 37 shows results of dSTRIDE HR signals data analysis, cell line comparison. No response is visible in NCI-H1693 cell line.

Figure 38 shows dSTRIDE-RPA staining in BRCA2 WT cell line with and without etopside. An increase in the number of foci is visible after treatment with etoposide.

Figures 39- 42 show results of dSTRIDE RPA signals data analysis, measurement: level of double-strand DNA breaks associated with RPA70 after the treatment with etoposide. The results show that in both cell lines there is a dose-dependent increase in the number of dSTRIDE-RPA foci after treatment with etoposide.

Figure 43 shows results of dSTRIDE RPA signals data analysis, cell line comparison. The results show that there are more dSTRIDE-RPA foci detected in NCI-H1693 cell line after 50 uM etoposide treatment.

Figure 44 shows dSTRIDE, dSTRIDE-HR (Rad51) and dSTRIDE-RPA comparison in BRCA2 WT cell line with etoposide treatment. The results show that there is a dose-dependent response visible in all assays after etoposide treatment.

Figure 45 shows dSTRIDE, dSTRIDE-HR (Rad51) and dSTRIDE-RPA comparison in HR defective (termed "BRCA2 KO") cell line with etoposide treatment. Quantitative image analysis revealed that etoposide treatment leads to a dose-dependet formation of double-strand DNA breaks in NCI-H1693 cells. The results from the dSTRIDE-RPA assay provide proof that RPA is involved in the repair of those lesions, while the dSTRIDE-HR (RAD51) assay results confirm that RAD51 recruiment to sites of DSBs in not efficient.

### Conclusions

Etoposide efficiently induces DSBs in both tested cell lines, yet the mutation in the HR pathway in NCI-H1693 cell line results in an increased level of breaks. The inability of this cell line to successfully perform HR repair is clearly shown when combining dSTRIDE, dSTRIDE-RPA and dSTRIDE-RAD51 data, as no response is seen in the latter assay confirming that RAD51 loading is not sufficient. dSTRIDE-RAD51 assay can be thus considered as a functional HR assay which informs about the status of HR repair.

### Example 7- Development of sSTRIDE-MMR (PMS1 and MLH1) assay

The sSTRIDE-MMR methods were performed as in Examples 1 and 5. However, either PMS1 or MLH1 were targeted instead of PMS2.

Figure 46 shows results of quantitative analysis in HAP1 cells (WT and KO) for sSTRIDE-MLH1. Quantitative image analysis reveals that MLH1 protein is present at SSBs in both PMS2 WT and KO cell lines and that 6TG treatment results in an increase in sSTRIDE-MMR (MLH1) readouts Figure 47 shows results of quantitative analysis in HAP1 cells for sSTRIDE-MLH1 with or without 6TG treatment. Frequency distribution plots confirm the readouts from the sSTRIDE-MMR (MLH1) assay. Figure 48 shows comparison between PMS2 and MLH1 results. The results show that while PMS2 is present at a very low levels in HAP1 PMS2 KO cells, MLH1 can be recruited to SSBs in both cell lines.

Figure 49 shows results of quantitative analysis in HAP1 cells (WT and KO) for sSTRIDE-PMS1. The obtained results show that PMS1 is present at SSBs in both cell lines and that it is engaged in MMR processes after treatment with 6TG.

Figure 50 shows comparison between PMS2, PMS1 and MLH1 results. The results show that both MLH1 and PMS1 are involved in MMR in the absence of PMS2.

## Claims

1. A method for detection of nucleic acid end(s) in a biological material containing nucleic acids, wherein the method comprises the steps of:
a) incubating the biological material with a proteinase for less than 2 minutes;
b) adding nucleic acid binding molecules to the biological material under conditions such that the nucleic acid binding molecules bind to the nucleic acid end(s) in the biological material;
c) adding two different binding molecules to the biological material under conditions such that one of the binding molecules binds to the nucleic acid binding molecules bound to the nucleic acid end(s) and the other binding molecule binds to a nucleic acid binding protein that is bound to the nucleic acid at the nucleic acid end(s); and
d) detecting the nucleic acid end(s) in the biological material by detecting a co-localisation of the nucleic acid binding protein and the nucleic acid end via binding of the two different binding molecules.

2. The method of claim 1, wherein the nucleic acids comprise DNA and/or RNA, preferably DNA.

3. The method of claim 1 or claim 2, wherein the nucleic acid end(s) is a single stranded nucleic acid break or a double stranded nucleic acid break.

4. The method of any one of claims 1-3, wherein the proteinase is an aspartic protease, a glutamic protease, a metalloprotease, a cysteine protease, a serine protease, or a threonine protease, preferably wherein the protease is a serine protease, such as proteinase K.

5. The method of any one of claims 1-4, wherein incubating the biological material with a proteinase is performed:
a. for between 1 second and less than 2 minutes, between 15 seconds and 90 seconds, between 20 seconds and 80 seconds, between 25 seconds and 70 seconds, preferably between about 30 seconds and about 60 seconds; and/or
b. at a temperature of between 15ºC and 30 ºC, preferably between 20ºC and 25ºC.

6. The method of any one of claims 1-5, wherein the proteinase is in solution, preferably at a concentration of between 1 µg/mL and 20 µg/mL, between 3 µg/mL and 15 µg/mL, between 5 µg/mL and 10 µg/mL, or between 6 µg/mL and 8 µg/mL, preferably about 7 µg/mL.

7. The method of any one of claims 1-6, wherein the nucleic acid binding molecules:
a. are halogenated nucleotide or nucleoside molecules, DNA precursor analogues, and/or biotinylated nucleotide molecules; and/or
b. bind to the nucleic acid end(s) in the biological material by an enzyme catalysed process of addition, for example, using DNA polymerase I, TdT, Klenow fragment, Phu polymerase, Taq polymerase, T4 DNA Polymerase, T7 DNA Polymerase, T4 Polynucleotide Kinase, or RNA polymerase.

8. The method of any one of claims 1-7, wherein:
a. the two different binding molecules may be or may comprise two different monoclonal antibodies, or fragments thereof, and/or
b. each of the two different binding molecules is attached to a different oligonucleotide molecule.

9. The method of any one of claims 1-8, wherein the nucleic acid binding protein is:
a. a protein found in the biological material;
b. bound to the nucleic acid at the nucleic acid end(s) either directly or indirectly;
c. a nucleic acid repair protein, preferably a nucleic acid end (e.g. break) repair protein; and/or
d. RAD51, RPA (e.g. RPA70), PMS2, MLH1, PMS1, p53, MSH2, Ataxia telangiectasia and Rad3 related protein, ATM serine/threonine kinase, RAD52, XRCC1, Proliferating cell nuclear antigen, XPC, Ku70, Ku80, Nibrin, DDB2, Bloom syndrome protein, CHEK2, RAD51C, DNA polymerase eta, Rad50, DDB1, RBBP8, FANCB, PALB2, H2AX, yH2AX DNA repair and recombination protein RAD54-like, PrimPol, REV1, Terminal deoxynucleotidyl transferase, DNA polymerase nu, Fanconi anemia, complementation group C, FANCF, ERCC8, Artemis, Ubiquitin ligase, RNF4, TP53BP1, AP endonuclease, ERCC4, Transcription factor II H, XRCC3, XRCC2, RecA, ERCC6, SLX4, Sirtuin 1, PTEN, Replication protein A2, Replication protein A3, Alkb homolog 3, alpha-ketoglutaratedependent dioxygenase, Exonuclease 5, DNA polymerase alpha catalytic subunit, Cyclin H, or PARP1/2.

10. The method of any one of claims 1-9, wherein the method further comprises a step of adding two different further binding molecules that respectively bind to the two different binding molecules after the step of adding the two different binding molecules, optionally wherein each of the two different further binding molecules is attached to a different oligonucleotide molecule.

11. The method of any one of claims 1-10, wherein the method further comprises, after the step of adding two different binding molecules (or after the step of adding the further binding molecules when dependent from claim 10) a step of adding different further oligonucleotide molecules which hybridise with the oligonucleotide molecules attached to the two different binding molecules (or to the further binding molecules) to form a circular template, optionally wherein the circular template is formed by ligation of the two different further oligonucleotide molecules.

12. The method of any one of claims 1-11, wherein detecting the nucleic acid end(s) comprises a step of performing nucleic acid amplification to produce an amplification product that is detected, optionally wherein the nucleic acid amplification is rolling circle amplification.

13. The method of any one of claims 1-12, wherein detecting the co-localisation of the nucleic acid binding protein and the nucleic acid end comprises:
a. a proximity ligation assay;
b. a branched proximity hybridization assay;
c. a FRET detection; or
d. a proximity-driven reaction with fluorophores or dyes.

14. A kit for detection of nucleic acid end(s) in a biological material, the kit comprising:
a) nucleic acid binding molecules;
b) a binding molecule that binds to the nucleic acid binding molecules; and
c) a binding molecule that binds to a nucleic acid binding protein.

15. A method for assessing effectiveness of a therapeutic agent, the method comprising the steps:
a) performing the method of any one of claims 1-13 on a sample obtained from a subject before the administration of the therapeutic agent,
b) performing the method of any one of claims 1-13 on a sample obtained from the subject after the administration of the therapeutic agent,
c) comparing the amount of the nucleic acid end(s) detected in steps a) and b), wherein the therapeutic agent is effective if the amount of the detected nucleic acid end(s) is greater in step a) than step b),
wherein steps a) and b) can be performed in any order,
optionally wherein the therapeutic agent targets PMS1, PMS2 or MLH1;
or
a) performing the method of any one of claims 1-13 on a sample obtained from a subject before the administration of the therapeutic agent,
b) performing the method of any one of claims 1-13 on a sample obtained from the subject after the administration of the therapeutic agent,
c) comparing the amount of the nucleic acid end(s) detected in steps a) and b), wherein the therapeutic agent is effective if the amount of the nucleic acid end(s) is greater in step b) than step a),
wherein steps a) and b) can be performed in any order,
optionally wherein the therapeutic agent targets RPA or RAD51.
